# EUROPEAN PATENT SPECIFICATION

(11) **EP 4 306 521 B1**
(45) Date of publication and mention of the grant of the patent: **15.04.2026**
(21) Application number: 22787621.6
(22) Date of filing: 15.04.2022
(51) Int. Cl.: C07F 15/00, C07D 413/14, C09K 11/06, C07D 471/04, C07D 263/52, C07D 401/14, C07D 413/12, C07D 417/12, H10K 85/00, H10K 85/30

(54) **CENTRAL CHIRALITY INDUCED SPIRO CHIRAL TETRADENTATE CYCLOMETALATED PLATINUM (II) AND PALLADIUM (II) COMPLEX-BASED CIRCULARLY POLARIZED LUMINESCENCE MATERIAL AND APPLICATION THEREOF**
DURCH ZENTRALCHIRALITÄT INDUZIERTES SPIRO CHIRAL TETRADENTATES CYCLOMETALIERTES PLATINUM (II) UND ZIRKULÄR POLARISIERTEM LUMINESZENZMATERIAL BASIEREND AUF PALLADIUM (II)-KOMPLEXEN UND DESSEN ANWENDUNG
MATÉRIAU DE LUMINESCENCE À POLARISATION CIRCULAIRE À BASE DE COMPLEXE DE PALLADIUM (II) CYCLOMÉTALLÉ DE TÉTRADENTATE SPIRO CHIRAL INDUIT PAR CHIRALITÉ CENTRALE ET À BASE DE COMPLEXE DE PALLADIUM (II)

(30) Priority: 15.04.2021 CN 202110403546
(43) Date of publication of application: 17.01.2024
(73) Proprietor: Zhejiang University of Technology, Hangzhou, Zhejiang 310014 (CN); Zhejiang Huaxian Photoelectricity Technology Co., Ltd, Jiaxing, Zhejiang 314107 (CN)
(72) Inventor: LI, Guijie, Hangzhou, Zhejiang 310014 (CN); SHE, Yuanbin, Hangzhou, Zhejiang 310014 (CN); GUO, Hua, Hangzhou, Zhejiang 310014 (CN); XU, Kewei, Hangzhou, Zhejiang 310014 (CN); LIU, Shun, Hangzhou, Zhejiang 310014 (CN); ZHAN, Feng, Hangzhou, Zhejiang 310014 (CN); WEN, Jianfeng, Hangzhou, Zhejiang 310014 (CN)
(74) Representative: Loo, Chi Ching
(86) International application number: PCT/CN2022/086993
(87) International publication number: WO 2022/218399

(56) References cited:
- CN-A- 104 232 076
- CN-A- 104 693 243
- CN-A- 109 111 487
- US-A1- 2015 105 556
- US-A1- 2015 162 552
- US-A1- 2015 194 616
- US-A1- 2021 363 420
- DATABASE REAXYS [online] 1 January 2015 (2015-01-01), ARIZONA BOARD OF REGENTS: "Stable emitters - US2015/162552 A1", XP093185001, Database accession no. XRN = 29138197, 29138228, 29138231, 29138234, 2913
- LI GUIJIE, ZHENG JIANBING, FANG XIAOLI, XU KEWEI, YANG YUN-FANG, WU JIANG, CAO LINYU, LI JIAN, SHE YUANBIN: "N -Heterocyclic Carbene-Based Tetradentate Pd(II) Complexes for Deep-Blue Phosphorescent Materials", ORGANOMETALLICS, AMERICAN CHEMICAL SOCIETY, vol. 40, no. 4, 22 February 2021 (2021-02-22), pages 472 - 481, XP055976833, ISSN: 0276-7333, DOI: 10.1021/acs.organomet.0c00648

## Description

### TECHINCAL FIELD

The disclosure relate to a circularly polarized luminescence material and application thereof, in particular to a central chirality induced spiro chiral tetradentate cyclometalated platinum (II) and palladium (II) complex-based circularly polarized luminescence material and application thereof.

### BACKGROUND ART

Circularly polarized luminescence (CPL) is a phenomenon that chiral luminescence materials are excited and emit left-handed or right-handed circularly polarized light. Therefore, design and development of chiral luminescence materials are the key to this field. With in-depth researches by researchers, circularly polarized luminescence materials exhibit important applications in 3D display, data storage, quantum computing, optical anti-counterfeiting, biological imaging and asymmetric synthesis so far.

Cyclometalated platinum (II) and palladium (II) complex-based phosphorescent material can fully utilize all of singlet and triplet excitons generated by electroexcitation due to its heavy atom effect, so that its maximum theoretical quantum efficiency can be up to 100%, and thus such complex is an ideal luminescence material. Bidentate cyclometalated platinum (II) and palladium (II) complexes have relatively low rigidity, and its luminescent quantum efficiency is reduced due to a fact that two bidentate ligands are easy to twist and vibrate so that energy of material molecule with an excited state is consumed in a non-radiation mode (Inorg. Chem. 2002, 41, 3055). Although tridentate ligand-based cyclometalated platinum (II) and palladium (II) complexes can be with improved luminescence quantum efficiency due to enhanced molecular rigidity (Inorg. Chem. 2010, 49, 11276), a second unidentate ligand (such as Cl⁻, phenoxy negative ions, alkyne negative ions, carbene and the like) contained greatly reduces chemical stability and thermal stability of the complexes, which is difficult to sublimate and purify for preparation of OLED devices. Therefore, the luminescence material base on that bidentate and tridentate ligand-based cyclometalated complexes do not facilitate their application in stable and efficient OLED devices. Central metal ions of divalent cyclometalated platinum (II) and palladium (II) complexes are all dsp² hybridized, and are easy to coordinate with the tetradentate ligand to form stable and rigid molecules with a planar quadrilateral configuration. High molecular rigidity can inhibit nonradiative relaxation caused by molecular vibration and rotation, and reduce energy loss of material molecules with the excited state, thereby facilitating improving of the luminescent quantum efficiency of the material molecules. Due to steric hindrance of two aryl groups at an end of a tetradentate cyclometalated platinum (II) and palladium (II) complex, material molecules exhibit a distorted quadrilateral configuration (Chem. Mater. 2020, 32, 537), which theoretically has a spirochiral property. However, the molecules are easily racemized by up and down vibration of the two aryl groups at the end of the ligand in a solution or in a heating and sublimation process, and enantiomers cannot be separated. It is extremely difficult to obtain optically pure cyclometalated platinum (II) and palladium (II) complex material molecules so that they do not have circularly polarized luminescence property. Therefore, how to design and develop the optically pure cyclometalated platinum (II) and palladium (II) complex material molecules with high chemical stability and thermal stability and with circularly polarized luminescence property is of great significance and great practical value for its application in circularly polarized light-emitting OLED devices (CP-OLED), and is also an urgent problem to be solved in the CP-OLED field. Example platinum complexes with applications in OLED devices known in the art can be found in for example US20150105556A1 and US2015162552A1.

### SUMMARY

In view of defects of related art, a central chirality induced spiro chiral tetradentate cyclometalated platinum (II) and palladium (II) complex-based circularly polarized luminescence material and application thereof are provided in this disclosure. Spiro chiral metal complex molecules can autonomously induce a whole tetradentate ligand to coordinate with metal ions in a less sterically hindered manner by means of a central chiral fragment L^{a} in the tetradentate ligand, to form an optically pure spiro chiral metal complex-based circularly polarized luminescence material, without need for chiral resolution. Moreover, the material has high chemical stability and thermal stability, and has important applications in circularly polarized luminescence devices.

An object of the disclosure can be achieved by following technical schemes.

A central chirality induced spiro chiral tetradentate cyclometalated platinum (II) and palladium (II) complex-based circularly polarized luminescence material is characterize by having a chemical formula as shown in general formulas **(III)** and **(III'),** where **(III)** and **(III')** are enantiomers of each other.

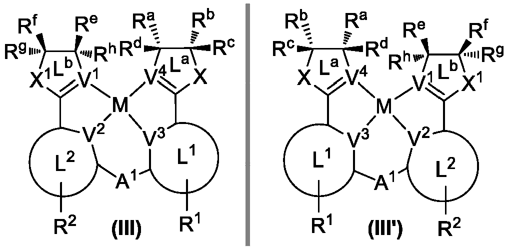

M is Pt or Pd; and V¹, V², V³ and V⁴ are independently N or C.

L¹, L² and L³ are each independently a five or six membered carbocyclic, heterocyclic, aromatic or heteroaromatic ring; and L^{a} and L^{b} are each independently a five-membered central chiral carbocyclic or heterocyclic ring. Due to steric hindrance between L^{a} and L¹, L^{a} and L^{b}, the metal complex is in a non-planar configuration as a whole, and the central chiral L^{a} can autonomously induce formation of a spiro chiral tetradentate cyclometalated platinum (II) and palladium (II) complex with metal as a center in a less sterically hindered manner.

A¹, A², X and X¹ are each independently O, S, CR^{x}R^{y}, C=O, SiR^{x}R^{y}, GeR^{x}R^{y}, NR^{z}, PR^{z}, R^{z}P=O, AsR^{z}, R^{z}As=O, S=O, SO₂, Se, Se=O, SeO₂, BH, BR^{z}, R^{z}Bi=O or BiR^{z}.

R¹and R² each independently represent mono-,di-,tri-or tetra-substituted or unsubstituted, and meanwhile, R¹, R², R³, R^{a}, R^{b}, R^{c}, R^{d}, R^{e}, R^{f}, R^{g}, R^{h}, R^{x}, R^{y} and R^{z} are each independently hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphonamido, imino, sulfo, carboxyl, hydrazino, substituted silyl, or combinations thereof. Two adjacent R¹and R² can be selectively connected to form a fused ring; any two of R^{a}, R^{b}, R^{c} and R^{d} can be connected to form a cyclic system, and any two of R^{e}, R^{f}, R^{g} and R^{h} can be connected to form a cyclic system.

L^{a} and L^{b} in structures of the general formulas of the central chirality induced spiro chiral tetradentate cyclometalated platinum (II) and palladium (II) complex-based circularly polarized luminescence material can be of, but not limited to, following structures: 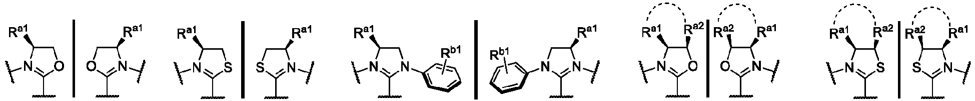 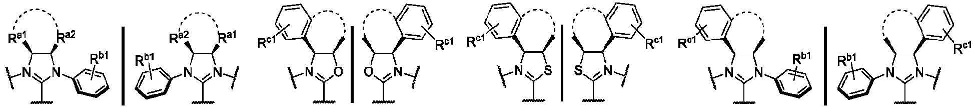 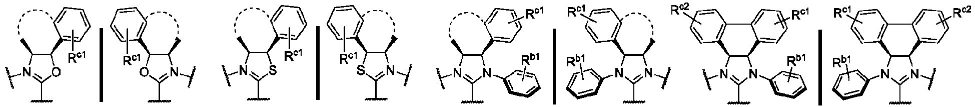 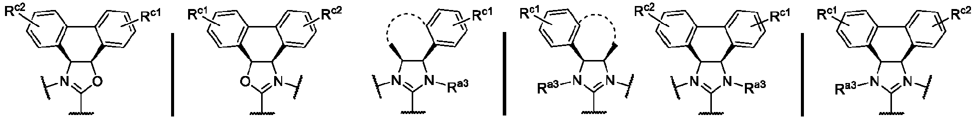
wherein R^{a1}, R^{a2} and R^{a3} each independently represent hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, substituted silyl, or combinations thereof; and
where R^{b1}, R^{c1} and R^{c2} each independently represent mono-,di-,tri-or tetra-substituted or unsubstituted, while R^{b1}, R^{c1} and R^{c2} are each independently hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, substituted silyl, or combinations thereof; and two or more adjacent R^{b1}, R^{c1}, and R^{c2} may be selectively joined to form a fused ring.

L^{a} and L^{b} described above may further be specifically of, but not limited to, following structures: 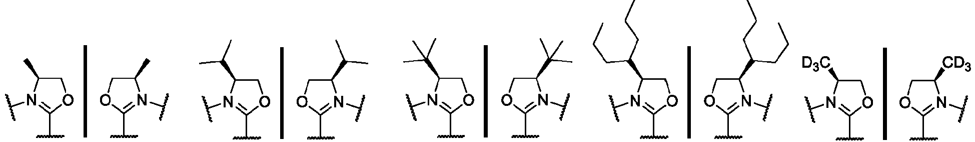 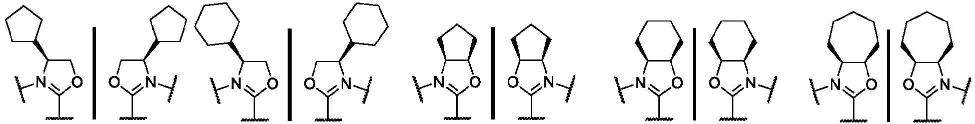 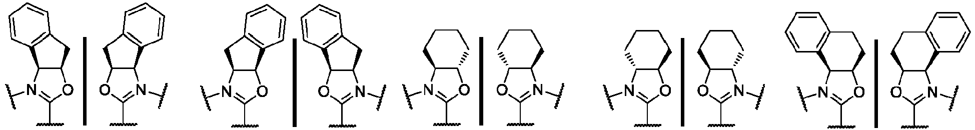 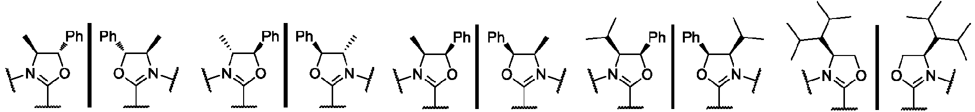 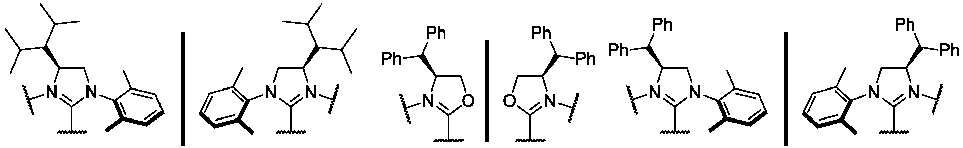 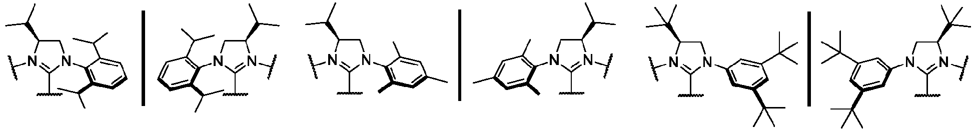 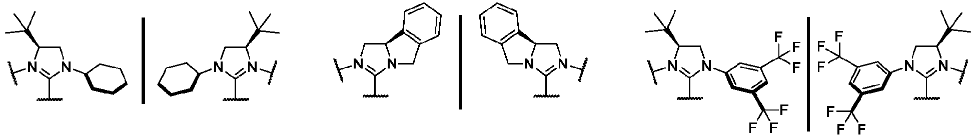 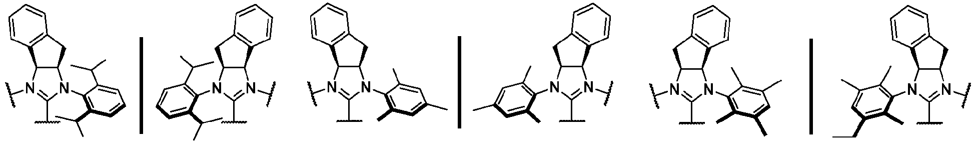 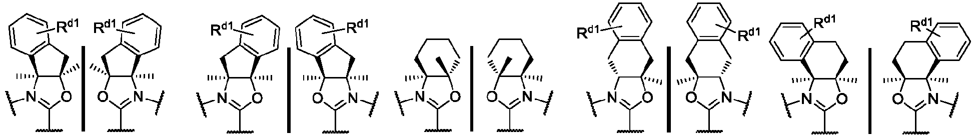 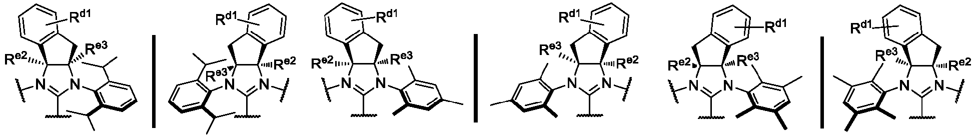 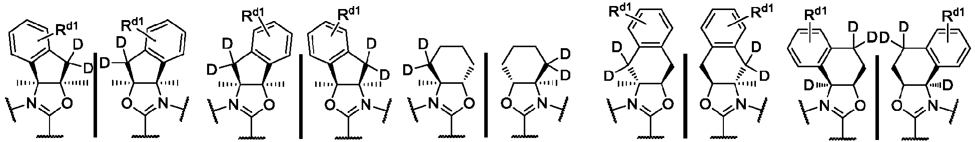 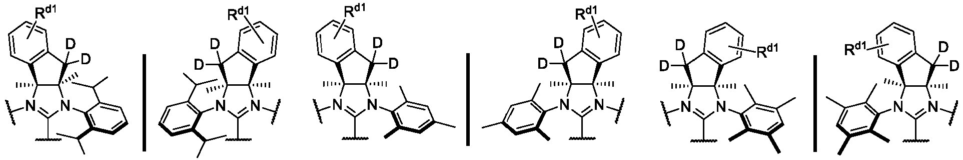 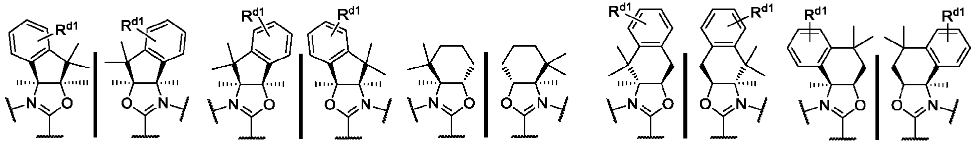 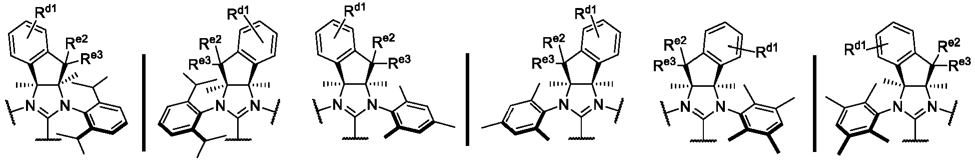 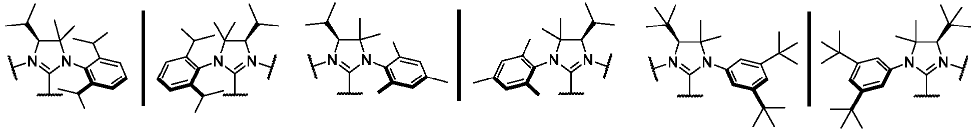 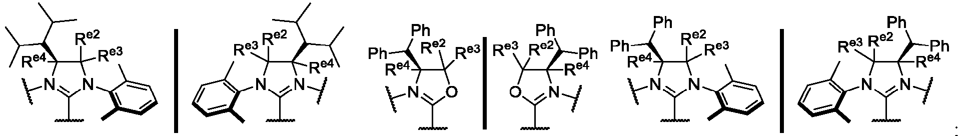 where R^{d1}, R^{e1}, R^{e2}, R^{e3} and R^{e4} each independently represent mono-,di-,tri-or tetra-substituted or unsubstituted, while R^{b1}, R^{c1} and R^{c2} are each independently hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, substituted silyl, or combinations thereof; and two or more adjacent R^{b1}, R^{c1}, and R^{c2} are selectively joined to form a fused ring.

Further, the central chirality induced spiro chiral tetradentate cyclometalated platinum (II) and palladium (II) complex-based circularly polarized luminescence material is preferably selected from a group consisting of:

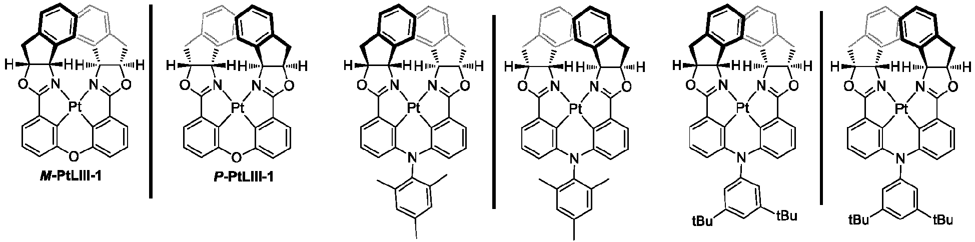

Further, the central chirality induced spiro chiral tetradentate cyclometalated platinum (II) and palladium (II) complex-based circularly polarized luminescence material is applied to an organic light emitting element. The organic light emitting element is an organic light emitting diode, a light emitting diode, or a light emitting electrochemical cell.

The organic light emitting element includes a first electrode, a second electrode and at least one organic layer provided between the first electrode and the second electrode. The organic layer includes the central chirality induced spiro chiral tetradentate cyclometalated platinum (II) and palladium (II) complex-based circularly polarized luminescence material.

Further, the central chirality induced spiro chiral tetradentate cyclometalated platinum (II) and palladium (II) complex-based circularly polarized luminescence material is applied to an organic light emitting element. The organic light emitting element is a 3D display device, a three-dimensional imaging device, an optical information encryption device, an information storage device, a biological imaging device, or the like.

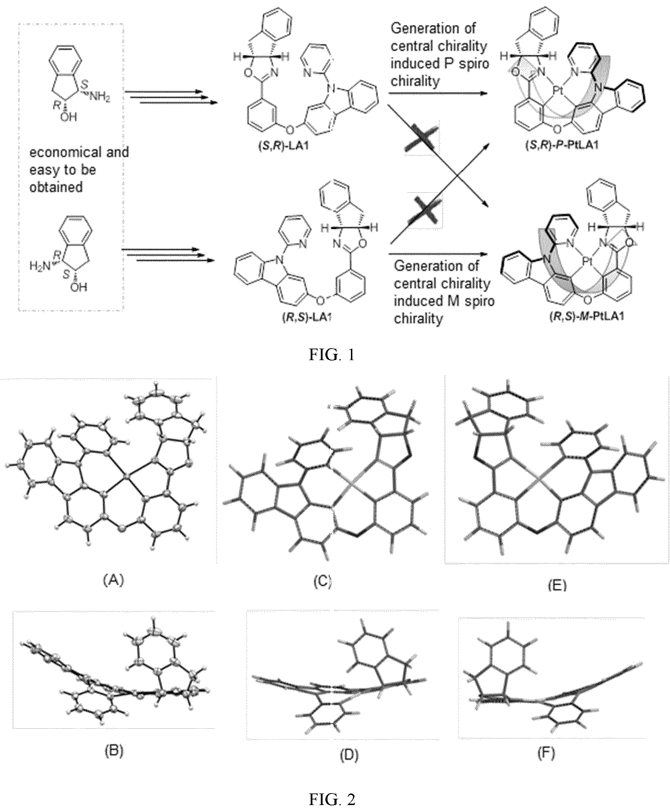

The disclosure includes following beneficial effects.
(1) Generation of central chirality autonomously induced spiro chirality. By designing and developing a tetradentate ligand with central chiral L^{a}, a whole tetradentate cyclometalated platinum (II) and palladium (II) complex molecule can be in a twisted quadrilateral configuration by using steric hindrance effect between the tetradentate ligand and a ligand L¹ or L^{b} at another end. Meanwhile, the central chiral L^{a} can independently induce the whole tetradentate ligand to coordinate with a metal ion in a less sterically hindered manner so as to form the optically pure spiro chiral tetradentate cyclometalated platinum (II) and palladium (II) complex circularly polarized luminescence material with the metal ion as a center, as shown in FIG. 1.
(2) Optically pure raw materials are economical and easy to be obtained. The two corresponding optically pure chiral isomers required for preparing the tetradentate ligand containing the central chiral L^{a} are both commercial, economic and easily-obtained compounds, which facilitates preparation of the two optically pure chiral tetradentate ligands in a large amount.
(3) There's no need for chiral resolution for the circularly polarized luminescence material. Circularly polarized luminescence material of two corresponding optically pure chiral isomers of the spiro chiral tetradentate cyclometalated platinum (II) and palladium (II) complex can be conveniently prepared from the two optically pure chiral tetradentate ligands, which greatly reduces preparation cost of the material without separation and purification through a chiral column.
(4) High chemical stability and thermal stability of the material. The designed and developed tetradentate ligand can well coordinate with dsp² hybridized platinum (II) and palladium (II) metal ions to form molecules with a stable and rigid quadrilateral configuration, with high chemical stability; meanwhile, since large steric hindrance effect exists between the designed central chiral ligand L^{a} and the ligand L¹ or L^{b} at another end, the metal complex molecule as a whole can form a stable spiro chiral tetradentate cyclometalated complex, so that the spiro chiral tetradentate cyclometalated complex can not be racemized and lose circularly polarized luminescence property in a solution or in a process of sublimation at a high temperature.

### BRIEF DESCRIPTION OF THE DRAWINGS

FIG. 1 shows a design idea of an optically pure spiro chiral tetradentate cyclometalated platinum (II) and palladium (II) complex circularly polarized luminescence material with metal ion as a center;
In **FIG. 2****,** plot (A) is a front view of a X- ray diffraction single crystal structure of an optically pure spiral chiral material (*R*, *S*)-*M*-PtLA1; plot (B) is a top view of the X- ray diffraction single crystal structure of the optically pure spiral chiral material (*R*, *S*)-*M*-PtLA1; plot (C) is a front view of a molecular structure of (*R, S*)-*M*-PtLA1 optimized by Density Functional Theory (DFT) computation, plot (D) is a top view of the molecular structure of (*R*, *S*)-*M*-PtLA1 optimized by Density Functional Theory (DFT) computation, plot (E) is a front view of a molecular structure of (*S, R*)-*P*-PtLA1 optimized by DFT computation, and plot (F) is a top view of the molecular structure of (*S, R*)-*P*-PtLA1 optimized by DFT computation;
In **FIG. 3****,** plot (A) is a front view of a molecular structure of (*R*, *S*)-*M*-PtLB3 optimized by DFT computation, plot (B) is a top view of the molecular structure of (*R*, *S*)-*M*-PtLB3 optimized by DFT computation, plot (C) is a molecular structure of (*R*, *S*)-*M*-PtLB3, plot (D) is a front view of a molecular structure of (*S, R*)-*P*-PtLB3 optimized by DFT computation; plot (E) is a top view of the molecular structure of (*S, R*)-*P*-PtLB3 optimized by DFT computation, and plot (F) is a molecular structure of (*S, R*)*-P*-PtLB3;
In **FIG. 4****,** plot (A) is a front view of a molecular structure of (*R, S*)-*M*-PtLH1 optimized by DFT computation, plot (B) is a top view of the molecular structure of (*R, S*)-*M*-PtLH1 optimized by DFT computation, plot (C) is a molecular structure of (*R*, *S*)-M-PtLH1, plot (D) is a front view of a molecular structure of (*S, R*)-*P*-PtL H1 optimized by DFT computation; plot (E) is a top view of the molecular structure of (*S, R*)-*P*-PtL H1 optimized by DFT computation, and plot (F) is a molecular structure of (*S, R*)-*P*-PtL H1;
In **FIG. 5****,** plot (A) is a front view of a molecular structure of *M*-PtLIII-1 optimized by DFT computation, plot (B) is a top view of the molecular structure of *M*-PtLIII-1 optimized by DFT computation, plot (C) is a front view of a molecular structure of *P*-PtLIII-1 optimized by DFT computation; plot (E) is a top view of the molecular structure of *P*-PtLIII-1 optimized by DFT computation;
**FIG. 6** is an emission spectrum of optically pure (R, S)-M-PtLA1 and (*S*, *R*)-P-PtLA1 in dichloromethane at a room temperature;
**FIG. 7** is an emission spectrum of optically pure (*R, S*)-M-PtLA2 and (*S, R*)-P-PtLA2 in dichloromethane at a room temperature;
**FIG. 8** is an emission spectrum of optically pure (*R, S*)-M-PtLA3 and (*S, R*)-P-PtLA3 in dichloromethane at a room temperature;
**FIG. 9** is an emission spectrum of optically pure (*R, S*)-*M*-PtLAN and (*S, R*)-*P*-PtLAN in dichloromethane at a room temperature;
**FIG. 10** is an emission spectrum of optically pure (*R*, *S*)-*M*-PtLH1 and (*S, R*)-*P*-PtLH1 in dichloromethane at a room temperature;
**FIG. 11** is an emission spectrum of optically pure (*S*, *R*)-P-PtLC3 and *P*-PtLIII-1 in dichloromethane at a room temperature;
**FIG. 12** **is** an emission spectrum of optically pure *M*-PdLA1 and *P*-PdLA1 in dichloromethane at a room temperature;
**FIG. 13** is an emission spectrum of optically pure *M*-PtLB1 and *P*-PtLB 1 in dichloromethane at a room temperature;
**FIG. 14** is an emission spectrum of optically pure *M*-PtLC1 and *P*-PtLC1 in dichloromethane at a room temperature;
**FIG. 15** is an emission spectrum of optically pure M-PtLD1 and *P*-PtL1 in dichloromethane at a room temperature;
**FIG. 16** is an emission spectrum of optically pure *M*-PtLE1 and *P*-PtLE1 in dichloromethane at a room temperature;
**FIG. 17** is an emission spectrum of optically pure *M*-PtLF1 and *P*-PtLF1 in dichloromethane at a room temperature;
**FIG. 18** is an emission spectrum of optically pure(*R*, *S*)-*M*-PtLK1 and (*S, R*)-*P*-PtL K1 in dichloromethane at a room temperature;
**FIG. 19** is an emission spectrum of optically pure *M*-PtL1 and *P*-PtL1 in dichloromethane at a room temperature;
**FIG. 20** is an emission spectrum of optically pure *M*-PtL3 and *P*-PtL3 in dichloromethane at a room temperature;
**FIG. 21** is a circular dichroism (CD) spectrum of (*S, R*)-*P*-PtLA1 and (*R*, S)-*M*-PtLA1 in dichloromethane;
**FIG. 22** is a circular dichroism (CD) spectrum of *P*-PtOO and *M*-PtLOO in dichloromethane;
**FIG. 23** is a circular dichroism (CD) spectrum of (*S, R*)-*P*-PtLA3 and (*R*, *S*)-*M*-PtLA3 in dichloromethane;
**FIG. 24** is a circular dichroism (CD) spectrum of (*S, R*)-*P*-PtLJ1 and (*R*, *S*)-*M*-PtLJ1 in dichloromethane;
**FIG. 25** is a circular dichroism (CD) spectrum of *P*-PtLB3 and *M*-PtL B3 in dichloromethane;
**FIG. 26** is a circularly polarized luminescence spectrum (CPPL) of P-PtOO, M-PtLOO, and their mixtures at an equivalent dose in dichloromethane;
**FIG. 27** is a circularly polarized luminescence spectrum (CPPL) of (*S, R*)-P-PtLA1 and (*R*, *S*)-*M*-PtLA1 in dichloromethane;
**FIG. 28** is a circularly polarized luminescence spectrum (CPPL) of (*S, R*)-*P*-PtLA3 and (*R*, *S*)-*M*-PtLA3 in dichloromethane;
**FIG. 29** is a circularly polarized luminescence spectrum (CPPL) of optically pure *M*-PtL1 and *P*-PtL1 in dichloromethane at a room temperature;
**FIG. 30** is a circularly polarized luminescence spectrum (CPPL) of (*S, R*)-*P*-PtLA3 and (*R*, *S*)-*M*-PtLA3 in dichloromethane;
**FIG. 31** is a circularly polarized luminescence spectrum (CPPL) of (*S, R*)-*P*-PtLJ1 and (*R*, *S*)-*M*-PtLJ1 in dichloromethane;
**FIG. 32** is a circularly polarized luminescence spectrum (CPPL) of *P*-PtLB3 and *M*-PtL B3 in dichloromethane;
**FIG. 33** is a circularly polarized luminescence spectrum (CPPL) of *P*-PtLB9 and *M*-PtL B9 in dichloromethane;
**FIG. 34** shows a high performance liguid chromatography (HPLC) spectrum of a mixture of (R, S)-M-PtLA1 and (S, R)-P-PtLA1, a high performance liguid chromatography spectrum of optically pure (*R*, *S*)-*M*-PtLA1; a high performance liguid chromatography spectrum of optically pure (*S, R*)-*P*-PtLA1, and a high performance liguid chromatography spectrum of sublimated (*R*, *S*)-*M*-PtLA1 from top to bottom;
**FIG. 35** is a thermogravimetric analysis curve of (*R*, *S*)-*M*-PtLA1;

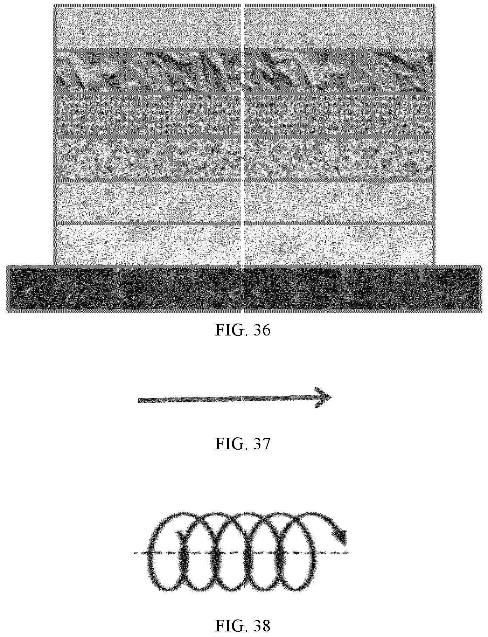

**FIG. 36** is a structural diagram of an organic light emitting element;
**FIG. 37** shows propagation of sunlight; and
**FIG. 38** shows propagation of a circularly polarized light beam.

### DETAILED DESCRIPTION

Contents of the present disclosure are described in detail below. Following description of constituent elements described below is sometimes based on representative embodiments or specific examples of the present disclosure, but the present disclosure is not limited to such embodiments or specific examples.

A specific example of a circularly polarized luminescence material of the present disclosure represented by above general formulas is illustrated below, however, it is not to be construed as limiting of the present disclosure.

The present disclosure may be more readily understood by reference to the following detailed description and examples included therein. Before compounds, devices, and/or methods of the present disclosure are disclosed and described, it should be understood that they are not limited to specific synthetic methods or specific reagents unless otherwise specified, as those may vary. It should also be understood that terminologies used in the present disclosure is for a purpose of describing particular aspects only but is not intended to be limiting. While any method and material similar or equivalent to those described herein may be used in this practice or test, example methods and materials are now described.

As used in the specification and appended claims, terms "a," "an," and "the" include plural referents unless the context clearly dictates otherwise. Thus, for example, reference to a "component" includes a mixture of two or more components.

A term "optional" or "optionally" as used herein means that a subsequently described event or condition may or may not occur, and that such description includes both instances in which the described event or condition occurs and instances in which it does not.

Components useful in preparing compositions of the present disclosure are disclosed, as well as the compositions themselves to be used in the methods disclosed in the present disclosure. These and other materials are disclosed, and it is to be understood that combinations, subsets, interactions, groups, etc. of these materials are disclosed, and that while specific references to each of various individual and general combinations and permutations of these compounds are not specifically disclosed, each of them is specifically contemplated and described. For example, if a particular compound is disclosed and discussed and many modifications that can be made to many molecules comprising the compound are discussed, each combination and permutation of the compound and possible modifications are specifically contemplated unless specifically indicated to the contrary. Thus, if examples of a type of molecules A, B, and C and a type of molecules D, E, and F, and combined molecules A-D are disclosed, it is contemplated that respective individual and general combination of meanings, A-E, A-F, B-D, B-E, B-F, C-D, C-E and C-F, are disclosed, even if they are not individually recited. Likewise, any subset or combination of these are also disclosed. For example, subgroups such as A-E, B-F, and C-E are also disclosed. This concept applies to all aspects of the present disclosure, including, but not limited to, steps in methods of making and using the composition. Thus, if there are various additional steps that can be performed, it should be understood that each of these additional steps can each be performed in a particular implementation of the method or a combination of implementations thereof.

A linking atom used in the present disclosure is capable of linking two groups, for example, linking N and C. The linking atom can optionally (if valence bonds allow) attach other chemical groups. For example, an oxygen atom will not have any other chemical group attachment since once two atoms (e. g., N or C) are bonded, the valence bonds are already fully used. In contrast, when the linking atom is carbon, two additional chemical groups can be attached to the carbon atom. Suitable chemical groups include, but are not limited to, hydrogen, hydroxyl, alkyl, alkoxy, =O, halogen, nitro, amine, amide, mercapto, aryl, heteroaryl, cycloalkyl, and heterocyclyl.

A term "cyclic structure" or the like as used herein refers to any cyclic chemical structure including, but not limited to, aryl, heteroaryl, cycloalkyl, cycloalkenyl, heterocyclyl, carbene and N-heterocyclic carbene.

A term "substituted" or the like as used herein includes all permissible substituents of an organic compound. Broadly, permissible substituents include cyclic and acyclic, branched and unbranched, carbocyclic and heterocyclic, aromatic and non-aromatic substituents of organic compounds. For example, exemplary substituents include following content. For suitable organic compounds, the permissible substituents may be one or more, the same or different. For a purposes of the present disclosure, heteroatom (e. g., nitrogen) can have a hydrogen substituent and/or any permissible substituent of the organic compound of the present disclosure satisfying valence bonds of the heteroatom. The present disclosure is not intended to be limiting in any way with the permissible substituents of the organic compound. As such, a term "substituted" or "substituted with" encompasses an implicit condition that such a substitution conforms to permissible valence bonds of a substituted atom and the substituent, and that the substitution results in stable compounds (e. g., compounds that do not spontaneously undergo conversion (e. g., by rearrangement, cyclization, elimination, etc.)). In certain aspects, individual substituents can be further optionally substituted (i.e., further substituted or unsubstituted) unless explicitly stated to the contrary.

In defining various terms, "R¹", "R²", "R3" and "R⁴" are used in the present disclosure as general symbols to denote various specific substituents. These symbols can be any substituent, not limited to those disclosed herein, which, when defined in an example as certain substituents, can also be defined in another example as some other substituents.

A term "alkyl" as used herein is a branched or unbranched saturated hydrocarbon group of 1 to 30 carbon atoms such as methyl, ethyl, n-propyl, isopropyl, n-butyl, isobutyl, sec-butyl, tert-butyl, n-pentyl, isopentyl, sec-pentyl, neopentyl, hexyl, heptyl, octyl, nonyl, decyl, dodecyl, tetradecyl, hexadecyl, eicosyl, tetracosyl and the like. The alkyl is acylic, while cyclic variants thereof are named "cycloalkyl". The alkyl may be branched or unbranched. The alkyl may also be substituted or unsubstituted. For example, the alkyl may be substituted with one or more group including, but not limit to, optionally substituted alkyl, cycloalkyl, alkoxy, amino, ether, halogen, hydroxy, nitro, silyl, sulfo-oxo and thiol as described herein. A "low alkyl" is an alkyl having 1 to 6 (e.g., 1 to 4) carbon atoms.

Throughout this specification, "alkyl" generally refer to unsubstituted alkyl However, substituted alkyl is also specifically mentioned in this present disclosure by determining specific substituents on the alkyl. For example, a term "halogenated alkyl" or "haloalkyl" specifically refers to an alkyl substituted with one or more halogens (e.g., fluorine, chlorine, bromine, or iodine). A term "alkoxyalkyl" specifically refers to an alkyl substituted with one or more alkoxy, as described below. A term "alkylamino" specifically refers to an alkyl substituted with one or more amino, as described below or the like. When "alkyl" is used in one case and specific terms such as "alkyl alcohol" are used in another case, it is not intended to imply that the term "alkyl" does not simultaneously refer to specific terms such as "alkyl alcohol" or the like.

This practice is also applicable for other groups described herein. That is, when terms such as "cycloalkyl" refer to unsubstituted cycloalkane moieties, the substituted moiety may additionally be specifically determined in the present disclosure. For example, a specifically substituted cycloalkyl may be referred to as, for example, "alkyl cycloalkyl". Similarly, a substituted alkoxy may be specifically referred to as, for example, "haloalkoxy" and a specific substituted alkenyl may be, for example, "enol" or the like.

The term "cycloalkyl" as used herein is a non-aromatic carbon-based ring of 3 to 30 carbon atoms consisting of at least three carbon atoms. Examples of cycloalkyl include, but are not limited to, cyclopropyl, cyclobutyl, cyclopentyl, cyclohexyl, cyclononyl, and the like. A term "heterocycloalkyl" is a type of cycloalkyl as defined above and is included in meaning of the term "cycloalkyl" in which at least one ring carbon atom is substituted by a heteroatom such as, but not limited to, a nitrogen, oxygen, sulfur or phosphorus atom. The cycloalkyl and heterocycloalkyl may be substituted or unsubstituted. The cycloalkyl and heterocycloalkyl may be substituted with one or more groups including, but not limited to, alkyl, cycloalkyl, alkoxy, amino, ether, halogen, hydroxy, nitro, silyl, sulfo-oxo and thiol as described herein.

A term "polyolefin group" as used herein refers to a group containing two or more CH₂ groups attached to each other. The "polyolefin group" may be express as -(CH₂)ₐ-, where "a" is an integer between 2 and 500.

Terms "alkoxy" and "alkoxy group" as used herein refer to an alkyl or cycloalkyl of 1 to 30 carbon atoms bonded by ether bonds. That is, "alkoxy" may be defined as -OR¹, where R¹ is an alkyl or cycloalkyl as defined above. "Alkoxy" further includes alkoxy polymers just described. That is, the alkoxy may be a polyether such as -OR¹-OR² or -OR¹-(OR²)ₐ-OR³, where "a" is an integer between 1 to 500, and R¹, R² and R³ are each independently an alkyl, a cycloalkyl, or a combination thereof.

A term "alkenyl" as used herein is a hydrocarbon of 2 to 30 carbon atoms with a structural formula containing at least one carbon-carbon double bond. An asymmetric structure such as (R¹R²)C=C(R³R⁴) contains E and Z isomers. This can be inferred in a structural formula of the present disclosure in which an asymmetric olefin is present, or it can be explicitly expressed by a bond symbol C=C. The alkenyl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxylic acid, ester, ether, halogen, hydroxy, ketone, azido, nitro, silyl, sulfo-oxo or thiol as described herein

A term "cycloalkenyl" as used herein is a non-aromatic carbon-based ring of 3 to 30 carbon atoms which consists of at least 3 carbon atoms and contains at least one carbon-carbon double bond. i.e. C=C. Examples of cycloalkenyl include, but are not limited to, cyclopropenyl, cyclobutenyl, cyclopentenyl, cyclopentadienyl, cyclohexenoyl, cycloheptene, and the like. A term "heterocyclic alkenyl" is a type of cycloalkenyl as defined above and is included in meaning of the term "cycloalkenyl" in which at least one carbon atom of the ring is substituted with a heteroatom such as, but not limited to, a nitrogen, oxygen, sulfur or phosphorus atom. The cycloalkenyl and heterocyclic alkenyl may be substituted or unsubstituted. The cycloalkenyl and heterocyclic alkenyl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxylic acid, ester, ether, halogen, hydroxy, ketone, azido, nitro, silyl, sulfo-oxo or thiol as described herein.

A term "alkynyl" as used herein is a hydrocarbon having 2 to 30 carbon atoms and having a structural formula containing at least one carbon-carbon triple bond. The alkynyl may be unsubstituted or substituted with one or more groups including, but not limited to, alkyl, cycloalkyl, alkoxy, alkenyl, cycloalkenyl, alkynyl, cycloalkynyl, aryl, heteroaryl, aldehyde, amino, carboxylic acid, ester, ether, halogen, hydroxy, ketone, azido, nitro, silyl, sulfo-oxo or thiol as described herein.

A term "cycloalkynyl" as used herein is a non-aromatic carbon-based ring that contains at least 7 carbon atoms and contains at least one carbon-carbon triple bond. Examples of cycloalkynyl include, but are not limited to, cycloheptynyl, cyclooctynyl, cyclononynyl, and the like. A term "heterocyclic alkynyl" is a cycloalkenyl as defined above and is included within meaning of the term "cycloalkynyl" in which at least one of the carbon atoms of the ring is replaced by a heteroatom such as, but not limited to, a nitrogen, oxygen, sulfur or phosphorus atom. The cycloalkynyl and heterocyclic alkynyl may be substituted or unsubstituted. The cycloalkynyl and heterocyclic alkynyl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxylic acid, ester, ether, halogen, hydroxy, ketone, azido, nitro, silyl, sulfo-oxo or thiol as described herein.

A term "aryl" as used herein refers to a group containing 60 or less carbon atoms of any carbon-based aromatic group, including but not limited to benzene, naphthalene, phenyl, biphenyl, phenoxybenzene, and the like. The term "aryl" further includes "heteroaryl", which is defined as a group containing an aromatic group having at least one heteroatom within a ring. Examples of heteroatoms include, but are not limited to, a nitrogen, oxygen, sulfur, or phosphorus atom. Likewise, a term "non-heteroaryl" (which is also included in the term "aryl") defines an aromatic-containing group that is free of heteroatoms. The aryl may be substituted or unsubstituted. The aryl may be substituted with one or more groups including, but not limited to, alkyl, cycloalkyl, alkoxy, alkenyl, cycloalkenyl, alkynyl, cycloalkynyl, aryl, heteroaryl, aldehyde, amino, carboxylic acid, ester, ether, halogen, hydroxy, ketone, azido, nitro, silyl, sulfo-oxo or thiol as described herein. A term "biaryl" is a particular type of aryl and is included in definition of "aryl". The biaryl refers to two aryl joined together by a fused ring structure, as in naphthalene, or two aryl joined by one or more carbon-carbon bonds, as in biphenyl.

A term "aldehyde" as used herein is represented by a formula -C(O)H. Throughout this specification, "C(O)" is a short form of carbonyl (i.e., C=O).

A term "amine" or "amino" as used herein is represented by the formula -NR¹R², where R¹ and R² may independently be selected from hydrogen, alkyl, cycloalkyl, alkenyl, cycloalkenyl, alkynyl, cycloalkynyl, aryl or heteroaryl.

A term "alkylamino" as used herein is represented by a formula -NH(-alkyl), where the alkyl is as described in the present disclosure. Representative examples include, but are not limited to, methylamino, ethylamino, propylamino, isopropylamino, butylamino, isobutylamino, sec-butylamino, tert-butylamino, pentylamino, isopentylamino, tert-pentylamino, hexylamino and the like.

A term "dialkylamino" as used herein is represented by a formula -N(-alkyl)₂, where the alkyl is as described in the present disclosure. Representative example include, but are not limited to, dimethylamino, diethylamino, dipropylamino, diisopropylamino, dibutylamino, diisobutylamino, di-sec-butylamino, di-tert-butylamino, dipentylamino, diisopentylamino, di-tert-pentylamino, dihexylamino, N-ethyl-N-methylamino, N-methyl-N-propylamino, N-ethyl-N-propylamino and the like.

A term "carboxylic acid" as used herein is represented by a formula -C(O)OH.

A term "ester" as used herein is represented by a formula -OC(O)R¹ or -C(O)OR¹, where R¹ may be alkyl, cycloalkyl, alkenyl, cycloalkenyl, alkynyl, cycloalkynyl, aryl or heteroaryl as described herein. A term "polyester" as used herein is represented by a formula -(R¹O(O)C-R²-C(O)O)ₐ- or -(R¹O(O)C-R²-OC(O))ₐ-, where R¹ and R² can independently be alkyl, cycloalkyl, alkenyl, cycloalkenyl, alkynyl, cycloalkynyl, aryl or heteroaryl as described herein and "a" is an integer between 1 to 500. The term "polyester" is used to describe groups produced by reaction between a compound having at least two carboxyl and a compound having at least two hydroxy.

A term "ether" as used herein is represented by a formula R¹OR², where R¹ and R² may independently be alkyl, cycloalkyl, alkenyl, cycloalkenyl, alkynyl, cycloalkynyl, aryl or heteroaryl as described herein. A term "polyether" as used herein is represented by a formula -(R¹O-R²O)ₐ₋, where R¹ and R²may independently be alkyl, cycloalkyl, alkenyl, cycloalkenyl, alkynyl, cycloalkynyl, aryl or heteroaryl as described herein and "a" is an integer between 1 to 500. Examples of polyether groups include polyethylene oxide, polypropylene oxide and polybutylene oxide.

A term "halogen" as used herein refers to halogen fluorine, chlorine, bromine and iodine.

A term "heterocyclyl" as used herein refers to monocyclic and polycyclic non-aromatic ring systems, and "heteroaryl" as used herein refers to monocyclic and polycyclic aromatic ring systems of not more than 60 carbon atoms, where at least one of ring members is not carbon. This term includes azetidinyl, dioxane, furyl, imidazolyl, isothiazolyl, isoxazolyl, morpholinyl, oxazolyl (oxazolyl including 1,2,3- oxadiazole, 1,2,5- oxadiazole and 1,3,4- oxadiazole), piperazinyl, piperidyl, pyrazinyl, pyrazolyl, pyridazinyl, pyridinyl, pyrimidinyl, pyrrolyl, pyrrolidinyl, tetrahydrofuran, tetrahydropyranyl, tetrazinyl including 1,2,4, 5- tetrazinyl, tetrazolyl including 1,2,3,4- tetrazolyl and 1,2,4,5- tetrazolyl, thiadiazole including 1,2,3- thiadiazole, 1,2,5- thiadiazole and 1,3,4- thiadiazole, thiazolyl, thienyl, triazinyl including 1,3,5- triazinyl and 1,2,4- triazinyl, thiadiazolyl including 1,2,3- triazolinyl and 1,3,4- triazolinyl, and the like.

A term "hydroxyl" as used herein is represented by a formula -OH.

A term "ketone" as used herein is represented by a formula R¹C(O)R², where R¹ and R² may independently be alkyl, cycloalkyl, alkenyl, cycloalkenyl, alkynyl, cycloalkynyl, aryl or heteroaryl as described herein.

A term "azido" as used herein is represented by a formula -N₃.

A term "nitro" as used herein is represented by a formula -NO₂.

A term "nitrile" as used herein is represented by a formula -CN.

A term "silyl" as used herein is represented by a formula -SiR¹R²R³, where R¹, R² and R³ may independently be alkyl, cycloalkyl, alkoxy, alkenyl, cycloalkenyl, alkynyl, cycloalkynyl, aryl or heteroaryl as described herein.

A term "sulfo-oxo" as used herein is represented by a formula -S(O)R¹, -S(O)₂R¹, -OS(O)₂R¹ or -OS(O)₂OR¹, where R1 may be alkyl, cycloalkyl, alkenyl, cycloalkenyl, alkynyl, cycloalkynyl, aryl or heteroaryl as described herein. Throughout this specification, "S(O)" is a short form of S=O. A term "sulfonyl" as used herein refers to a sulfo-oxo represented a formula -S(O)₂R¹, where R¹ may be alkyl, cycloalkyl, alkenyl, cycloalkenyl, alkynyl, cycloalkynyl, aryl or heteroaryl. A term "sulfone" as used herein is represented by a formula R¹S(O)₂R², where R¹ and R² may independently be alkyl, cycloalkyl, alkenyl, cycloalkenyl, alkynyl, cycloalkynyl, aryl or heteroaryl as described herein. A term "sulfoxide" as used herein is represented by a formula R¹S(O)R², where R¹ and R² may independently be alkyl, cycloalkyl, alkenyl, cycloalkenyl, alkynyl, cycloalkynyl, aryl or heteroaryl as described herein.

A term "thiol" as used herein is represented by a formula -SH.

"R¹", "R²", "R³", "Rⁿ" (where n is an integer) as used herein may independently have one or more of groups listed above. For example, if R¹ is linear alkyl, one hydrogen atom of the alkyl may be optionally substituted with hydroxy, alkoxy, alkyl, halogen or the like. Depending on a selected group, a first group may be incorporated within a second group, or the first group may be side linked (i.e., connected) to the second group. For example, for a phrase "an alkyl including amino", the amino may be incorporated within a main chain of the alkyl. Alternatively, the amino may be linked to the main chain of the alkyl. Nature of the selected group may determine whether the first group is embedded in or linked to the second group.

The compound of that present disclosure may contain an "optionally substituted" moiety. In general, a term "substituted" (whether or not a term "optionally" exists before it) means that one or more hydrogen atoms of a given moiety are substituted with a suitable substituent. Unless otherwise stated, an "optionally substituted" group may have suitable substituents at each of substitutable positions of the group, and when more than one position in any given structure may be substituted with more than one substituent selected from groups designated, the substituents may be the same or different at each position. Combinations of substituents contemplated in the present disclosure are preferably combinations that form stable or chemically feasible compounds. It is also contemplated that in certain aspects, respective substituents may be further optionally substituted (i. e., further substituted or unsubstituted) unless explicitly stated to the contrary.

A structure of the compound may be represented by a following formula: 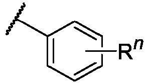
which is understood as equivalent to a following formula: 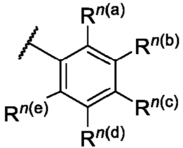
where n is typically an integer. That is, Rⁿ is understood to indicate five individual substituents R^{*n*(a)}, R^{*n*(b)}, R^{*n*(c)}, R^{*n*(d)} and R^{*n*(e)}. "individual substituents" means that each R substituent may be defined independently. For example, if R^{*n*(a)} is halogen in one case, R^{*n*(b)} is not necessarily halogen in this case.

R¹, R², R³, R⁴, R⁵, R⁶, etc. are mentioned several times in chemical structures and units disclosed and described in this disclosure. Any description of R¹, R², R³, R⁴, R⁵, R⁶, etc. in the specification is applicable to any structure or unit referring to R¹, R², R³, R⁴, R⁵, R⁶, etc. respectively, unless otherwise specified.

A term "fused ring" as used herein means that two adjacent substituents may be fused to form a six-member aromatic ring, a heteroaromatic ring, such as a benzene ring, a pyridine ring, a pyrazine ring, a pyridazine ring, a m-diazepine ring, and the like, as well as a saturated six or seven-member carbocyclic ring or carboheterocyclic ring, and the like.

Unless otherwise specified, all commercial reagents involved in following tests are used directly after purchase and are not further purified. Both hydrogen and carbon nuclear magnetic resonance spectra were measured in deuterated chloroform (CDCl₃) or deuterated dimethyl sulfoxide (DMSO-d₆), in which the hydrogen spectra is made using a 400 or 500 MHz nuclear magnetic resonance spectrometer and the carbon spectra is made using a 100 or 126 MHz nuclear magnetic resonance spectrometer, with chemical shifts being based on tetramethylsilane (TMS) or residual solvent. If CDCl₃ is used as a solvent, TMS (δ= 0.00 ppm) and CDCl₃ (δ = 77.00 ppm) are used as internal standards for the hydrogen and carbon spectra, respectively. If DMSO-d₆ is used as a solvent, TMS (δ= 0.00 ppm) and DMSO-d₆ (δ = 39.52 ppm) are used as internal standards for the hydrogen and carbon spectra, respectively. Following abbreviations (or combinations) are used to explain hydrogen peaks: s indicates a single peak, d indicates a double peak, t indicates a triple peak, q indicates a quadruple peak, p indicates a quintic peak, m indicates a multiple peaks, and br indicates a wide peak. A high-resolution mass spectrum was measured on an ESI-QTOF mass spectrometer from Applied Biosystems, with an ionization mode of samples being electrospray ionization.

**Example 1, not in accordance with the present invention and provided for illustrative purposes only:** synthesis route of tetradentate cyclometalated platinum (II) complex **(*S*, R)-*P*-PtLA1** as follow. 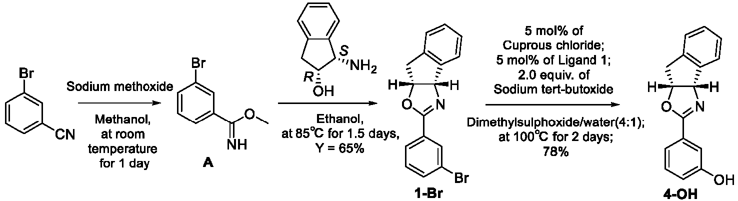 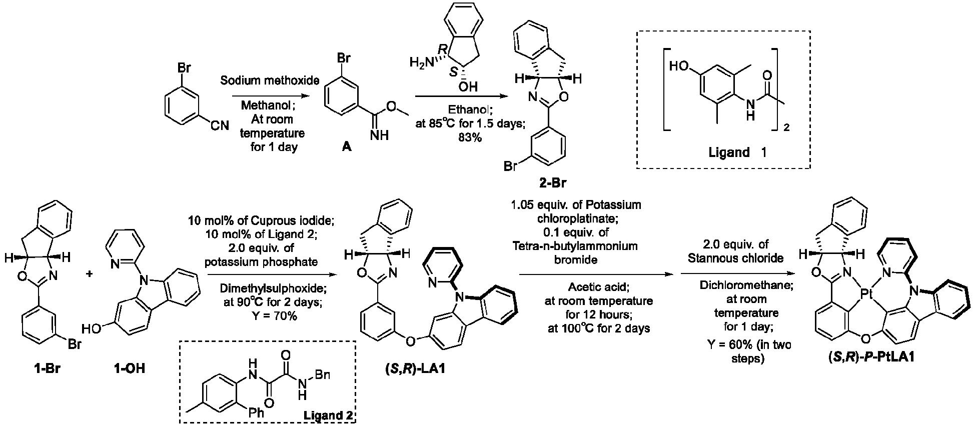
(1) Synthesis of intermediate 1-Br: 3-Bromobenzonitrile (10 g, 54.94 mmol, 1.0 equiv.) and sodium methoxide (297 mg, 5.49 mmol, 0.1 equiv.) were sequentially added into a single-necked flask equipped with a magnetic rotor and stirred at a room temperature for 1 day. Acetic acid was added until solid disappeared, and solvent was distilled off at a reduced pressure so as to obtain crude A, then (1S,2R)-1- amino-2,3- dihydro -1H- indene -2-ol (4.10 g, 27.47 mmol, 0.5 equiv.) and anhydrous ethanol (30 mL) were added, and this mixture was reacted with stiring in an oil bath at 85 °C for 1.5 days, cooled to the room temperature, and the solvent was distilled off at a reduced pressure. The crude was separated and purified by a silica gel chromatography column with an eluent of petroleum ether/ethyl acetate of 50: 1 to 10: 1 so as to obtain a product **1-Br,** 5.60 g as a white solid, with a yield of 65%. ¹H NMR (500 MHz, CDCl₃): δ 3.40 (d, *J* = 18.0 Hz, 1H), 3.51-3.56 (m, 1H), 5.57 (t, *J* = 7.5 Hz, 1H), 7.79 (d, *J* = 8.0 Hz, 1H), 7.26-7.31 (m, 4H), 7.59-7.63 (m, 2H), 7.96 (d, *J* = 8.0 Hz, 1H), 8.12 (t, *J* = 2.0 Hz, 1H).
(2) Synthesis of intermediate **4-OH:** chiral bromine (1.5 g, 4.77 mmol, 1.0 equiv.), cuprous chloride (24 mg, 0.24 mmol, 5 mol%), ligand 1 (87 mg, 0.24 mmol, 5 mol%) and sodium tert-butoxide (917 mg, 9.55 mmol, 2.0 equiv.) were sequentially added into a dry sealed tube with a magnetic rotor. Nitrogen was purged for three time, and dimethyl sulfoxide (8 mL) and deionized water (2 mL) were added under nitrogen protection. The sealed tube was placed in an oil bath at 110°C, stirred for reacting for 2 days, cooled to the room temperature, and then this mixture was washed with water, dilute hydrochloric acid was added to adjust to neutral or weak acid, then sodium bicarbonate was added to adjust to weak base, ethyl acetate was added for extraction, with an aqueous layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **4-OH,** 934 mg as brown solid, with a yield of 78%. ¹H NMR (500 MHz, DMSO-*d₆*): δ 3.22 (d, *J* = 18.0 Hz, 1H), 3.46-3.51 (m, 1H), 5.47-5.50 (m, 1H), 5.68 (d, *J* = 8.0 Hz, 1H), 6.87-6.90 (m, 1H), 7.21-7.3 (m, 6H), 7.43-7.44 (m, 1H), 9.67 (s, 1H).
(3) Synthesis of intermediate chiral **2-Br:** 3-Bromobenzonitrile (10 g, 54.94 mmol, 1.0 equiv.) and sodium methoxide (297 mg, 5.49 mmol, 0.1 equiv.) were sequentially added into a single-necked flask with a magnetic rotor and stirred at a room temperature for 1 day. Acetic acid was added until solid disappeared, and solvent was distilled off at a reduced pressure so as to obtain crude A, then (1*R*,2*S*)-1- amino-2,3- dihydro -1H- indene -2-ol (3.28 g, 21.97 mmol, 0.4 equiv.) and anhydrous ethanol (30 mL) were added, and this mixture was reacted with stiring in an oil bath at 85°C for 1.5 days, cooled to the room temperature, and the solvent was distilled off at a reduced pressure. The crude was separated and purified by a silica gel chromatography column with an eluent of petroleum ether/ethyl acetate of 50: 1 to 10: 1 so as to obtain a product **2-Br,** 5.74 g as a white solid, with a yield of 83%. ¹H NMR (500 MHz, CDCl₃): δ 3.41 (d, *J* = 18.5 Hz, 1H), 3.52-3.57 (m, 1H), 5.59 (s, 1H), 5.81 (d, *J =* 3.0 Hz, 1H), 7.27-7.31 (m, 4H), 7.60-7.65 (m, 2H), 7.98-8.00 (m, 1H), 8.13 (s, 1H).
(4) Synthesis of ligand **(*S*, R)-LA1:** 1-Br (579 mg, 1.84 mmol, 1.2 equivalent), 1-OH (400 mg, 1.54 mmol, 1.0 equivalent) and cuprous iodide (29 mg, 0.15 mmol, 10 mol%), ligand 2 (50 mg, 0.15 mmol, 10 mol%) and potassium phosphate (654 mg, 3.08 mmol, 2.0 equiv.) were sequentially added into a dry sealed tube with a magnetic rotor. Nitrogen was purged for three time, and dimethyl sulfoxide (10 mL) was added under nitrogen protection. The sealed tube was placed in an oil bath at 90°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain the ligand **(S, R)-LA1,** 533 mg as white solid, with a yield of 70%. ¹H NMR (500 MHz, DMSO-*d₆*): δ 3.19 (d, *J* = 17.5 Hz, 1H), 3.42-3.47 (m, 1H), 5.46-5.49 (m, 1H), 5.65 (d, *J* = 7.5 Hz, 1H), 7.03 (dd, *J* = 8.5, 2.5 Hz, 1H), 7.19-7.28 (m, 4H), 7.35-7.40 (m, 3H), 7.42-7.47 (m, 3H), 7.50 (d, *J* = 2.0 Hz, 1H), 7.57-7.59 (m, 1H), 7.76-7.79 (m, 2H), 8.04-8.07 (m, 1H), 8.23 (d, *J* = 7.5 Hz, 1H), 8.27 (d, *J* = 8.0 Hz, 1H), 8.65-8.66 (m, 1H).
(5) Synthesis of **(*S*, *R*)-*P*-PtLA1: (*S*, *R*)-LA1** (150 mg, 0.30 mmol, 1.0 equiv.), potassium chloroplatinate (132 mg, 0.32 mmol, 1.05 equiv.), tetra-n-butylammonium bromide (10 mg, 0.030 mmol, 0.1 equiv.) were sequentially added into a 100 mL dry three-necked flask with a magnetic rotor and a condenser tube, followed by nitrogen purge for three times and addition of acetic acid (25 mL) pre-purged with nitrogen. After a reaction solution was bubbled with nitrogen for 30 minutes, it was stirred at the room temperature for 12 hours, and then was reacted with stirring at 100°C for 2 days, cooled to the room temperature, and solvent was distilled off at a reduced pressure, and stannous chloride (114 mg, 0.6 mmol, 2.0 equiv.) and dichloromethane (30 mL) were added and stirred at the room temperature for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 1:1 so as to obtain a product **(*S*, *R*)-*P*-PtLA1,** 126 mg as pale primrose solid, with a yield of 60%. ¹H NMR (500 MHz, DMSO-*d₆*): δ 3.49 (d, *J* = 18.5 Hz, 1H), 3.62-3.67 (m, 1H), 6.05 (t, *J* = 7.5 Hz, 1H), 6.26 (d, *J* = 7.5 Hz, 1H), 7.01 (d, *J* = 7.5 Hz, 1H), 7.10 (t, *J* = 7.5 Hz, 1H), 7.12-7.18 (m, 3H), 7.20-7.24 (m, 2H), 7.35 (d, *J* = 7.5 Hz, 1H), 7.41-7.44 (m, 2H), 7.48-7.52 (m, 1H), 7.88 (d, *J* = 8.0 Hz, 1H), 8.15-8.17 (m, 1H), 8.20 (d, *J* = 8.5 Hz, 1H), 8.26-8.29 (m, 1H), 8.36 (d, *J* = 8.5 Hz, 1H), 9.57 (dd, *J* = 6.0, 1.5 Hz, 1H).

**Example 2, not in accordance with the present invention and provided for illustrative purposes only:** synthesis of tetradentate cyclometalated platinum (II) complex **(*R*, *S*)-*M*-PtLA1** as follow. 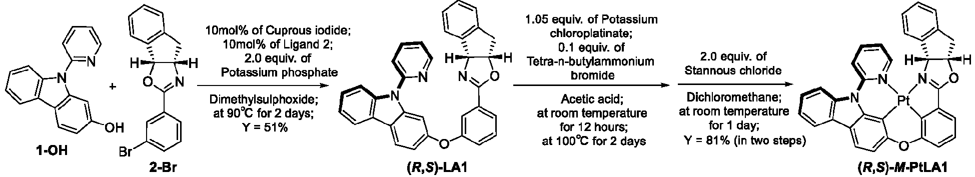
(1) Synthesis of ligand **(*R*, *S*)-LA1:** 2-Br (3.19 g, 10.14 mmol, 1.2 equivalent), 1-OH (2.20 g, 8.45 mmol, 1.0 equivalent) and cuprous iodide (29 mg, 0.84 mmol, 10 mol%), ligand 2 (276 mg, 0.84 mmol, 10 mol%) and potassium phosphate (3.59 g, 16.90 mmol, 2.0 equiv.) were sequentially added into a dry sealed tube with a magnetic rotor. Nitrogen was purged for three times, and dimethyl sulfoxide (40 mL) was added under nitrogen protection. The sealed tube was placed in an oil bath at 100°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*R*, *S*)-LA1,** 2.12 g as a white solid, with a yield of 51%. ¹H NMR (500 MHz, DMSO-*d₆*): δ 3.19 (d, *J* = 18.0 Hz, 1H), 3.42-3.47 (m, 1H), 5.46-5.49 (m, 1H), 5.65 (d, *J* = 7.5 Hz, 1H), 7.03 (dd, *J* = 7.5, 2.0 Hz, 1H), 7.19-7.28 (m, 4H), 7.34-7.40 (m, 3H), 7.43-7.48 (m, 3H), 7.50 (d, *J* = 2.0 Hz, 1H), 7.57-7.59 (m, 1H), 7.77-7.79 (m, 2H), 8.04-8.08 (m, 1H), 8.24 (d, *J* = 8.0 Hz, 1H), 8.28 (d, *J* = 8.5 Hz, 1H ), 8.65-8.67 (m, 1H).
(2) Synthesis of **(*R, S*)-*M*-PtLA1: (*R, S*)-LA1** (900 mg, 1.82 mmol, 1.0 equiv.), potassium chloroplatinate (795 mg, 1.92 mmol, 1.05 equiv.), tetra-n-butylammonium bromide (59 mg, 0.182 mmol, 0.1 equiv.) were sequentially added into a 100 mL dry three-necked flask with a magnetic rotor and a condenser tube, followed by nitrogen purge for three times and addition of acetic acid (110 mL) pre-purged with nitrogen. After a reaction solution was bubbled with nitrogen for 30 minutes, it was stirred at the room temperature for 12 hours, and then was reacted with stirring at 100 °C for 2 days, cooled to the room temperature, and solvent was distilled off at a reduced pressure. The crude was separated and purified by a silica gel chromatography column with an eluent of petroleum ether/dichloromethane of 1:1 so as to obtain a product **(*R*, *S*)-*M*-PtLA1,** 1.01 g as a pale primrose solid, with a yield of 81%. ¹H NMR (500 MHz, DMSO-*d₆*): δ 3.49 (d, *J* = 18.0 Hz, 1H), 3.62-3.67 (m, 1H), 6.04 (t, *J* = 7.0 Hz, 1H), 6.27 (d, *J* = 7.0 Hz, 1H), 7.01 (d, *J* = 8.0 Hz, 1H), 7.10 (t, *J* = 7.0 Hz, 1H), 7.12-7.18 (m, 3H), 7.21-7.24 (m, 2H), 7.35 (d, *J* = 7.0 Hz, 1H), 7.41-7.44 (m, 2H), 7.48-7.52 (m, 1H), 7.88 (d, *J* = 8.5 Hz, 1H), 8.16-8.18 (m, 1H), 8.20 (d, *J* = 8.5 Hz, 1H), 8.26-8.30 (m, 1H), 8.37 (d, *J* = 8.5 Hz, 1H), 9.57 (m, 1H).

**Example 3, not in accordance with the present invention and provided for illustrative purposes only:** synthesis of tetradentate cyclometalated platinum (II) complex **(*S*, R)-*P*-PtLA2.** 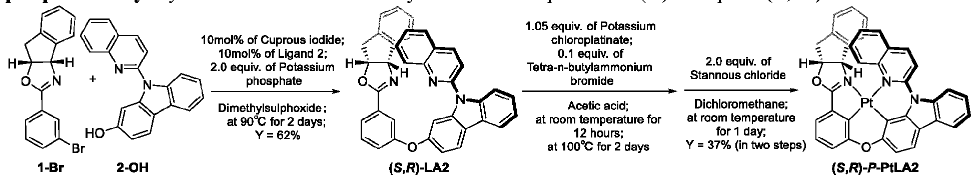
(1) Synthesis of ligand **(S, *R*)-LA2:** 1-Br (1.82 g, 5.80 mmol, 1.2 equivalent), 2-OH (1.50 g, 4.83 mmol, 1.0 equivalent) and cuprous iodide (91 mg, 0.48 mmol, 10 mol%), ligand 2 (157 mg, 0.48 mmol, 10 mol%) and potassium phosphate (2.05 g, 9.05 mmol, 2.0 equiv.) were sequentially added into a dry sealed tube with a magnetic rotor. Nitrogen was purged for three times, and dimethyl sulfoxide (30 mL) was added under nitrogen protection. The sealed tube was placed in an oil bath at 100°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 20: 1 to 5: 1 so as to obtain a product **(*S*, *R*)-LA2,** 1.63 g as a brown solid, with a yield of 62%. ¹H NMR (600 MHz, CDCl₃): δ 3.45 (d, *J =* 18.6 Hz, 1H), 3.53-3.56 (m, 1H), 5.66-5.69 (m, 1H), 5.88 (s, 1H), 7.01 (dd, *J =* 8.4, 1.8 Hz, 1H), 7.24-7.31 (m, 6H), 7.35-7.40 (m, 2H), 7.44-7.47 (m, 1H), 7.56-7.59 (m, 1H), 7.63 (t, *J* = 1.8 Hz, 1H), 7.70 (d, 8.4 Hz, 1H), 7.73-7.75 (m, 1H), 7.77 (d, *J* = 8.0 Hz, 1H), 7.87-7.88 (m, 1H), 7.97 (d, *J* = 8.4 Hz, 1H). 8.07 (d, *J* = 8.4 Hz, 1H), 8.08-8.10 (m, 2H), 8.32 (d, *J* = 8.0 Hz, 1H).
(2) Synthesis of **(S, R)-*P*-PtLA2: (*S*, *R*)-LA2** (200 mg, 0.37 mmol, 1.0 equiv.), potassium chloroplatinate (161 mg, 0.39 mmol, 1.05 equiv.), tetra-n-butylammonium bromide (12 mg, 0.037 mmol, 0.1 equiv.) were sequentially added into a 100 mL dry three-necked flask with a magnetic rotor and a condenser tube, followed by nitrogen purge for three times and addition of acetic acid (22 mL) pre-purged with nitrogen. After a reaction solution was bubbled with nitrogen for 30 minutes, it was stirred at the room temperature for 12 hours, and then was reacted with stirring at 120°C for 2 days, cooled to the room temperature, and solvent was distilled off at a reduced pressure, and stannous chloride (140 mg, 0.74 mmol, 2.0 equiv.) and dichloromethane (30 mL) were added and stirred at the room temperature for 1 day. The reaction solution was washed with water, the aqueous phase was extracted three times with dichloromethane, organic phases were combined, dried with anhydrous sodium sulfate and filtered, and solvent was distilled off at a reduced pressure. The crude was separated and purified by a silica gel chromatography column with an eluent of petroleum ether/dichloromethane of 1:1 so as to obtain a product **(*S*, *R*)-*P*-PtLA2,** 101 mg as pale primrose solid, with a yield of 37%. ¹H NMR (600 MHz, DMSO-*d₆*): δ3.47 (d, *J =* 18.0 Hz, 1H), 3.49-3.51 (m, 1H), 5.12 (d, *J* = 7.8 Hz, 1H), 5.32 (d, *J* = 4.8 Hz, 1H), 5.93-5.95 (m, 1H), 6.05 (d, *J* = 8.4 Hz, 1H), 6.93 (d, *J* = 7.2 Hz, 1H), 7.10 (t, *J* = 7.2 Hz, 1H), 7.15 (t, *J* = 8.4 Hz, 1H), 7.17-7.18 (m, 1H), 7.21 (d, *J* = 7.2 Hz, 1H), 7.23-7.25 (m, 1H), 7.45 (t, *J* = 7.8 Hz, 1H), 7.53-7.56 (m, 1H), 7.66-7.69 (m, 1H), 7.78 (d, *J* = 8.4 Hz, 1H), 8.04-8.06 (m, 1H), 8.13 (dd, *J* = 7.8, 1.2 Hz, 1H), 8.18 (dd, *J* = 7.8, 1.2 Hz, 1H), 8.36 (d, *J* = 7.8 Hz, 1H), 8.66 (d, *J* = 9.0 Hz, 1H), 8.82 (d, *J* = 9.0 Hz, 1H), 9.40 (d, *J*= 8.4 Hz, 1H).

**Example 4, not in accordance with the present invention and provided for illustrative purposes only:** synthesis of tetradentate cyclometalated platinum (II) complex **(*R*, *S*)-*M*-PtLA2** as follow. 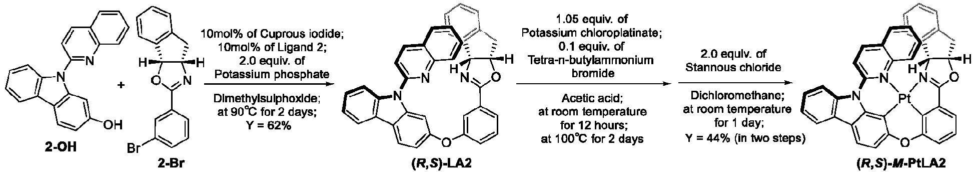
(1) Synthesis of ligand **(R, *S***)-LA2: 2-Br (1.38 g, 4.40 mmol, 1.2 equivalent), 2-OH (1.14 g, 3.67 mmol, 1.0 equivalent) and cuprous iodide (70 mg, 0.37 mmol, 10 mol%), ligand 2 (121 mg, 0.37 mmol, 10 mol%) and potassium phosphate (1.56 g, 7.34 mmol, 2.0 equiv.) were sequentially added into a dry sealed tube with a magnetic rotor. Nitrogen was purged for three times, and dimethyl sulfoxide (20 mL) was added under nitrogen protection. The sealed tube was placed in an oil bath at 90°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*R*, *S*)-LA2,** 1.23 g as a white solid, with a yield of 62%. 1H NMR (500 MHz, CDCl₃): δ 3.44 (d, J = 18.0 Hz, 1H), 3.53-3.58 (m, 1H), 5.66-5.70 (m, 1H), 5.85-5.88 (m, 1H), 7.01 (dd, J = 8.0, 2.0 Hz, 1H), 7.24-7.31 (m, 6H), 7.35-7.47 (m, 3H), 7.56-7.59 (m, 1H), 7.62 (t, J = 2.0 Hz, 1H), 7.70 (d, J = 2.0 Hz, 1H), 7.72-7.78 (m, 2H), 7.88 (d, J = 7.5 Hz, 1H), 7.96 (d, J = 8.0 Hz, 1H), 8.06-8.10 (m, 3H), 8.31 (d, J = 8.5 Hz, 1H).
(2) Synthesis of **(*R*, *S*)-*M*-PtLA2: (*R, S*)-LA2** (200 mg, 0.37 mmol, 1.0 equiv.), potassium chloroplatinate (161 mg, 0.39 mmol, 1.05 equiv.), tetra-n-butylammonium bromide (12 mg, 0.037 mmol, 0.1 equiv.) were sequentially added into a 100 mL dry three-necked flask with a magnetic rotor and a condenser tube, followed by nitrogen purge for three times and addition of acetic acid (22 mL) pre-purged with nitrogen. After a reaction solution was bubbled with nitrogen for 30 minutes, it was stirred at the room temperature for 12 hours, and then was reacted with stirring at 110 °C for 2 days, cooled to the room temperature, and solvent was distilled off at a reduced pressure, and stannous chloride (140 mg, 0.74 mmol, 2.0 equiv.) and dichloromethane (30 mL) were added and stirred at the room temperature for 1 day. The reaction solution was washed with water, the aqueous phase was extracted three times with dichloromethane, organic phases were combined, dried with anhydrous sodium sulfate and filtered, and solvent was distilled off at a reduced pressure. The crude was separated and purified by a silica gel chromatography column with an eluent of petroleum ether/dichloromethane of 1:1 so as to obtain a product **(*R*, *S*)-*M*-PtLA2,** 119 mg as pale primrose solid, with a yield of 44%. ¹H NMR (500 MHz, DMSO-*d₆*): δ 3.29-3.34 (m, 1H), 3.45-3.50 (m, 1H), 5.12 (d, *J* = 7.5 Hz, 1H), 5.94 (d, *J* = 7.0 Hz, 1H), 6.04 (d, *J* = 8.0 Hz, 1H), 6.94 (t, *J* = 7.5 Hz, 1H), 7.10 (t, *J* = 7.5 Hz, 1H), 7.15 (d, *J* = 8.0 Hz, 1H), 7.17-7.21 (m, 3H), 7.23-7.25 (m, 1H), 7.45 (t, *J* = 7.5 Hz, 1H), 7.53-7.56 (m, 1H), 7.68 (t, *J* = 7.0 Hz, 1H), 7.78 (d, *J* = 8.0 Hz, 1H), 8.04-8.07 (m, 1H), 8.12-8.14 (m, 1H), 8.17-8.19 (m, 1H), 8.36 (d, *J* = 8.0 Hz, 1H), 8.66 (d, *J* = 9.5 Hz, 1H), 8.82 (d, *J* = 9.5 Hz, 1H), 9.40 (d, *J* = 8.5 Hz, 1H).

**Example 5, not in accordance with the present invention and provided for illustrative purposes only:** synthesis of tetradentate cyclometalated platinum (II) complex **(*S*, R)-*P*-PtLA3.** 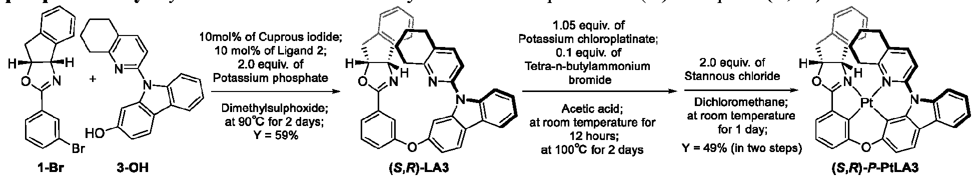
(1) Synthesis of ligand **(S, *R*)-LA3:** 1-Br (477 mg, 1.52 mmol, 1.2 equivalent), 3-OH (400 mg, 1.27 mmol, 1.0 equivalent) and cuprous iodide (25 mg, 0.13 mmol, 10 mol%), ligand 2 (43 mg, 0.13 mmol, 10 mol%) and potassium phosphate (539 mg, 2.54 mmol, 2.0 equiv.) were sequentially added into a dry sealed tube with a magnetic rotor. Nitrogen was purged for three times, and dimethyl sulfoxide (10 mL) was added under nitrogen protection. The sealed tube was placed in an oil bath at 100°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/dichloromethane/ethyl acetate of 5:1:1 so as to obtain a product **(S, *R*)-LA3,** 412 mg as brown solid, with a yield of 59%. ¹H NMR (500 MHz, CDCl₃): δ 1.83-1.94 (m, 4H), 2.82 (t, *J =* 6.0 Hz, 2H), 2.94 (d, *J* = 6.5 Hz, 2H), 3.31-3.35 (d, *J* = 17.5 Hz, 1H), 3.44-3.49 (m, 1H), 5.43-5.46 (m, 1H), 5.71 (d, *J* = 8.0 Hz, 1H), 6.96 (dd, *J* = 8.5, 2.5 Hz, 1H), 7.11-7.14 (m, 1H), 7.24-7.27 (m, 7H), 7.28-7.30 (m, 2H), 7.32 (d, *J* = 8.0 Hz, 1H), 7.37-7.41 (dd, *J* = 3.0, 1.5 Hz, 1H), 7.65-7.67 (m, 1H), 7.77 (d, *J* = 6.0 Hz, 1H), 8.02-8.06 (m, 2H).
(2) Synthesis of **(S, *R*)-*P*-PtLA3: (*S*, *R*)-LA3** (200 mg, 0.37 mmol, 1.0 equiv.), potassium chloroplatinate (161 mg, 0.39 mmol, 1.05 equiv.), tetra-n-butylammonium bromide (12 mg, 0.037 mmol, 0.1 equiv.) were sequentially added into a 100 mL dry three-necked flask with a magnetic rotor and a condenser tube, followed by nitrogen purge for three times and addition of acetic acid (22 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure. The crude was separated and purified by a silica gel chromatography column with an eluent of petroleum ether/dichloromethane of 1:1 to 1:2 so as to obtain a product **(*S*, *R*)-*P*-PtLA3,** 134 mg as pale primrose solid, with a yield of 49%. ¹H NMR (400 MHz, CDCl₃): δ 1.83-1.91 (m, 2H), 2.85-2.92 (m, 4H), 2.94 (d, *J* = 6.5 Hz, 2H), 3.41-3.46 (d, *J* = 19.6 Hz, 1H), 3.53-3.59 (m, 1H), 5.61 (d, *J* = 7.6 Hz, 1H), 5.65-5.69 (m, 1H), 6.96-7.00 (m, 1H), 7.08-7.17 (m, 2H), 7.15-7.31 (m, 6H), 7.36-7.40 (m, 2H), 7.57-7.62 (m 1H), 7.91-7.96 (m, 2H), 8.11-8.13 (m, 1H).

**Example 6, not in accordance with the present invention and provided for illustrative purposes only:** synthesis of tetradentate cyclometalated platinum (II) complex **(*R*, *S*)-*M*-PtLA3** as follow. 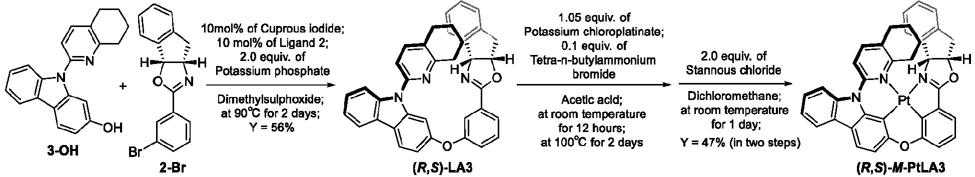
(1) Synthesis of ligand **(R, *S*)-LA3:** 2-Br (477 mg, 1.52 mmol, 1.2 equivalent), 3-OH (400 mg, 1.27 mmol, 1.0 equivalent) and cuprous iodide (25 mg, 0.13 mmol, 10 mol%), ligand 2 (43 mg, 0.13 mmol, 10 mol%) and potassium phosphate (539 mg, 2.54 mmol, 2.0 equiv.) were sequentially added into a dry sealed tube with a magnetic rotor. Nitrogen was purged for three times, and dimethyl sulfoxide (10 mL) was added under nitrogen protection. The sealed tube was placed in an oil bath at 100°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/dichloromethane/ethyl acetate of 5:1:1 so as to obtain a product **(R, S)-M-LA3,** 392 mg as brown solid, with a yield of 56%. ¹H NMR (500 MHz, CDCl₃): δ 1.86-1.93 (m, 4H), 2.84 (t, *J* = 7.5 Hz, 2H), 2.94 (d, *J* = 6.5 Hz, 2H), 3.41 (d, *J* = 18.0 Hz, 1H), 3.49-3.55 (m, 1H), 5.61-5.66 (m, 1H), 5.77-5.82 (m, 1H), 6.94-6.96 (m, 1H), 7.24-7.27 (m, 7H), 7.28-7.31 (m, 2H), 7.37-7.42 (m, 2H), 7.52-7.55 (m, 1H), 7.56-7.58 (m, 1H), 7.73 (d, *J* = 8.5 Hz, 1H), 8.03-8.06 (m, 2H).
(2) Synthesis of **(*R, S*)-PtLA3: (*R, S*)-LA3** (200 mg, 0.37 mmol, 1.0 equiv.), potassium chloroplatinate (161 mg, 0.39 mmol, 1.05 equiv.), tetra-n-butylammonium bromide (12 mg, 0.037 mmol, 0.1 equiv.) were sequentially added into a 100 mL dry three-necked flask with a magnetic rotor and a condenser tube, followed by nitrogen purge for three times and addition of acetic acid (22 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure. The crude was separated and purified by a silica gel chromatography column with an eluent of petroleum ether/dichloromethane of 1:1 to 1:2 so as to obtain a product **(*R*, *S*)-*M*-PtLA3,** 128 mg as pale primrose solid, with a yield of 47%. δ 1.88-1.93 (m, 4H), 2.84-2.94 (m, 4H), 3.55 (d, *J* = 18.0 Hz, 1H), 3.56-3.61 (m, 1H), 5.62 (d, *J* = 7.5 Hz, 1H), 5.85-5.88 (m, 1H), 6.68 (d, *J* = 7.5 Hz, 1H), 6.99-7.01 (m, 1H), 7.01-7.13 (m, 1H), 7.18-7.24 (m, 5H), 7.31-7.35 (m, 1H), 7.37-7.41 (m, 1H), 7.60 (d, *J* = 8.0 Hz, 1H), 7.68 (d, *J* = 8.0 Hz, 1H), 7.93-7.98 (m, 2H), 8.13-8.14 (d, *J* = 8.5 Hz, 1H).

**Example 7, not in accordance with the present invention and provided for illustrative purposes only:** synthesis of tetradentate cyclometalated platinum (II) complex ***P*-PtLC1** as follow.

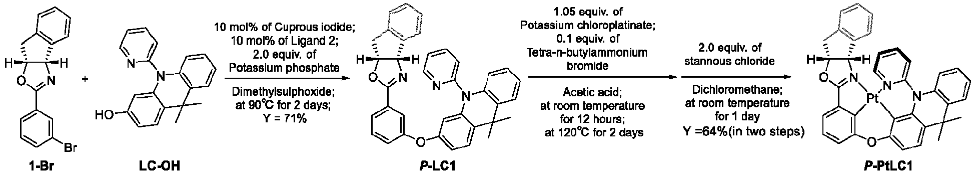

Synthesis of P-LC1: **LC- OH** (500 mg, 1.99 mmol, 1.0 equiv.), **1-Br** (873 mg, 2.39 mmol, 1.2 equiv.), cuprous iodide (76 mg, 0.40 mmol, 20 mol%), **ligand 2** (138 mg, 0.40 mmol, 20 mol%) and potassium phosphate (845 mg, 3.98 mmol, 2.0 equivalents) were successively added into a dry 50 mL three-necked flask with a magnetic rotor. Nitrogen was purged three times, dimethyl sulfoxide (10 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 10: 1 to 5: 1, so as to obtain 758 mg white solid, with a yield of 71%. ¹H NMR (500 MHz, DMSO-*d₆*): δ (ppm) 1.61 (s, 6H), 3.23 (d, *J* = 18.0 Hz, 1H), 3.48 (dd, *J* = 18.0 Hz, 7.0 Hz, 1H), 5.49-5.52 (m, 1H), 5.68 (d, *J* = 8.0 Hz, 1H), 6.05 (d, *J* = 2.5 Hz, 1H), 6.36 (dd, *J* = 8.0, 1.5 Hz, 1H), 6.59 (dd, *J* = 7.5, 2.5 Hz, 1H), 6.98-7.05 (m, 2H), 7.11 (ddd, *J* = 7.5, 2.5, 1.0 Hz, 1H), 7.22-7.31 (m, 4H), 7.35-7.44 (m, 4H), 7.49-7.53 (m, 2H), 7.55-7.57 (m, 1H), 7.89-7.92 (m, 1H), 8.58 (ddd, *J* = 7.5, 2.0, 1.0 Hz, 1H).

Synthesis of *P*-PtLC1: P-LC1 (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 8: 1 to 1: 1, so as to obtain 172 mg pale primrose solid, with a yield of 64%. ¹H NMR (500 MHz, CDCl₃): δ (ppm) 1.34 (s, 3H), 1.91 (s, 3H), 3.55 (s, 2H), 5.79-5.84 (m, 2H), 6.76 (d, *J* = 7.5 Hz, 1H), 6.90-6.93 (m, 1H), 7.01 (d, *J* = 8.5 Hz, 1H), 7.05-7.15 (m, 4H), 7.16-7.26 (m, 6H), 7.37 (d, *J* = 8.5 Hz, 1H), 7.47-7.49 (m, 1H), 7.67-7.71 (m, 1H), 9.11 (dd, *J* = 5.5, 2.0 Hz, 1H).

**Example 8, not in accordance with the present invention and provided for illustrative purposes only:** synthesis of tetradentate cyclometalated platinum (II) complex ***M*-PtLC1** as follow.

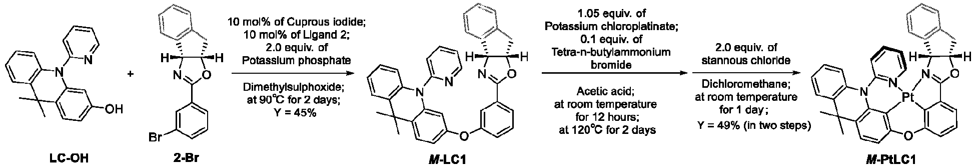

Synthesis of ***M*-LC1: LC- OH** (550 mg, 2.19 mmol, 1.0 equiv.), **2-Br** (961 mg, 2.63 mmol, 1.2 equiv.), cuprous iodide (84 mg, 0.44 mmol, 20 mol%), **ligand 2** (145 mg, 0.44 mmol, 20 mol%) and potassium phosphate (930 mg, 4.38 mmol, 2.0 equivalents) were successively added into a dry 50 mL three-necked flask with a magnetic rotor. Nitrogen was purged three times, dimethyl sulfoxide (10 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 10: 1 to 5: 1, so as to obtain 525 mg white solid, with a yield of 45%. ¹H NMR (500 MHz, CDCl₃): δ (ppm) 1.66 (s, 6H), 3.35 (d, *J* = 18.0 Hz, 1H), 3.48 (dd, *J* = 18.0 Hz, 7.0 Hz, 1H), 5.44-5.47 (m, 1H), 5.72 (d, *J* = 7.5 Hz, 1H), 6.30 (d, *J* = 2.5 Hz, 1H), 6.54-6.58 (m, 2H), 6.99-7.06 (m, 3H), 7.20 (ddd, *J* = 7.5, 5.0, 1.0 Hz, 1H), 7.25-7.30 (m, 5H), 7.36 (d, *J* = 8.5 Hz, 1H), 7.47 (dd, *J =* 7.5, 2.0 Hz, 1H), 7.52-7.56 (m, 2H), 7.62-7.64 (m, 1H), 7.71-7.74 (m, 1H), 8.62 (ddd, *J* = 5.0, 2.0, 1.0 Hz, 1H).

Synthesis of ***M*-PtLC1: *M*-LC1** (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 8: 1 to 1: 1, so as to obtain 132 mg pale primrose solid, with a yield of 49%. ¹H NMR (500 MHz, CDCl₃): δ (ppm) 1.34 (s, 3H), 1.91 (s, 3H), 3.55 (s, 2H), 5.77-5.82 (m, 2H), 6.75 (d, *J* = 7.5 Hz, 1H), 6.87-6.90 (m, 1H), 7.02 (d, *J* = 8.5 Hz, 1H), 7.05-7.15 (m, 4H), 7.18-7.25 (m, 6H), 7.36 (d, *J =* 8.5 Hz, 1H), 7.47-7.49 (m, 1H), 7.66-7.69 (m, 1H), 9.12 (dd, *J* = 5.5, 1.5 Hz, 1H).

**Example 9, not in accordance with the present invention and provided for illustrative purposes only:** synthesis of tetradentate cyclometalated platinum (II) complex **(*S*, *R*)-*P*-PtLAN.** 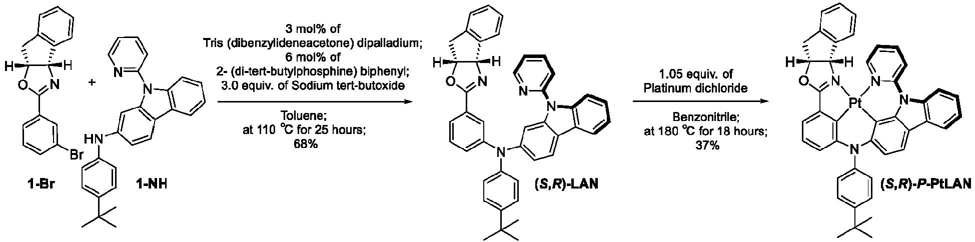
(1) Synthesis of ligand **(*S*, *R*)-LAN: 1-Br** (528 mg, 1.68 mmol, 1.2 equiv.), **1-NH** (548 mg, 1.40 mmol, 1.0 equiv.), tris (dibenzylideneacetone) dipalladium (39 mg, 0.042 mmol, 3 mol%), 2- (di-tert-butylphosphine) biphenyl (13 mg, 0.032 mmol, 8 mol%) and sodium tert-butoxide (404 mg, 4.2 mmol, 3.0 equivalents) were sequentially added into a reaction tube with a magnetic rotor, and then nitrogen was purged for three times and toluene (10 mL) was added under nitrogen protection. Subsequently, this mixture was reacted with stirring in an oil bath at 110°C for 25 hours, cooled to the room temperature, and the solvent was distilled off at a reduced pressure so as to obtain a crude. The crude was separated and purified by a silica gel chromatography column with an eluent with a volume ratio of petroleum ether/ethyl acetate being 6: 1 to 2: 1, so as to obtain a product **(*S*, *R*)-P-LAN,** 599 mg as foamy solid, with a yield of 68%. ¹H NMR (400 MHz, DMSO-*d₆*): δ 1.27 (s, 9H), 3.13-3.18 (m, 1H), 3.44-3.45 (m, 1H), 5.44 (t, *J* = 6.0 Hz, 1H), 5.62 (d, *J* = 7.6 Hz, 1H), 6.95 (d, *J* = 7.6 Hz, 1H), 7.00 (d, *J* = 7.6 Hz, 2H), 7.10 (d, *J* = 7.6 Hz, 1H), 7.19-7.25 (m, 3H), 7.29-7.38 (m, 7H), 7.41-7.43 (m, 2H), 7.51 (s, 1H), 7.62 (d, *J* = 8.0 Hz, 1H), 7.74 (d, *J* = 8.0 Hz, 1H), 7.90 (t, *J* = 8.0 Hz, 1H), 8.13-8.15 (m, 2H), 8.55 (s, 1H).
(2) Synthesis of **(*S*, *R*)-*P*-PtLAN: (*S*, *R*)-*P*-LAN** (187 mg, 0.30 mmol, 1.0 equiv.), platinum dichloride (84 mg, 0.315 mmol, 1.05 equiv.) were added into a three-necked flask with a magnetic rotor, and then nitrogen was purged for three times, benzonitrile (18 mL) was added under nitrogen protection. Subsequently, this mixture was reacted with stirring in an oil bath at 180°C for 18 hours, cooled to the room temperature, and the solvent was distilled off at a reduced pressure so as to obtain a crude. The crude was separated and purified by a silica gel chromatography column with an eluent with a volume ratio of petroleum ether/dichloromethane being 4: 1 to 2: 1, so as to obtain a product **(*S*, *R*)-*P*-PtLAN,** 91 mg as red solid, with a yield of 37%. ¹H NMR (400 MHz, DMSO-*d₆*): δ 1.39 (s, 9H), 3.49 (s, 1H), 3.60-3.67 (m, 1H), 5.99 (t, *J* = 6.8 Hz, 1H), 6.19 (d, *J* = 7.2 Hz, 1H), 6.26 (d, *J* = 8.0 Hz, 1H), 6.32 (d, *J* = 8.8 Hz, 1H), 6.88-6.94 (m, 2H), 7.00 (d, *J* = 7.2 Hz, 1H), 7.08 (t, *J* = 7.6 Hz, 1H), 7.16-7.23 (m, 3H), 7.33-7.46 (m, 4H), 7.57 (d, *J* = 8.8 Hz, 1H), 7.69 (d, *J* = 8.0 Hz, 2H), 7.99 (d, *J* = 7.6 Hz, 1H), 8.15 (d, *J* = 8.4 Hz, 1H), 8.22-8.30 (m, 2H), 9.55 (d, *J* = 5.2 Hz, 1H).

**Example 10, not in accordance with the present invention and provided for illustrative purposes only:** synthesis of tetradentate cyclometalated platinum (II) complex **(*R*, *S*)-*M*-PtLAN as** follow. 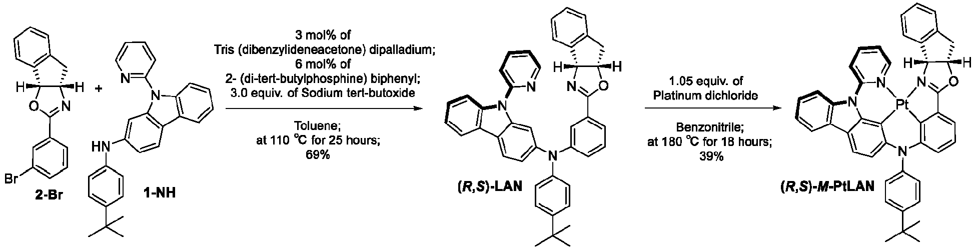
(1) Synthesis of ligand **(*R*, *S*)-LAN: 1-Br** (528 mg, 1.68 mmol, 1.2 equiv.), **1-NH** (548 mg, 1.40 mmol, 1.0 equiv.), tris (dibenzylideneacetone) dipalladium (39 mg, 0.042 mmol, 3 mol%), 2-(di-tert-butylphosphine) biphenyl (13 mg, 0.032 mmol, 8 mol%) and sodium tert-butoxide (404 mg, 4.2 mmol, 3.0 equivalents) were sequentially added into a reaction tube with a magnetic rotor, and then nitrogen was purged for three times and toluene (10 mL) was added under nitrogen protection. Subsequently, this mixture was reacted with stirring in an oil bath at 110 °C for 25 hours, cooled to the room temperature, and the solvent was distilled off at a reduced pressure so as to obtain a crude. The crude was separated and purified by a silica gel chromatography column with an eluent with a volume ratio of petroleum ether/ethyl acetate being 6: 1 to 2: 1, so as to obtain a product **(*R*, *S*)-*M*-LAN,** 608 mg as foamy solid, with a yield of 69%. ¹H NMR (400 MHz, DMSO-*d*₆): δ 1.27 (s, 9H), 3.14-3.18 (m, 1H), 3.44-3.46 (m, 1H), 5.44 (t, *J* = 7.2 Hz, 1H), 5.62 (d, *J* = 7.6 Hz, 1H), 6.95 (d, *J* = 8.4 Hz, 1H), 7.00 (d, *J* = 7.6 Hz, 2H), 7.10 (d, *J* = 8.4 Hz, 1H), 7.18-7.27 (m, 3H), 7.29-7.38 (m, 7H), 7.41-7.44 (m, 2H), 7.51 (s, 1H), 7.62 (d, *J* = 8.0 Hz, 1H), 7.74 (d, *J* = 8.4 Hz, 1H), 7.91 (t, *J* = 8.0 Hz, 1H), 8.13-8.15 (m, 2H), 8.56 (d, *J* = 4.8 Hz, 1H).
(2) Synthesis of **(*R, S*)-*M*-PtLAN: (*R, S*)-*M*-LAN** (250 mg, 0.40 mmol, 1.0 equiv.), platinum dichloride (112 mg, 0.42 mmol, 1.05 equiv.) were sequentially added into a three-necked flask with a magnetic rotor, and then nitrogen was purged for three times, benzonitrile (20 mL) was added under nitrogen protection. Subsequently, this mixture was reacted with stirring in an oil bath at 180°C for 18 hours, cooled to the room temperature, and the solvent was distilled off at a reduced pressure so as to obtain a crude. The crude was separated and purified by a silica gel chromatography column with an eluent with a volume ratio of petroleum ether/dichloromethane being 4: 1 to 2: 1, so as to obtain a product **(*R*, *S*)-*M*-PtLAN,** 127 mg as red solid, with a yield of 39%. ¹H NMR (400 MHz, DMSO-*d*₆): δ 1.39 (s, 9H), 3.49 (s, 1H), 3.60-3.67 (m, 1H), 5.99 (t, *J* = 6.8 Hz, 1H), 6.19 (d, *J* = 7.2 Hz, 1H), 6.26 (d, *J* = 8.0 Hz, 1H), 6.32 (d, *J* = 8.8 Hz, 1H), 6.88-6.94 (m, 2H), 7.00 (d, *J* = 7.2 Hz, 1H), 7.08 (t, *J* = 7.6 Hz, 1H), 7.16-7.23 (m, 3H), 7.33-7.46 (m, 4H), 7.57 (d, *J* = 8.8 Hz, 1H), 7.69 (d, *J* = 8.0 Hz, 2H), 7.99 (d, *J* = 7.6 Hz, 1H), 8.15 (d, *J* = 8.4 Hz, 1H), 8.22-8.30 (m, 2H), 9.55 (d, *J* = 5.2 Hz, 1H).

**Example 11, not in accordance with the present invention and provided for illustrative purposes only:** synthesis of tetradentate cyclometalated platinum (II) complex **(*R*, *S*)-*M*-PtLH1** as follow. 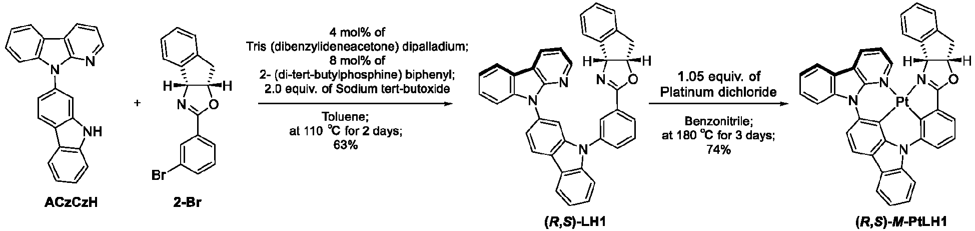
(1) Synthesis of ligand **(*R*, *S*)-LH1: ACzCzH** (200 mg, 0.6 mmol, 1.0 equiv.), **2-Br** (245 mg, 0.78 mmol, 1.3 equiv.), tris (dibenzylideneacetone) dipalladium (22 mg, 0.024 mmol, 4 mol%), 2-(di-tert-butylphosphine) biphenyl (14 mg, 0.048 mmol, 8 mol%) and sodium tert-butoxide (115 mg, 1.2 mmol, 2.0 equivalents) were sequentially added into a dry sealed tube with a magnetic rotor, and then nitrogen was purged for three times and toluene (12 mL) was added under nitrogen protection. This mixture was reacted with stirring in an oil bath at 110 °C for 2 days, cooled to the room temperature, and the solvent was distilled off at a reduced pressure. The crude was separated and purified by a silica gel chromatography column with an eluent of petroleum ether/ethyl acetate of 10: 1 to 5: 1 so as to obtain a product **(*R*, *S*)-LH1,** 214 mg as white solid, with a yield of 63%. ¹H NMR (500 MHz, DMSO-*d*₆): δ 3.21-3.24 (m, 1H), 3.47-3.55 (m, 1H), 5.53-5.56 (m,1H), 5.72 (d, *J* = 8.5 Hz, 1H), 7.20-7.29 (m, 3H), 7.31-7.43 (m, 5H), 7.47-7.54 (m, 3H), 7.57-7.59 (m, 2H), 7.72 (t, *J* = 7.5 Hz, 1H), 7.87-7.89 (m, 1H), 7.95 (d, *J* = 9.0 Hz, 1H), 8.07 (t, *J* = 2.5 Hz, 1H), 8.30 (d, *J* = 8.5 Hz, 1H), 8.37-8.38 (m, 2H), 8.51 (d, *J* = 9.0 Hz, 1H), 8.64 (dd, *J* =8.0, 1.5 Hz, 1H).
(2) Synthesis of **(*R, S*)-*M*-PtLH1:** ligand **(*R, S*)-LH1** (212 mg, 0.37 mmol, 1.0 equiv.) and platinum dichloride (104 mg, 0.39 mmol, 1.05 equiv) were sequentially added into a dry three-necked flask with a magnetic rotor. Nitrogen was purged for three times, benzonitrile (15 mL) was added under nitrogen protection. This mixture was reacted with stirring in an electric heating jacket at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product **(*R*, *S*)-*M*-PtLH1,** 207 mg as yellow solid, with a yield of 74%. ¹H NMR (500 MHz, DMSO-*d*₆): δ 3.51-3.61 (m, 2H), 5.73-5.76 (m, 1H), 5.98 (t, *J* = 6.0 Hz, 1H), 6.22 (d, *J* = 7.0 Hz, 1H), 6.72 (t, *J* = 7.5 Hz, 1H), 7.11 (t, *J* = 7.5 Hz, 1H), 7.27-7.29 (m, 1H), 7.31-7.34 (m, 3H), 7.47-7.51 (m, 1H), 7.57 (t, *J* = 7.5 Hz, 1H), 7.63 (dd, *J* = 7.5, 5.5 Hz, 1H), 7.74-7.77 (m, 1H), 7.85 (d, *J* = 8.5 Hz, 1H), 8.05 (d, *J* = 8.5 Hz, 1H), 8.18-8.20 (m, 1H), 8.25-8.26 (m, 2H), 8.32 (d, *J* = 8.5 Hz, 1H), 8.54 (d, *J* =7.5 Hz, 1H), 9.11 (dd, *J* = 7.5, 1.0 Hz, 1H), 9.47-9.48 (m, 1H).

**Example 12, not in accordance with the present invention and provided for illustrative purposes only:** synthesis of tetradentate cyclometalated platinum (II) complex **(*S*, R)-*P*-PtLH1.** 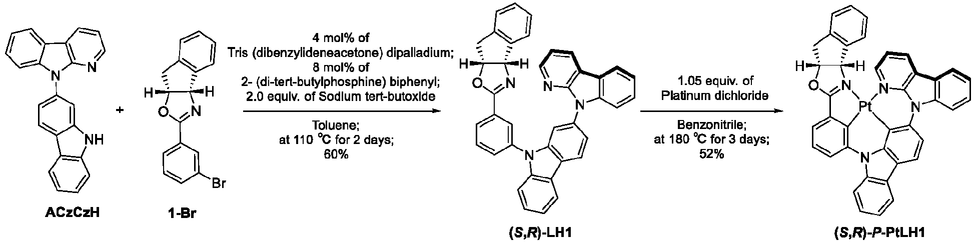
(1) Synthesis of ligand **(*S*, R)-LH1: ACzCzH** (200 mg, 0.6 mmol, 1.0 equiv.), **1-Br** (245 mg, 0.78 mmol, 1.3 equiv.), tris (dibenzylideneacetone) dipalladium (22 mg, 0.024mmol, 4 mol%), 2-(di-tert-butylphosphine) biphenyl (14 mg, 0.048 mmol, 8 mol%) and sodium tert-butoxide (115 mg, 1.2 mmol, 2.0 equivalents) were sequentially added into a dry sealed tube with a magnetic rotor, and then nitrogen was purged for three times and toluene (12 mL) was added under nitrogen protection. This mixture was reacted with stirring in an oil bath at 110°C for 2 days, cooled to the room temperature, and the solvent was distilled off at a reduced pressure. The crude was separated and purified by a silica gel chromatography column with an eluent of petroleum ether/ethyl acetate of 10: 1 to 5: 1 so as to obtain a product **(*S*, *R*)-LH1,** 203 mg as white solid, with a yield of 60%. ¹H NMR (500 MHz, DMSO-*d*₆): δ 3.21-3.24 (m, 1H), 3.46-3.51 (m, 1H), 5.52-5.55 (m,1H), 5.72 (d, *J* = 7.5 Hz, 1H), 7.20-7.29 (m, 3H), 7.31-7.42 (m, 5H), 7.47-7.53 (m, 3H), 7.57-7.58 (m, 2H), 7.72 (t, *J* = 7.5 Hz, 1H), 7.87-7.8 (m, 1H), 7.95 (d, *J* = 8.0 Hz, 1H), 8.07 (t, *J* = 1.5 Hz, 1H), 8.29 (d, *J* = 7.5 Hz, 1H), 8.36-8.38 (m, 2H), 8.51 (d, *J* = 8.5 Hz, 1H), 8.63 (dd, *J* =8.0, 2.0 Hz, 1H).
(2) Synthesis of **(*S, R*)-*P*-PtLH1:** ligand **(*S*, *R*)-LH1** (120 mg, 0.21 mmol, 1.0 equiv.) and platinum dichloride (71 mg, 0.27 mmol, 1.05 equiv) were sequentially added into a dry three-necked flask with a magnetic rotor. Nitrogen was purged for three times, benzonitrile (15 mL) was added under nitrogen protection. This mixture was reacted with stirring in an electric heating jacket at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product **(*S*, *R*)-P-PtLH1,** 83 mg as yellow solid, with a yield of 52%. ¹H NMR (500 MHz, DMSO-*d*₆): δ 3.51-3.61 (m, 2H), 5.74 (d, *J* = 8.0 Hz, 1H), 5.98 (t, *J* = 5.5 Hz, 1H), 6.22 (d, *J* = 6.5 Hz, 1H), 6.72 (t, *J* = 8.0 Hz, 1H), 7.11 (t, *J* = 8.0 Hz, 1H), 7.27-7.29 (m, 1H), 7.31-7.34 (m, 3H), 7.47-7.51 (m, 1H), 7.58 (t, *J* = 7.5 Hz, 1H), 7.64 (dd, *J* = 7.5, 5.5 Hz, 1H), 7.74-7.78 (m, 1H), 7.85 (d, *J* = 8.0 Hz, 1H), 8.05 (d, *J* = 8.5 Hz, 1H), 8.19 (d, *J* = 7.5 Hz, 1H), 8.25-8.26 (m, 2H), 8.32 (d, *J* = 8.5 Hz, 1H), 8.54 (d, *J* =7.5 Hz, 1H), 9.12 (dd, *J* = *7.5,* 1.0 Hz, 1H), 9.47-9.48 (m, 1H).

**Example 13:** synthesis of tetradentate cyclometalated platinum (II) complex ***P*-PtLIII-1** as follow. 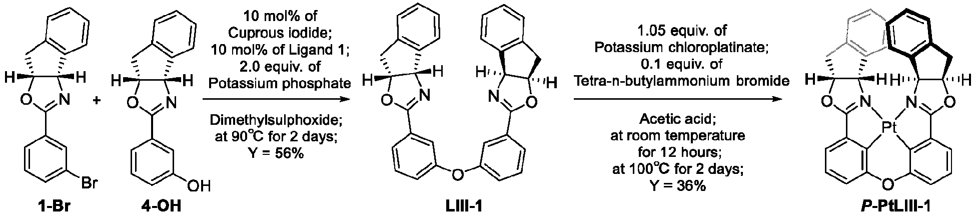
(1) Synthesis of ligand **LIII-1:** 1-Br (750 mg, 2.39 mmol, 1.5 equivalent), 4-OH (400 mg, 1.59 mmol, 1.0 equivalent) and cuprous iodide (37 mg, 0.20 mmol, 10 mol%), ligand 1 (65 mg, 0.20 mmol, 10 mol%) and potassium phosphate (832 mg, 3.92 mmol, 2.0 equiv.) were sequentially added into a dry sealed tube with a magnetic rotor. Nitrogen was purged for three times, and dimethyl sulfoxide (15 mL) was added under nitrogen protection. The sealed tube was placed in an oil bath at 90°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **L III-1,** 431 mg as a white solid, with a yield of 56%. ¹H NMR (500 MHz, DMSO-*d*₆): δ 3.22 (d, *J* = 18.5 Hz, 2H), 3.45-3.50 (m, 2H), 5.49-5.53 (m, 2H), 5.69 (d, *J =* 7.5 Hz, 2H), 7.21-7.30 (m, 8H), 7.38 (s, 2H), 7.42 (d, *J* = 6.5 Hz, 2H), 7.50 (t, *J* = 8.0 Hz, 2H), 7.65 (d, *J* = 7.5 Hz, 2H).
(2) Synthesis of ***P*-PtLIII-1: L6** (200 mg, 0.41 mmol, 1.0 equiv.), potassium chloroplatinate (180 mg, 0.43 mmol, 1.05 equiv.), tetra-n-butylammonium bromide (13 mg, 0.041 mmol, 0.1 equiv.) were sequentially added into a 100 mL dry three-necked flask with a magnetic rotor and a condenser tube, followed by nitrogen purge for three times and addition of acetic acid (25 mL) pre-purged with nitrogen. After a reaction solution was bubbled with nitrogen for 30 minutes, it was stirred at the room temperature for 12 hours, and then was reacted with stirring at 100°C for 2 days, cooled to the room temperature, and solvent was distilled off at a reduced pressure. The crude was separated and purified by a silica gel chromatography column with an eluent of petroleum ether/dichloromethane of 1:1 so as to obtain a product ***P*-PtLIII-1,** 104 mg as pale primrose solid, with a yield of 36%. ¹H NMR (500 MHz, DMSO-*d*₆): δ 3.63 (d, *J =* 18.5 Hz, 2H), 3.67-3.72 (m, 2H), 6.27-6.29 (m, 2H), 6.35 (d, *J* = 7.0 Hz, 2H), 7.27-7.33 (m, 6H), 7.34-7.38 (m, 2H), 7.40-7.43 (m, 4H), 7.87 (d, *J* = 8.0 Hz, 2H).

**Example 14:** synthesis of tetradentate cyclometalated platinum (II) complex ***M*-PtLIII-1** as follow. 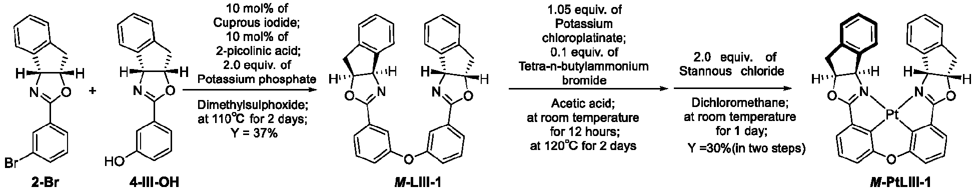
(1) Synthesis of **M-LIII-1: 4-III- OH** (500 mg, 1.99 mmol, 1.0 equiv.), **2-Br** (751 mg, 2.39 mmol, 1.2 equiv.), cuprous iodide (152 mg, 0.80 mmol, 40 mol%), 2-picolinic acid (196 mg, 1.59 mmol, 80 mol%) and potassium phosphate (845 mg, 3.98 mmol, 2.0 equivalents) were successively added into a dry 50 mL three-necked flask with a magnetic rotor. Nitrogen was purged three times, dimethyl sulfoxide (10 mL) was added, and reaction was carried out in a 110 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 30: 1 to 10: 1, so as to obtain 353 mg white solid, with a yield of 37%. ¹H NMR (500 MHz, CDCl₃): δ (ppm) 3.35 (dd, *J* = 17.5, 1.5 Hz, 1H), 3.48 (dd, *J* = 17.5, 6.5 Hz, 1H), 5.44-5.48 (m, 1H), 5.72 (d, *J* = 8.0 Hz, 1H), 7.06 (ddd, *J* = 8.5, 2.5, 1.0 Hz, 1H), 7.25-7.28 (m, 3H), 7.32 (d, *J* = 8.0 Hz, 1H), 7.53-7.56 (m, 2H), 7.68-7.70 (m, 1H).
(2) Synthesis of M-PtLIII-1: ***M*-LIII-1** (200 mg, 0.41 mmol, 1.0 equiv.), potassium chloroplatinate (178 mg, 0.43 mmol,1.05 equiv.) and tetra-n-butylammonium bromide (13 mg, 0.041 mmol, 10 mol%) were sequentially added in a 50 mL three-necked flask with a magnetic rotor, and nitrogen was purged for three times, acetic acid (25 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (155 mg, 0.82 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 4: 1 to 1: 1, so as to obtain 83 mg pale primrose solid, with a yield of 30%. ¹H NMR (500 MHz, CDCl₃): δ (ppm) 3.60 (dd, *J* = 18.0, 5.0 Hz, 1H), 3.65 (d, *J* = 18.0 Hz, 1H), 5.87 (d, *J* = 7.0 Hz, 1H), 5.93-5.96 (m, 1H), 7.07-7.13 (m, 2H), 7.19 (dd, *J* = 7.0, 2.0 Hz, 1H), 7.29-7.34 (m, 3H), 7.78-7.81 (m, 1H).

**Example 15, not in accordance with the present invention and provided for illustrative purposes only:** synthesis of tetradentate cyclometalated platinum (II) complex ***P*-PtLB1** as follow. 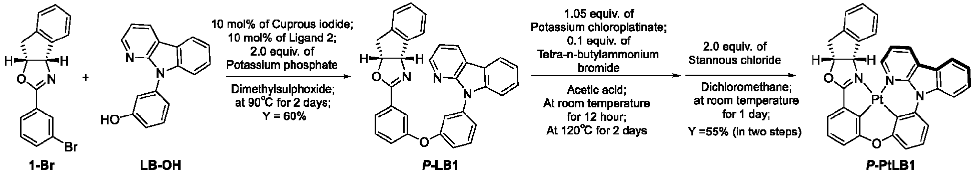
(1) Synthesis of ***P*-LB1: LB-OH** (500 mg, 1.92 mmol, 1.0 equiv.), **2-Br** (723 mg, 2.30 mmol, 1.2 equiv.), cuprous iodide (36 mg, 0.19 mmol, 10 mol%), **ligand 2** (65 mg, 0.19 mmol, 10 mol%) and potassium phosphate (815 mg, 3.84 mmol, 2.0 equivalents) were successively added into a dry 50 mL three-necked flask with a magnetic rotor. Nitrogen was purged three times, dimethyl sulfoxide (10 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 10: 1 to 5: 1, so as to obtain 678 mg white solid, with a yield of 60%. ¹H NMR (500 MHz, CDCl₃): δ (ppm) 3.36 (dd, *J* = 18.0, 1.5 Hz 1H), 3.50 (dd, *J* = 18.0, 7.0 Hz, 1H), 5.46-5.50 (m, 1H), 5.74 (d, *J* = 8.0 Hz, 1H), 7.06 (ddd, *J* = 8.0, 2.5, 1.0 Hz, 1H), 7.21-7.24 (m, 2H), 7.26-7.27 (m, 3H), 7.29-7.32 (m, 2H), 7.35-7.45 (m, 3H), 7.51-7.57 (m, 3H), 7.70-7.72 (m, 2H), 8.10 (d, *J* = 7.5 Hz, 1H), 8.37 (dd, *J* = 7.5, 1.5 Hz, 1H), 8.47 (d, *J* = 4.5, 1.5 Hz, 1H).
(2) Synthesis of ***P*-PtLB1: *P*-LB1** (200 mg, 0.41 mmol, 1.0 equiv.), potassium chloroplatinate (178 mg, 0.43 mmol,1.05 equiv.) and tetra-n-butylammonium bromide (13 mg, 0.041 mmol, 10 mol%) were sequentially added in a 50 mL three-necked flask with a magnetic rotor, and nitrogen was purged for three times, acetic acid (25 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (155 mg, 0.82 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 4: 1 to 1: 1, so as to obtain 154 mg pale primrose solid, with a yield of 55%. P-PtLB1 ¹H NMR (500 MHz, DMSO-*d*₆): δ (ppm) 3.48 (d, *J* = 18.8 Hz, 1H), 3.57 (dd, *J* = 18.4 Hz, 5.6 Hz, 1H), 5.60 (d, *J* = 8.0 Hz, 1H), 5.96 (t, *J* = 6.4 Hz, 1H), 6.14 (d, *J* = 7.2 Hz, 1H), 6.68 (t, *J* = 7.2 Hz, 1H), 7.02 (dd, *J* = 7.6, 0.8 Hz, 1H), 7.07-7.19 (m, 4H), 7.23 (t, *J* = 8.0 Hz, 1H), 7.30 (d, *J* = 7.6 Hz, 1H), 7.52-7.57 (m, 2H), 7.63 (dd, *J* = 7.6, 5.6 Hz, 1H), 7.69-7.74 (m, 1H), 8.19 (d, *J* = 8.4 Hz, 1H), 8.51 (d, *J* = 7.6 Hz, 1H), 9.08 (dd, *J* = 6.8, 1.2 Hz, 1H), 9.37 (d, *J* = 5.6 Hz, 1H).

**Example 16, not in accordance with the present invention and provided for illustrative purposes only:** synthesis of tetradentate cyclometalated platinum (II) complex M-PtLB1 as follow. 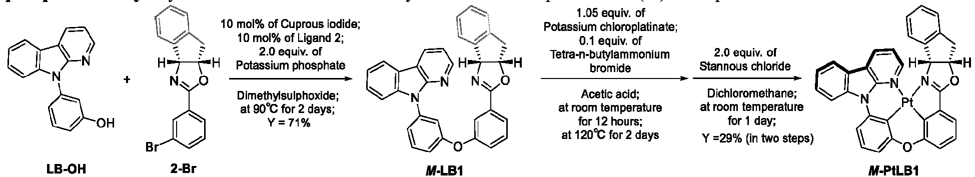
(1) Synthesis of *M*-LB1: **LB-OH** (500 mg, 1.92 mmol, 1.0 equiv.), **2-Br** (723 mg, 2.30 mmol, 1.2 equiv.), cuprous iodide (36 mg, 0.19 mmol, 10 mol%), **ligand 2** (65 mg, 0.19 mmol, 10 mol%) and potassium phosphate (815 mg, 3.84 mmol, 2.0 equivalents) were successively added into a dry 50 mL three-necked flask with a magnetic rotor. Nitrogen was purged three times, dimethyl sulfoxide (10 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 10: 1 to 5: 1, so as to obtain 678 mg white solid, with a yield of 60%. ¹H NMR (500 MHz, CDCl₃): δ (ppm) 3.36 (d, *J* = 18.0 Hz 1H), 3.49 (dd, *J* = 18.0, 7.0 Hz, 1H), 5.46-5.50 (m, 1H), 5.74 (d, *J* = 8.0 Hz, 1H), 7.06 (dd, *J* = 8.5, 2.0 Hz, 1H), 7.21-7.24 (m, 2H), 7.26-7.28 (m, 3H), 7.29-7.33 (m, 2H), 7.35-7.45 (m, 3H), 7.51-7.57 (m, 3H), 7.70-7.72 (m, 2H), 8.11 (d, *J* = 8.0 Hz, 1H), 8.37 (dd, *J* = 7.5, 1.5 Hz, 1H), 8.47 (d, *J* = 5.0, 1.5 Hz, 1H).
(2) Synthesis of ***M*-PtLB1: *P*-LB1** (200 mg, 0.41 mmol, 1.0 equiv.), potassium chloroplatinate (178 mg, 0.43 mmol,1.05 equiv.) and tetra-n-butylammonium bromide (13 mg, 0.041 mmol, 10 mol%) were sequentially added in a 50 mL three-necked flask with a magnetic rotor, and nitrogen was purged for three times, acetic acid (25 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (155 mg, 0.82 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 4: 1 to 1: 1, so as to obtain 83 mg pale primrose solid, with a yield of 29%. ¹H NMR (500 MHz, DMSO-*d*₆): δ (ppm) 3.47 (d, *J* = 18.0 Hz, 1H), 3.57 (dd, *J* = 18.0 Hz, 5.5 Hz, 1H), 5.60 (d, *J* = 8.0 Hz, 1H), 5.96 (t, *J* = 6.0 Hz, 1H), 6.14 (d, *J* = 7.0 Hz, 1H), 6.68 (t, *J* = 7.0 Hz, 1H), 7.02 (dd, *J* = 8.0, 1.5 Hz, 1H), 7.07-7.18 (m, 4H), 7.22 (t, *J* = 8.0 Hz, 1H), 7.29 (d, *J* = 7.5 Hz, 1H), 7.51-7.56 (m, 2H), 7.62 (dd, *J* = 7.5, 6.0 Hz, 1H), 7.69-7.72 (m, 1H), 8.18 (d, *J* = 8.0 Hz, 1H), 8.51 (d, *J* = 7.5 Hz, 1H), 9.08 (d, *J* = 7.5, 1.5 Hz, 1H), 9.36 (d, *J* = 5.5 Hz, 1H).

**Example 17, not in accordance with the present invention and provided for illustrative purposes only:** synthesis of tetradentate cyclometalated platinum (II) complex **P-PtLD1** as follow. 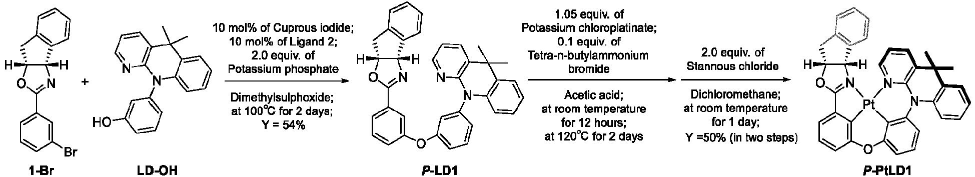
(1) Synthesis of *P*-LD1: **LD-OH** (500 mg, 1.65 mmol, 1.0 equiv.), **1-Br** (572 mg, 1.82 mmol, 1.1 equiv.), cuprous iodide (32 mg, 0.17 mmol, 10 mol%), **ligand 2** (59 mg, 0.17 mmol, 10 mol%) and potassium phosphate (700 g, 3.30 mmol, 2.0 equivalents) were successively added into a dry 50 mL three-necked flask with a magnetic rotor. Nitrogen was purged three times, dimethyl sulfoxide (10 mL) was added, and reaction was carried out in a 100 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 20: 1 to 10: 1, so as to obtain 480 mg white solid, with a yield of 54%. ¹H NMR (500 MHz, CDCl₃): δ (ppm) 1.64 (s, 6H), 3.35 (dd, *J* = 17.5, 1.5 Hz, 1H), 3.50 (dd, *J* = 18.0, 7.0 Hz, 1H), 5.46-5.49 (m, 1H), 5.73 (d, *J* = 8.0 Hz, 1H), 6.37 (dd, *J* = 8.0, 1.5 Hz, 1H), 6.83 (dd, *J* = 7.5, 5.0 Hz, 1H), 6.95-7.03 (m, 3H), 7.07-7.12 (m, 2H), 7.20 (ddd, *J* = 8.0, 2.5, 1.0 Hz, 1H), 7.26-7.28 (m, 3H), 7.34 (t, *J* = 8.5 Hz, 1H), 7.42 (dd, *J* = 7.5, 1.5 Hz, 1H), 7.52-7.57 (m, 2H), 7.65 (dd, *J* = 7.5, 1.5 Hz, 1H), 7.67-7.69 (m, 2H), 8.00 (dd, *J* = 5.0, 2.0 Hz, 1H).
(2) Synthesis of ***P*-PtLD1: *P*-LD1** (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 3: 1 to 1: 1, so as to obtain 135 mg pale primrose solid, with a yield of 50%. ¹H NMR (500 MHz, DMSO-*d*₆): δ (ppm) 1.41 (s, 3H), 1.99 (s, 3H), 3.42 (d, *J* = 18.0 Hz, 1H), 3.56 (dd, *J* = 18.5 Hz, 6.0 Hz, 1H), 5.92-5.98 (m, 3H), 6.50 (t, *J* = 7.5 Hz, 1H), 6.68 (dd, *J* = 7.5, 1.0 Hz, 1H), 6.80-6.82 (m, 1H), 6.88 (dd, *J* = 8.0, 1.0 Hz, 1H), 6.98 (t, *J* = 7.5 Hz, 1H), 7.06-7.20 (m, 6H), 7.27 (d, *J* = 7.5 Hz, 1H), 7.34 (dd, *J* = 7.5, 5.5 Hz, 1H), 7.57-7.59 (m, 1H), 8.26 (dd, *J* = 7.5, 1.5 Hz, 1H), 9.06 (dd, *J* = 5.5, 1.5 Hz, 1H).

**Example 18, not in accordance with the present invention and provided for illustrative purposes only:** synthesis of tetradentate cyclometalated platinum (II) complex ***M*-PtLD1** as follow. 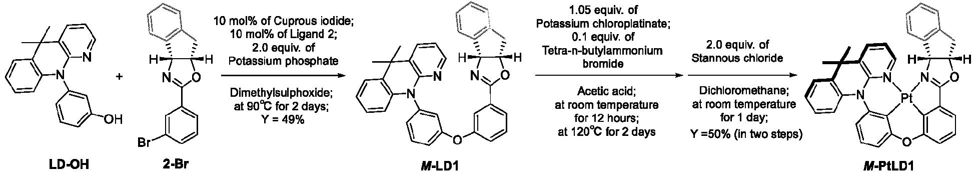
(1) Synthesis of *M*-LD1: **LD- OH** (500 mg, 1.65 mmol, 1.0 equiv.), **2-Br** (572 mg, 1.82 mmol, 1.1 equiv.), cuprous iodide (32 mg, 0.17 mmol, 10 mol%), **ligand 2** (59 mg, 0.17 mmol, 10 mol%) and potassium phosphate (700 g, 3.30 mmol, 2.0 equivalents) were successively added into a dry 50 mL three-necked flask with a magnetic rotor. Nitrogen was purged three times, dimethyl sulfoxide (10 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 20: 1 to 10: 1, so as to obtain 433 mg white solid, with a yield of 49%. ¹H NMR (500 MHz, CDCl₃): δ (ppm) 1.64 (s, 6H), 3.35 (d, *J* = 17.5 Hz, 1H), 3.50 (dd, *J* = 17.5, 6.5 Hz, 1H), 5.46-5.49 (m, 1H), 5.73 (d, *J* = 8.0 Hz, 1H), 6.37 (dd, *J* = 8.0, 1.5 Hz, 1H), 6.83 (dd, *J* = 7.5, 4.5 Hz, 1H), 6.95-7.03 (m, 3H), 7.07-7.12 (m, 2H), 7.20 (ddd, *J* = 7.5, 2.5, 1.5 Hz, 1H), 7.26-7.28 (m, 3H), 7.34 (t, *J= 8.0* Hz, 1H), 7.42 (dd, *J* = 7.5, 2.0 Hz, 1H), 7.52-7.57 (m, 2H), 7.65 (dd, *J* = 8.0, 1.5 Hz, 1H), 7.67-7.69 (m, 2H), 8.00 (dd, *J* = 4.5, 1.5 Hz, 1H).
(2) Synthesis of ***M*-PtLD1**: ***P*-LD1** (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 4: 1 to 1: 1, so as to obtain 134 mg pale primrose solid, with a yield of 50%. ¹H NMR (500 MHz, DMSO-*d*₆): δ (ppm) 1.41 (s, 3H), 1.99 (s, 3H), 3.43 (d, *J* = 18.5 Hz, 1H), 3.56 (dd, *J* = 18.5 Hz, 5.5 Hz, 1H), 5.92-5.98 (m, 3H), 6.50 (t, *J* = 7.5 Hz, 1H), 6.68 (dd, *J* = 8.0, 1.5 Hz, 1H), 6.80-6.82 (m, 1H), 6.88 (dd, *J* = 8.0, 1.5 Hz, 1H), 6.98 (t, *J* = 7.5 Hz, 1H), 7.06-7.20 (m, 6H), 7.27 (d, *J* = 7.5 Hz, 1H), 7.34 (d, *J* = 8.0, 6.0 Hz, 1H), 7.57-7.59 (m, 1H), 8.26 (dd, *J* = 7.5, 1.5 Hz, 1H), 9.06 (dd, *J* = 5.5, 1.5 Hz, 1H).

**Example 19, not in accordance with the present invention and provided for illustrative purposes only:** synthesis of tetradentate cyclometalated platinum (II) complex ***P*-PtLE1** as follow. 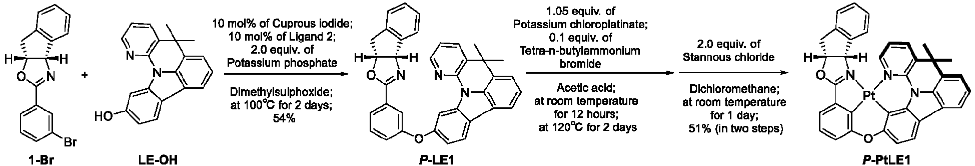
(1) Synthesis of *P*-LE1: **LE- OH** (500 mg, 1.66 mmol, 1.0 equiv.), **1-Br** (575 mg, 1.83 mmol, 1.1 equiv.), cuprous iodide (32 mg, 0.17 mmol, 10 mol%), **ligand 2** (59 mg, 0.17 mmol, 10 mol%) and potassium phosphate (705 g, 3.32 mmol, 2.0 equivalents) were successively added into a dry 50 mL three-necked flask with a magnetic rotor. Nitrogen was purged three times, dimethyl sulfoxide (10 mL) was added, and reaction was carried out in a 100 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 10: 1 to 5: 1, so as to obtain 0.55 g white solid, with a yield of 62%. ¹H NMR (500 MHz, CDCl₃): δ (ppm) 1.73 (s, 6H), 3.34 (dd, *J* = 17.5, 1.5 Hz, 1H), 3.46 (dd, *J* = 18.0 Hz, 7.0 Hz, 1H), 5.43-5.47 (m, 1H), 5.72 (d, *J* = 7.5 Hz, 1H), 7.00 (dd, *J* = 8.5, 2.0 Hz, 1H), 7.04 (dd, *J* = 7.5, 4.5 Hz, 1H), 7.17 (ddd, *J* = 8.0, 2.5, 1.0 Hz, 1H), 7.24-7.25 (m, 3H), 7.32-7.36 (m, 2H), 7.39 (dd, *J* = 7.5, 1.0 Hz, 1H), 7.53-7.55 (m, 1H), 7.63 (dd, *J* = 2.5, 1.5 Hz, 1H), 7.67-7.69 (m, 1H), 7.80 (dd, *J* = 7.5, 1.5 Hz, 1H), 7.84 (dd, *J* = 7.5, 1.5 Hz, 1H), 8.00 (d, *J* = 8.5 Hz, 1H), 8.26 (dd, *J* = 5.0, 2.0 Hz, 1H), 8.82 (d, *J* = 2.0 Hz, 1H).
(2) Synthesis of ***P*-PtLE1: *P*-LE1** (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 3: 1 to 1: 1, so as to obtain 138 mg pale primrose solid, with a yield of 51%. ¹H NMR (400 MHz, DMSO-*d*₆): δ (ppm) 1.72 (s, 3H), 1.94 (s, 3H), 3.48 (d, *J* = 18.8 Hz, 1H), 3.48 (dd, *J* = 18.8 Hz, 6.4 Hz, 1H), 5.98 (t, *J* = 6.4 Hz, 1H), 6.21 (d, *J* = 7.2 Hz, 1H), 6.42 (d, *J* = 8.0 Hz, 1H), 6.95 (t, *J* = 7.6 Hz, 1H), 7.13-7.24 (m, 5H), 7.32-7.37 (m, 2H), 7.44 (t, *J* = 7.6 Hz, 1H), 7.57 (d, *J* = 7.6 Hz, 1H), 7.89 (d, *J* = 8.0 Hz, 1H), 7.95 (d, *J* = 8.0 Hz, 1H), 8.50 (dd, *J* = 7.6, 1.6 Hz, 1H), 9.30 (d, *J* = 5.6 Hz, 1H).

**Example 20, not in accordance with the present invention and provided for illustrative purposes only:** synthesis of tetradentate cyclometalated platinum (II) complex ***M*-PtLE1** as follow. 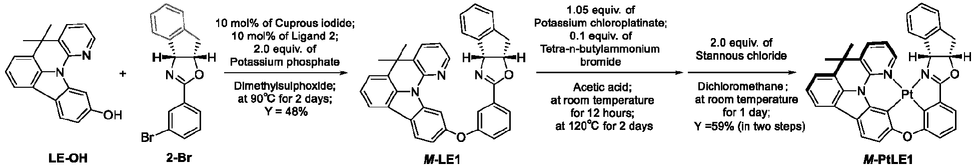
(1) Synthesis of *M*-LE1: **LE- OH** (500 mg, 1.66 mmol, 1.0 equiv.), **2-Br** (575 mg, 1.83 mmol, 1.1 equiv.), cuprous iodide (32 mg, 0.17 mmol, 10 mol%), **ligand 2** (59 mg, 0.17 mmol, 10 mol%) and potassium phosphate (705 g, 3.32 mmol, 2.0 equivalents) were successively added into a dry 50 mL three-necked flask with a magnetic rotor. Nitrogen was purged three times, dimethyl sulfoxide (8 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 10: 1 to 5: 1, so as to obtain 444 mg white solid, with a yield of 48%. ¹H NMR (500 MHz, CDCl₃): δ (ppm) 1.73 (s, 6H), 3.34 (dd, *J* = 18.0, 2.0 Hz, 1H), 3.46 (dd, *J* = 17.5, 6.5 Hz, 1H), 5.43-5.47 (m, 1H), 5.71 (d, *J* = 8.0 Hz, 1H), 7.00 (dd, *J* = 8.5, 2.0 Hz, 1H), 7.04 (dd, *J* = 7.5, 4.5 Hz, 1H), 7.17 (ddd, *J* = 8.0, 2.5, 1.0 Hz, 1H), 7.25-7.26 (m, 3H), 7.32-7.40 (m, 3H), 7.53-7.55 (m, 1H), 7.63 (dd, *J* = 2.5, 1.5 Hz, 1H), 7.68 (d, *J* = 8.0 Hz, 1H), 7.80 (dd, *J* = 7.5, 1.5 Hz, 1H), 7.83 (dd, *J* = 7.5, 1.0 Hz, 1H), 8.00 (d, *J* = 7.5 Hz, 1H), 8.26 (dd, *J* = 5.0, 2.0 Hz, 1H), 8.82 (d, *J* = 2.0 Hz, 1H).
(2) Synthesis of ***M*-PtLE1: *P*-LE1** (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 3: 1 to 1: 1, so as to obtain 158 mg pale primrose solid, with a yield of 59%. ¹H NMR (400 MHz, DMSO-*d*₆): δ (ppm) 1.72 (s, 3H), 1.94 (s, 3H), 3.48 (d, *J* = 18.8 Hz, 1H), 3.48 (dd, *J* = 18.0 Hz, 6.0 Hz, 1H), 5.98 (t, *J* = 6.4 Hz, 1H), 6.21 (d, *J* = 7.2 Hz, 1H), 6.42 (d, *J* = 7.6 Hz, 1H), 6.95 (t, *J* = 7.2 Hz, 1H), 7.13-7.24 (m, 5H), 7.32-7.37 (m, 2H), 7.44 (t, *J =* 7.6 Hz, 1H), 7.57 (d, *J =* 7.6 Hz, 1H), 7.89 (d, *J =* 8.0 Hz, 1H), 7.95 (dd, *J =* 7.6, 0.8 Hz, 1H), 8.50 (dd, *J* = 7.6, 1.2 Hz, 1H), 9.30 (d, *J* = 6.0 Hz, 1H).

**Example 21, not in accordance with the present invention and provided for illustrative purposes only:** synthesis of tetradentate cyclometalated platinum (II) complex ***P*-PtLF1** as follow. 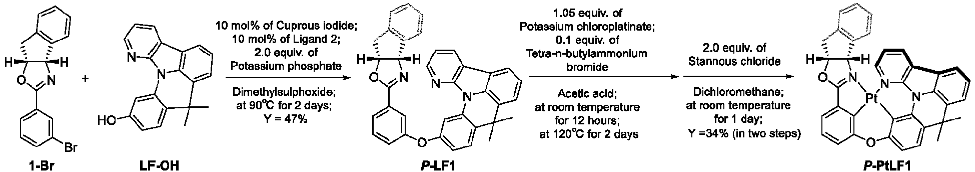
(1) Synthesis of *P*-LF1: **LF- OH** (500 mg, 1.66 mmol, 1.0 equiv.), **1-Br** (575 mg, 1.83 mmol, 1.1 equiv.), cuprous iodide (32 mg, 0.17 mmol, 10 mol%), **ligand 2** (59 mg, 0.17 mmol, 10 mol%) and potassium phosphate (705 g, 3.32 mmol, 2.0 equivalents) were successively added into a dry 50 mL three-necked flask with a magnetic rotor. Nitrogen was purged three times, dimethyl sulfoxide (10 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 10: 1 to 5: 1, so as to obtain 455 mg white solid, with a yield of 47%. ¹H NMR (500 MHz, CDCl₃): δ (ppm) 1.76 (s, 6H), 3.35 (dd, *J* = 18.0, 2.0 Hz, 1H), 3.48 (dd, *J* = 18.0, 7.0 Hz, 1H), 5.46-5.49 (m, 1H), 5.73 (d, *J* = 8.0 Hz, 1H), 6.75 (dd, *J* = 8.5, 2.5 Hz, 1H), 7.19-7.22 (m, 2H), 7.25-7.26 (m, 3H), 7.35-7.40 (m, 2H), 7.49-7.56 (m, 3H), 7.69-7.72 (m, 2H), 7.87 (dd, *J* = 7.5, 1.0 Hz, 1H), 8.32 (dd, *J* = 8.0, 2.0 Hz, 1H), 8.47 (dd, *J* = 5.0, 1.5 Hz, 1H), 9.40 (d, *J* = 2.5 Hz, 1H).
(2) Synthesis of ***P*-PtLF1: *P*-LF1** (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 8: 1 to 1: 1, so as to obtain 96 mg pale primrose solid, with a yield of 34%. ¹H NMR (500 MHz, DMSO-*d*₆): δ (ppm) 1.66 (s, 3H), 1.85 (s, 3H), 3.49 (d, *J* = 18.5 Hz, 1H), 3.61 (dd, *J* = 18.5 Hz, 6.0 Hz, 1H), 5.98 (t, *J* = 6.5 Hz, 1H), 6.30 (d, *J* = 7.5 Hz, 1H), 6.32 (d, *J* = 7.0 Hz, 1H), 6.89 (t, *J* = 7.5 Hz, 1H), 7.03 (d, *J* = 8.5 Hz, 1H), 7.07 (dd, *J* = 8.0, 1.5 Hz, 1H), 7.11-7.18 (m, 3H), 7.32 (d, *J* = 7.5 Hz, 1H), 7.50 (d, *J* = 8.5 Hz, 1H), 7.55-7.59 (m, 2H),7.80 (dd, *J* = 8.0, 1.0 Hz, 1H), 8.23 (dd, *J* = 8.0, 1.0 Hz, 1H), 9.04 (dd, *J* = 7.5, 1.5 Hz, 1H), 9.41 (dd, *J* = 5.5, 1.0 Hz, 1H).

**Example 22, not in accordance with the present invention and provided for illustrative purposes only:** synthesis of tetradentate cyclometalated platinum (II) complex ***M*-PtLF1** as follow. 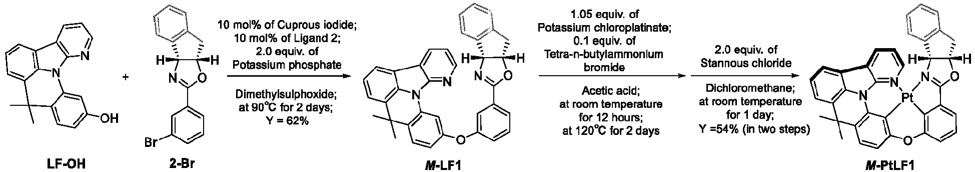
(1) Synthesis of *M*-LF1: **LF- OH** (500 mg, 1.66 mmol, 1.0 equiv.), **2-Br** (625 mg, 1.99 mmol, 1.2 equiv.), cuprous iodide (32 mg, 0.17 mmol, 10 mol%), **ligand 2** (59 mg, 0.17 mmol, 10 mol%) and potassium phosphate (705 g, 3.32 mmol, 2.0 equivalents) were successively added into a dry 50 mL three-necked flask with a magnetic rotor. Nitrogen was purged three times, dimethyl sulfoxide (10 mL) was added, and reaction was carried out in a 90 °C oil bath for 2 days. After being cooled to the room temperature, the reaction solution was washed with water and extracted with ethyl acetate for three times. Then organic phases were combined, washed once with water, dried with anhydrous sodium sulfate and filtered, mixed with silica gel, loaded in a dry manner, and separated and purified by column chromatography, with an eluant of petroleum ether: ethyl acetate of 15: 1 to 8: 1, so as to obtain 546 mg white solid, with a yield of 62%. ¹H NMR (500 MHz, CDCl₃): δ (ppm) 1.76 (s, 6H), 3.35 (dd, *J* = 18.0, 2.0 Hz, 1H), 3.48 (dd, *J* = 18.0, 7.0 Hz, 1H), 5.46-5.49 (m, 1H), 5.73 (d, *J* = 8.0 Hz, 1H), 6.75 (dd, *J* = 8.5, 2.5 Hz, 1H), 7.19-7.22 (m, 2H), 7.25-7.27 (m, 3H), 7.35-7.40 (m, 2H), 7.49-7.56 (m, 3H), 7.69-7.73 (m, 2H), 7.87 (dd, *J* = 7.5, 1.0 Hz, 1H), 8.32 (dd, *J* = 7.5, 1.5 Hz, 1H), 8.47 (dd, *J* = 5.0, 1.5 Hz, 1H), 9.40 (d, *J* = 2.5 Hz, 1H).
(2) Synthesis of ***M*-PtLF1: *P*-LF1** (200 mg, 0.37 mmol, 1.0 equiv.), potassium chloroplatinate (162 mg, 0.39 mmol,1.05 equiv.) and tetra-n-butylammonium bromide (12 mg, 0.037 mmol, 10 mol%) were sequentially added in a 50 mL three-necked flask with a magnetic rotor, and nitrogen was purged for three times, acetic acid (22 mL) was added, which was then bubbled with nitrogen for 30 minutes, stirred at the room temperature for 12 hours, warmed again to 120°C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 8: 1 to 1: 1, so as to obtain 145 mg pale primrose solid, with a yield of 54%. ¹H NMR (400 MHz, DMSO-*d*₆): δ (ppm) 1.66 (s, 3H), 1.85 (s, 3H), 3.49 (d, *J* = 18.4 Hz, 1H), 3.61 (dd, *J* = 18.5 Hz, 6.0 Hz, 1H), 5.99 (t, *J* = 6.4 Hz, 1H), 6.29 (d, *J* = 8.0 Hz, 1H), 6.32 (d, *J* = 7.2 Hz, 1H), 6.89 (t, *J* = 7.2 Hz, 1H), 7.02-7.19 (m, 5H), 7.32 (d, *J* = 7.6 Hz, 1H), 7.50 (d, *J* = 8.4 Hz, 1H), 7.55-7.60 (m, 2H),7.81 (d, *J* = 7.6 Hz, 1H), 8.23 (dd, *J* = 7.6, 0.8 Hz, 1H), 9.05 (dd, *J* = 7.6, 1.6 Hz, 1H), 9.41 (d, *J* = 5.6 Hz, 1H).

**Example 23, not in accordance with the present invention and provided for illustrative purposes only:** synthesis of tetradentate cyclometalated platinum (II) complex ***P*-PtB1** as follow. 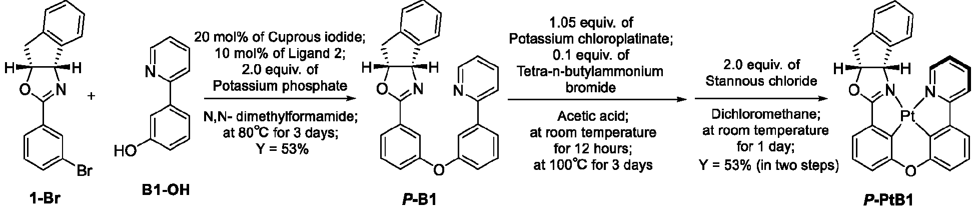
(1) Synthesis of ligand ***P*-B1:** 1-Br (689 mg, 2.20 mmol, 1.1 equivalent), B1-OH (340 mg, 2.00 mmol, 1.0 equivalent) and cuprous iodide (76 mg, 0.40 mmol, 20 mol%), ligand 2 (69 mg, 0.20 mmol, 10 mol%) and potassium phosphate (849 mg, 4.00 mmol, 2.0 equiv.) were sequentially added into a dry sealed tube with a magnetic rotor. Nitrogen was purged for three times, and N, N-dimethylformamide (5 mL) was added under nitrogen protection. The sealed tube was placed in an oil bath at 80°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***P*-B1,** 430 mg as a white solid, with a yield of 53%. ¹H NMR (500 MHz, CDCl₃): *δ* (ppm) 3.35 (dd, *J* = 18.0, 1.5 Hz, 1H), 3.48 (dd, *J* = 18.0, 7.0 Hz, 1H), 5.46 (ddd, *J* = 8.5, 7.0, 1.5 Hz, 1H), 5.72 (d, *J* = 8.0 Hz, 1H), 7.03 (ddd, *J* = 8.0, 2.5, 1.0 Hz, 1H), 7.13 (ddd, *J* = 8.5, 2.5, 1.0 Hz, 1H), 7.22 (ddd, *J* = 7.5, 4.5, 1.0 Hz, 1H), 7.25-7.27 (m, 3H), 7.34 (t, *J* = 8.0 Hz, 1H), 7.43 (t, *J =* 8.0 Hz, 1H), 7.53-7.56 (m, 1H), 7.61 (dd, *J* = 2.0*,* 1.5 Hz, 1H), 7.62 (t, *J* = 2.0 Hz, 1H), 7.67 (dt, *J* = 8.0, 1.0 Hz, 1H), 7.69 (dt, *J* = 7.5, 1.0 Hz, 1H), 7.72 (dd, *J* = 7.5, 2.0 Hz, 1H), 7.75 (ddd, *J* = 7.5, 1.5, 1.0 Hz, 1H), 8.66 (ddd, *J* = 5.0, 2.0, 1.0 Hz, 1H). ¹³C NMR (125 MHz, CDCl₃): *δ* (ppm) 39.73, 76.98, 83.24, 117.53, 118.82, 119.41, 120.59, 121.86, 122.05, 122.34, 123.36, 125.24, 125.60, 127.43, 128.44, 129.64, 129.70, 130.10, 136.71, 139.68, 141.43, 141.85, 149.64, 156.61, 157.16, 157.50, 163.41. HRMS (ESI): calcd for C₂₇H₂₁N₂O₂ [M+H]⁺ 405.1598, found 405.1581.
(2) Synthesis of ***P*-PtB1:** *P*-B1 (243 mg, 0.60 mmol, 1.0 equiv.), potassium chloroplatinate (262 mg, 0.63 mmol, 1.05 equiv.), tetra-n-butylammonium bromide (19 mg, 0.060 mmol, 0.1 equiv.) were sequentially added into a 100 mL dry three-necked flask with a magnetic rotor and a condenser tube, followed by nitrogen purge for three times and addition of acetic acid (36 mL) pre-purged with nitrogen. After a reaction solution was bubbled with nitrogen for 30 minutes, it was stirred at the room temperature for 12 hours, and then was reacted with stirring at 100 °C for 3 days, cooled to the room temperature, and solvent was distilled off at a reduced pressure, and stannous chloride (228 mg, 1.2 mmol, 2.0 equiv.) and dichloromethane (60 mL) were added and stirred at the room temperature for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 1:1 so as to obtain a product P-PtB1, 190 mg as red solid, with a yield of 53%. ¹H NMR (500 MHz, CDCl₃): *δ* (ppm) 3.57 (dd, *J =* 18.0, 5.0 Hz, 1H), 3.63 (d, *J* = 18.0 Hz, 1H), 5.76 (d, *J* = 7.0 Hz, 1H), 5.83 (ddd, *J* = 6.5, 5.0, 0.5 Hz, 1H), 7.11-7.16 (m, 2H), 7.19 (dd, *J* = 10.5, 1.0 Hz, 1H), 7.21-7.22 (m, 1H), 7.23-7.28 (m, 3H), 7.29-7.33 (m, 2H), 7.47-7.48 (m, 1H), 7.56 (d, *J =* 8.0 Hz, 1H), 7.90-7.96 (m, 2H), 9.00 (d, *J* = 5.5 Hz, 1H). HRMS (ESI): calcd for C₂₇H₁₉N₂O₂Pt [M+H]⁺ 598.1089, found 598.1090.

**Example 24, not in accordance with the present invention and provided for illustrative purposes only:** synthesis of tetradentate cyclometalated platinum (II) complex **M-PtB1** as follow. 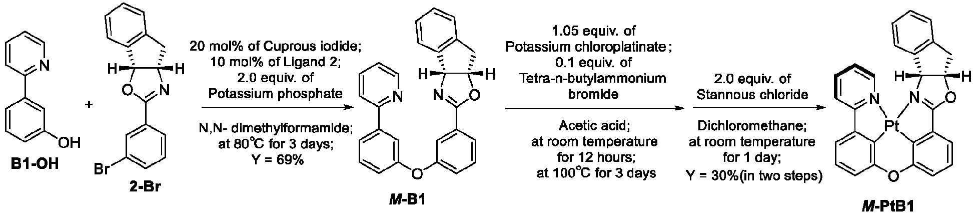
(1) Synthesis of ligand ***M*-LB1:** 2-Br (689 mg, 2.20 mmol, 1.1 equivalent), B1-OH (340 mg, 2.00 mmol, 1.0 equivalent) and cuprous iodide (76 mg, 0.40 mmol, 20 mol%), ligand 2 (69 mg, 0.20 mmol, 10 mol%) and potassium phosphate (849 mg, 4.00 mmol, 2.0 equiv.) were sequentially added into a dry sealed tube with a magnetic rotor. Nitrogen was purged for three times, and N, N-dimethylformamide (5 mL) was added under nitrogen protection. The sealed tube was placed in an oil bath at 80°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***M*-B1**, 560 mg as a white solid, with a yield of 69%. ¹H NMR (500 MHz, CDCl₃): *δ* (ppm) 3.35 (dd, *J* = 17.5, 1.0 Hz, 1H), 3.48 (dd, *J* = 18.0, 7.0 Hz, 1H), 5.46 (ddd, *J* = 8.5, 7.0, 1.5 Hz, 1H), 5.72 (d, *J* = 8.0 Hz, 1H), 7.03 (ddd, *J* = 8.0, 2.5, 0.5 Hz, 1H), 7.13 (ddd, *J* = 8.0, 2.5, 1.0 Hz, 1H), 7.23 (ddd, *J* = 7.5, 4.5, 1.0 Hz, 1H), 7.25-7.28 (m, 3H), 7.34 (t, *J* = 8.5 Hz, 1H), 7.43 (t, *J =* 8.0 Hz, 1H), 7.54-7.56 (m, 1H), 7.61 (dd, *J* = 2.5, 1.5 Hz, 1H), 7.62 (t, *J* = 2.0 Hz, 1H), 7.67 (dt, *J* = 7.5, 1.0 Hz, 1H), 7.69 (dt, *J* = 8.0, 1.0 Hz, 1H), 7.72 (dd, *J* = 7.5, 2.0 Hz, 1H), 7.74-7.76 (m, 1H), 8.66 (ddd, *J* = 5.0, 1.5, 1.0 Hz, 1H). ¹³C NMR (125 MHz, CDCl₃): *δ* (ppm) 39.73, 76.97, 83.24, 117.52, 118.81, 119.40, 120.59, 121.86, 122.05, 122.34, 123.36, 125.23, 125.60, 127.42, 128.44, 129.64, 129.69, 130.10, 136.71, 139.67, 141.42, 141.85, 149.64, 156.60, 157.16, 157.49, 163.41. HRMS (ESI): calcd for C₂₇H₂₁N₂O₂ [M+H]⁺ 405.1598, found 405.1587.
(2) Synthesis of ***M*-PtB1:** *M*-B1 (243 mg, 0.60 mmol, 1.0 equiv.), potassium chloroplatinate (262 mg, 0.63 mmol, 1.05 equiv.), tetra-n-butylammonium bromide (19 mg, 0.060 mmol, 0.1 equiv.) were sequentially added into a 100 mL dry three-necked flask with a magnetic rotor and a condenser tube, followed by nitrogen purge for three times and addition of acetic acid (36 mL) pre-purged with nitrogen. After a reaction solution was bubbled with nitrogen for 30 minutes, it was stirred at the room temperature for 12 hours, and then was reacted with stirring at 100 °C for 3 days, cooled to the room temperature, and solvent was distilled off at a reduced pressure, and stannous chloride (228 mg, 1.2 mmol, 2.0 equiv.) and dichloromethane (60 mL) were added and stirred at the room temperature for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 1:1 so as to obtain a product *M*-PtB1, 106 mg as red solid, with a yield of 30%. ¹H NMR (500 MHz, CDCl₃): *δ* (ppm) 3.58 (dd, *J =* 18.0, 5.0 Hz, 1H), 3.64 (d, *J* = 18.0 Hz, 1H), 5.78 (d, *J* = 7.0 Hz, 1H), 5.85 (ddd, *J* = 6.5, 5.0, 1.0 Hz, 1H), 7.11-7.17 (m, 2H), 7.19 (dd, *J* = 10.5, 1.5 Hz, 1H), 7.21-7.22 (m, 1H), 7.24-7.28 (m, 3H), 7.30-7.34 (m, 2H), 7.47-7.49 (m, 1H), 7.57 (d, *J =* 7.5 Hz, 1H), 7.91-7.97 (m, 2H), 9.02 (d, *J* = 5.0 Hz, 1H). HRMS (ESI): calcd for C₂₇H₁₉N₂O₂Pt [M+H]⁺ 598.1089, found 598.1090.

**Example 25, not in accordance with the present invention and provided for illustrative purposes only:** synthesis of tetradentate cyclometalated platinum (II) complex ***P*-PtB2** as follow. 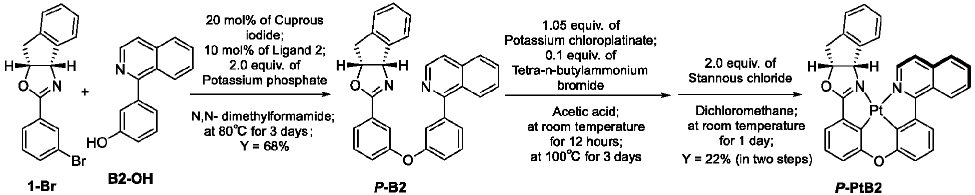
(1) Synthesis of ligand **P-B2:** 1-Br (689 mg, 2.20 mmol, 1.1 equivalent), 1-OH (443 mg, 2.00 mmol, 1.0 equivalent) and cuprous iodide (76 mg, 0.40 mmol, 20 mol%), ligand 2 (69 mg, 0.20 mmol, 10 mol%) and potassium phosphate (849 mg, 4.00 mmol, 2.0 equiv.) were sequentially added into a dry sealed tube with a magnetic rotor. Nitrogen was purged for three times, and N, N-dimethylformamide (5 mL) was added under nitrogen protection. The sealed tube was placed in an oil bath at 80°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***P*-B2,** 623 mg as a white solid, with a yield of 68%. ¹H NMR (500 MHz, CDCl₃): *δ* (ppm) 3.35 (dd, *J* = 13.0, 1.5 Hz, 1H), 3.49 (dd, *J* = 18.0, 7.0 Hz, 1H), 5.47 (ddd, *J* = 8.5, 7.0, 1.5 Hz, 1H), 5.73 (d, *J* = 8.0 Hz, 1H), 7.12 (ddd, *J* = 8.0, 2.5, 1.5 Hz, 1H), 7.17 (ddd, *J* = 8.0, 2.5, 1.0 Hz, 1H), 7.25-7.27 (m, 3H), 7.30-7.31 (m, 1H), 7.34 (t, *J* = 8.0 Hz, 1H), 7.44-7.51 (m, 3H), 7.53-7.56 (m, 1H), 7.63-7.67 (m, 3H), 7.68 (dt, *J* = 7.5, 1.0 Hz, 1H), 7.86 (d, *J* = 8.0 Hz, 1H), 8.08 (d, *J* = 8.5, 1.0 Hz, 1H), 8.58 (d, *J* = 6.0 Hz, 1H). ¹³C NMR (125 MHz, CDCl₃): *δ* (ppm) 39.72, 76.95, 83.26, 118.91, 118.92, 120.12, 120.33, 122.10, 123.48, 125.01, 125.23, 125.62, 126.55, 126.93, 127.28, 127.44, 128.45, 129.66, 129.69, 129.85, 130.02, 136.79, 139.67, 141.33, 141.82, 142.07, 157.00, 159.75, 163.38. HRMS (ESI): calcd for C₃₁H₂₃N₂O₂ [M+H]⁺ 455.1754, found 455.1748.
(2) Synthesis **of *P*-PtB2:** *P*-B1 (182 mg, 0.40 mmol, 1.0 equiv.), potassium chloroplatinate (174 mg, 0.42 mmol, 1.05 equiv.), tetra-n-butylammonium bromide (13 mg, 0.040 mmol, 0.1 equiv.) were sequentially added into a 50 mL dry three-necked flask with a magnetic rotor and a condenser tube, followed by nitrogen purge for three times and addition of acetic acid (24 mL) pre-purged with nitrogen. After a reaction solution was bubbled with nitrogen for 30 minutes, it was stirred at the room temperature for 12 hours, and then was reacted with stirring at 100 °C for 3 days, cooled to the room temperature, and solvent was distilled off at a reduced pressure, and stannous chloride (152 mg, 0.8 mmol, 2.0 equiv.) and dichloromethane (40 mL) were added and stirred at the room temperature for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 1:1 so as to obtain a product P-PtB2, 57 mg as red solid, with a yield of 22%. ¹H NMR (500 MHz, CDCl₃): *δ* (ppm) 3.57 (dd, *J* = 18.0, 4.0 Hz, 1H), 3.63 (d, *J* = 18.0 Hz, 1H), 5.80-5.83 (m, 2H), 7.09 (t, *J* = 7.5 Hz, 1H), 7.14 (t, *J* = 8.0 Hz, 1H), 7.20-7.24 (m, 3H), 7.25-7.28 (m, 1H), 7.31 (t, *J =* 6.5 Hz, 2H), 7.56 (d, *J* = 7.5 Hz, 1H), 7.59 (d, *J* = 6.0 Hz, 1H), 7.71-7.74 (m, 1H), 7.77 (t, *J* = 7.0 Hz, 1H), 7.88 (d, *J* = 8.0 Hz, 1H), 7.98 (d, *J* = 7.5 Hz, 1H), 8.89 (d, *J* = 6.0 Hz, 1H), 8.94 (d, *J* = 8.5 Hz, 1H). HRMS (ESI): calcd for C₃₁H₂₁N₂O₂Pt [M+H]⁺ 648.1245, found 648.1226.

**Example 26, not in accordance with the present invention and provided for illustrative purposes** only: synthesis of tetradentate cyclometalated platinum (II) complex **M-PtB2** as follow. 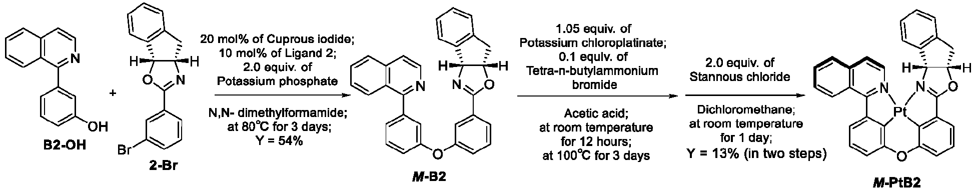
(1) Synthesis of ligand ***M-B2:*** 2-Br (689 mg, 2.20 mmol, 1.1 equivalent), 2-OH (443 mg, 2.00 mmol, 1.0 equivalent) and cuprous iodide (76 mg, 0.40 mmol, 20 mol%), ligand 2 (69 mg, 0.20 mmol, 10 mol%) and potassium phosphate (849 mg, 4.00 mmol, 2.0 equiv.) were sequentially added into a dry sealed tube with a magnetic rotor. Nitrogen was purged for three times, and *N, N-*dimethylformamide (5 mL) was added under nitrogen protection. The sealed tube was placed in an oil bath at 80°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***M*-B2,** 487 mg as a white solid, with a yield of 54%. ¹H NMR (500 MHz, CDCl₃): *δ* (ppm) 3.35 (dd, *J =* 13.0, 1.5 Hz, 1H), 3.49 (dd, *J* = 18.0, 7.0 Hz, 1H), 5.47 (ddd, *J* = 8.0, 7.0, 1.5 Hz, 1H), 5.73 (d, *J* = 8.0 Hz, 1H), 7.12 (ddd, *J* = 8.0, 2.5, 1.0 Hz, 1H), 7.18 (ddd, *J* = 8.0, 2.5, 1.0 Hz, 1H), 7.25-7.27 (m, 3H), 7.29-7.32 (m, 1H), 7.34 (t, *J* = 8.0 Hz, 1H), 7.44-7.52 (m, 3H), 7.53-7.57 (m, 1H), 7.62-7.67 (m, 3H), 7.68 (dt, *J* = 7.5, 1.0 Hz, 1H), 7.86 (d, *J* = 8.0 Hz, 1H), 8.04-8.09 (m, 1H), 8.58 (d, *J* = 6.0 Hz, 1H). ¹³C NMR (125 MHz, CDCl₃): *δ* (ppm) 39.74, 76.96, 83.27, 118.92, 118.93, 120.13, 120.35, 122.11, 123.49, 125.02, 125.24, 125.64, 126.57, 126.94, 127.30, 127.45, 128.46, 129.68, 129.70, 129.86, 130.03, 136.80, 139.68, 141.34, 141.83, 142.09, 156.98, 159.77, 163.39. HRMS (ESI): calcd for C₃₁H₂₃N₂O₂ [M+H]⁺ 455.1754, found 455.1742.
(2) Synthesis of ***M*-PtB2:** *M*-B2 (273 mg, 0.60 mmol, 1.0 equiv.), potassium chloroplatinate (262 mg, 0.63 mmol, 1.05 equiv.), tetra-n-butylammonium bromide (19 mg, 0.060 mmol, 0.1 equiv.) were sequentially added into a 100 mL dry three-necked flask with a magnetic rotor and a condenser tube, followed by nitrogen purge for three times and addition of acetic acid (36 mL) pre-purged with nitrogen. After a reaction solution was bubbled with nitrogen for 30 minutes, it was stirred at the room temperature for 12 hours, and then was reacted with stirring at 100 °C for 3 days, cooled to the room temperature, and solvent was distilled off at a reduced pressure, and stannous chloride (228 mg, 1.2 mmol, 2.0 equiv.) and dichloromethane (60 mL) were added and stirred at the room temperature for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 1:1 so as to obtain a product *M*-PtB2, 52 mg as red solid, with a yield of 13%. ¹H NMR (500 MHz, CDCl₃): *δ* (ppm) 3.59 (dd, *J* = 18.0, 4.0 Hz, 1H), 3.64 (d, *J* = 18.0 Hz, 1H), 5.82-5.85 (m, 2H), 7.11 (t, *J* = 7.5 Hz, 1H), 7.16 (t, *J* = 8.0 Hz, 1H), 7.21-7.26 (m, 3H), 7.27-7.30 (m, 1H), 7.33 (t, *J* = 6.5 Hz, 2H), 7.58 (d, *J* = 7.5 Hz, 1H), 7.61 (d, *J* = 6.0 Hz, 1H), 7.72-7.76 (m, 1H), 7.78-7.81 (m, 1H), 7.90 (d, *J* = 8.0 Hz, 1H), 8.00 (d, *J* = 7.5 Hz, 1H), 8.91 (d, *J* = 6.0 Hz, 1H), 8.96 (d, *J* = 8.5 Hz, 1H). HRMS (ESI): calcd for C₃₁H₂₁N₂O₂Pt [M+H]⁺ 648.1245, found 648.1230.

**Example 27, not in accordance with the present invention and provided for illustrative purposes only:** synthesis of tetradentate cyclometalated platinum (II) complex ***P*-PtB3** as follow. 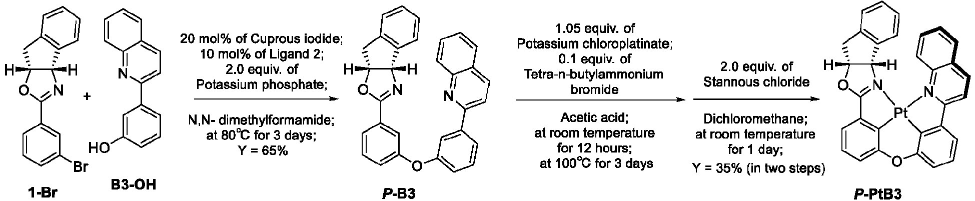
(1) Synthesis of ligand **P-B2:** 1-Br (689 mg, 2.20 mmol, 1.1 equivalent), B3-OH (443 mg, 2.00 mmol, 1.0 equivalent) and cuprous iodide (76 mg, 0.40 mmol, 20 mol%), ligand 2 (69 mg, 0.20 mmol, 10 mol%) and potassium phosphate (849 mg, 4.00 mmol, 2.0 equiv.) were sequentially added into a dry sealed tube with a magnetic rotor. Nitrogen was purged for three times, and N, N-dimethylformamide (5 mL) was added under nitrogen protection. The sealed tube was placed in an oil bath at 80°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***P*-B3,** 590 mg as a white solid, with a yield of 65%. ¹H NMR (500 MHz, CDCl₃): *δ* (ppm) 3.35 (dd, *J* = 18.0, 1.5 Hz, 1H), 3.49 (dd, *J* = 18.0, 7.0 Hz, 1H), 5.47 (ddd, *J* = 8.0, 7.0, 1.5 Hz, 1H), 5.72 (d, *J* = 8.0 Hz, 1H), 7.07 (ddd, *J* = 8.0, 2.5, 1.0 Hz, 1H), 7.16 (ddd, *J* = 8.0, 2.5, 1.0 Hz, 1H), 7.25-7.27 (m, 3H), 7.36 (t, *J* = 8.0 Hz, 1H), 7.49 (t, *J* = 8.0 Hz, 1H), 7.51-7.56 (m, 2H), 7.63 (dd, *J* = 2.5, 1.5 Hz, 1H), 7.70-7.74 (m, 2H), 7.80-7.83 (m, 3H), 7.93 (ddd, *J* = 8.0, 1.5, 1.0 Hz, 1H), 8.14 (d, *J* = 8.5 Hz, 1H), 8.21 (d, *J* = 8.5 Hz, 1H). ¹³C NMR (125 MHz, CDCl₃): *δ* (ppm) 39.69, 76.94, 83.22, 118.20, 118.70, 118.85, 119.77, 121.81, 122.72, 123.34, 125.22, 125.57, 126.38, 127.22, 127.37, 127.40, 128.42, 129.65, 129.67, 129.73, 130.19, 136.77, 139.65, 141.67, 141.81, 148.14, 156.38, 157.17, 157.45, 163.38. HRMS (ESI): calcd for C₃₁H₂₃N₂O₂ [M+H]⁺ 455.1754, found 455.1744.
(2) Synthesis of ***P*-PtB3:** *P*-B3 (182 mg, 0.40 mmol, 1.0 equiv.), potassium chloroplatinate (174 mg, 0.42 mmol, 1.05 equiv.), tetra-n-butylammonium bromide (13 mg, 0.040 mmol, 0.1 equiv.) were sequentially added into a 50 mL dry three-necked flask with a magnetic rotor and a condenser tube, followed by nitrogen purge for three times and addition of acetic acid (24 mL) pre-purged with nitrogen. After a reaction solution was bubbled with nitrogen for 30 minutes, it was stirred at the room temperature for 12 hours, and then was reacted with stirring at 100 °C for 3 days, cooled to the room temperature, and solvent was distilled off at a reduced pressure, and stannous chloride (152 mg, 0.8 mmol, 2.0 equiv.) and dichloromethane (40 mL) were added and stirred at the room temperature for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 1:1 so as to obtain a product P-PtB3, 91 mg as red solid, with a yield of 35%. ¹H NMR (500 MHz, CDCl₃): *δ* (ppm) 3.55 (d, *J* = 3.5 Hz, 2H), 5.90-5.93 (m, 1H), 6.23 (d, *J* = 7.0 Hz, 1H), 6.45 (d, *J* = 7.5 Hz, 1H), 6.73 (t, *J* = 7.5 Hz, 1H), 7.05 (t, *J* = 8.0 Hz, 1H), 7.15-7.20 (m, 2H), 7.25-7.30 (m, 4H), 7.54-7.58 (m, 1H), 7.65 (d, *J =* 7.5 Hz, 1H), 7.78 (ddd, *J* = 8.5, 7.0, 1.5 Hz, 1H), 7.92 (dd, *J* = 8.0, 1.0 Hz, 1H), 8.14 (d, *J* = 8.5 Hz, 1H), 8.42 (d, *J* = 8.5 Hz, 1H), 9.03 (d, *J* = 8.5 Hz, 1H). HRMS (ESI): calcd for C₃₁H₂₁N₂O₂Pt [M+H]⁺ 648.1245, found 648.1242.

**Example 28, not in accordance with the present invention and provided for illustrative purposes only:** synthesis of tetradentate cyclometalated platinum (II) complex **M-PtB3** as follow. 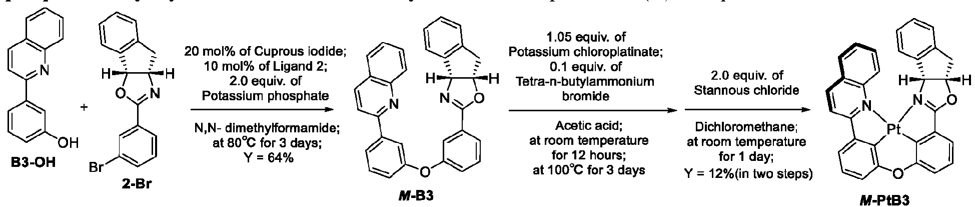
(1) Synthesis of ligand ***M*-B3:** 2-Br (689 mg, 2.20 mmol, 1.1 equivalent), B3-OH (443 mg, 2.00 mmol, 1.0 equivalent) and cuprous iodide (76 mg, 0.40 mmol, 20 mol%), ligand 2 (69 mg, 0.20 mmol, 10 mol%) and potassium phosphate (849 mg, 4.00 mmol, 2.0 equiv.) were sequentially added into a dry sealed tube with a magnetic rotor. Nitrogen was purged for three times, and N, N-dimethylformamide (5 mL) was added under nitrogen protection. The sealed tube was placed in an oil bath at 80°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***M*-B3,** 590 mg as a white solid, with a yield of 64%. ¹H NMR (500 MHz, CDCl₃): *δ* (ppm) 3.35 (dd, *J* = 18.0, 1.5 Hz, 1H), 3.48 (dd, *J* = 18.0, 7.0 Hz, 1H), 5.47 (ddd, *J* = 8.0, 7.0, 1.5 Hz, 1H), 5.72 (d, *J* = 8.0 Hz, 1H), 7.07 (ddd, *J* = 8.0, 2.5, 1.0 Hz, 1H), 7.16 (ddd, *J* = 8.0, 2.5, 1.0 Hz, 1H), 7.25-7.27 (m, 3H), 7.36 (t, *J* = 8.0 Hz, 1H), 7.49 (t, *J* = 8.0 Hz, 1H), 7.51-7.56 (m, 2H), 7.63 (dd, *J* = 2.5, 1.5 Hz, 1H), 7.70-7.74 (m, 2H), 7.80-7.83 (m, 3H), 7.93 (ddd, *J=* 8.0, 1.5, 1.0 Hz, 1H), 8.14 (d, *J* = 8.5 Hz, 1H), 8.21 (d, *J* = 8.5 Hz, 1H). ¹³C NMR (125 MHz, CDCl₃): *δ* (ppm) 39.73, 76.96, 83.25, 118.23, 118.74, 118.90, 119.79, 121.84, 122.75, 123.37, 125.24, 125.60, 126.41, 127.26, 127.40, 127.43, 128.44, 129.68, 129.76, 130.21, 136.81, 139.68, 141.70, 141.84, 148.17, 156.44, 157.19, 157.48, 163.42. HRMS (ESI): calcd for C₃₁H₂₃N₂O₂ [M+H]⁺ 455.1754, found 455.1744.
(2) Synthesis of ***M*-PtB3:** *M-B3* (273 mg, 0.60 mmol, 1.0 equiv.), potassium chloroplatinate (262 mg, 0.63 mmol, 1.05 equiv.), tetra-n-butylammonium bromide (19 mg, 0.060 mmol, 0.1 equiv.) were sequentially added into a 100 mL dry three-necked flask with a magnetic rotor and a condenser tube, followed by nitrogen purge for three times and addition of acetic acid (36 mL) pre-purged with nitrogen. After a reaction solution was bubbled with nitrogen for 30 minutes, it was stirred at the room temperature for 12 hours, and then was reacted with stirring at 100 °C for 3 days, cooled to the room temperature, and solvent was distilled off at a reduced pressure, and stannous chloride (228 mg, 1.2 mmol, 2.0 equiv.) and dichloromethane (60 mL) were added and stirred at the room temperature for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 1:1 so as to obtain a product M-PtB3, 46 mg as red solid, with a yield of 12%. ¹H NMR (500 MHz, CDCl₃): *δ* (ppm) 3.55 (d, *J* = 3.5 Hz, 2H), 5.89-5.92 (m, 1H), 6.23 (d, *J* = 7.0 Hz, 1H), 6.45 (d, *J* = 7.5 Hz, 1H), 6.73 (t, *J* = 7.0 Hz, 1H), 7.05 (t, *J* = 7.5 Hz, 1H), 7.16-7.20 (m, 2H), 7.25-7.30 (m, 4H), 7.56 (ddd, *J* = 8.0, 7.0, 1.0 Hz, 1H), 7.65 (d, *J* = 7.0 Hz, 1H), 7.78 (ddd, *J* = 8.5, 7.0, 1.5 Hz, 1H), 7.92 (dd, *J* = 8.0, 1.0 Hz, 1H), 8.14 (d, *J* = 9.0 Hz, 1H), 8.42 (d, *J* = 8.5 Hz, 1H), 9.03 (d, *J* = 8.5 Hz, 1H). HRMS (ESI): calcd for C₃₁H₂₁N₂O₂Pt [M+H]⁺ 648.1245, found 648.1253.

**Example 29, not in accordance with the present invention and provided for illustrative purposes only:** synthesis of tetradentate cyclometalated platinum (II) complex **M-PtB8** as follow. 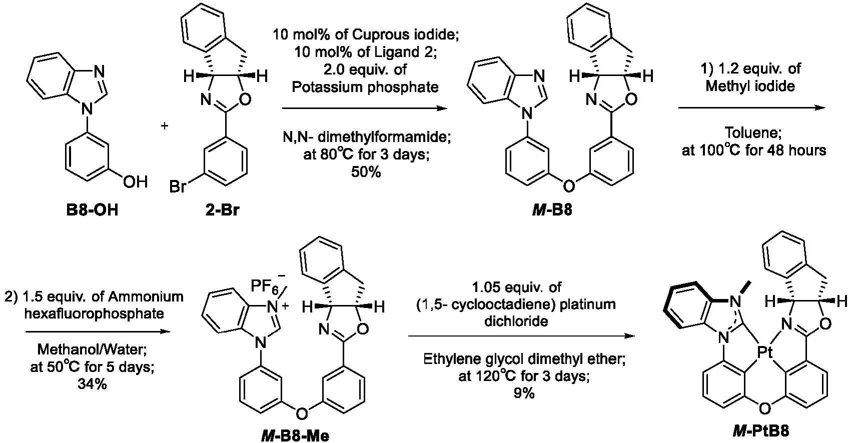
(1) Synthesis of ***M*-B8:** 2-Br (1.50 g, 4.77 mmol, 1.0 equivalent), **B8-OH** (1.0 g, 4.77 mmol, 1.0 equivalent) and cuprous iodide (91 mg, 0.48 mmol, 10 mol%), ligand 2 (165 mg, 0.48 mmol, 10 mol%) and potassium phosphate (2.02 g, 9.54 mmol, 2.0 equiv.) were sequentially added into a dry sealed tube with a magnetic rotor. Nitrogen was purged for three time, and N, N- dimethylformamide (30 mL) was added under nitrogen protection. The sealed tube was placed in an oil bath at 80°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ***M*-B8,** 1.05 g as white solid, with a yield of 50%. ¹H NMR (400 MHz, DMSO-*d*₆) δ 3.23 (d, *J* = 18.0 Hz, 1H), 3.49 (dd, *J* = 18.0, 6.8 Hz, 1H), 5.53 (t, *J* = 7.2 Hz, 1H), 5.71 (d, *J* = 7.6 Hz, 1H), 7.12 (d, *J* = 8.4 Hz, 1H), 7.21-7.35 (m, 6H), 7.37-7.45 (m, 2H), 7.46-7.56 (m, 3H), 7.60-7.68 (m, 3H), 7.77 (d, *J* = 7.6 Hz, 1H), 8.59 (s, 1H).
(2) Synthesis of Ligand ***M*-B8-Me:** M-B8 (1.0 g, 2.25 mmol, 1.0 equiv.) was sequentially added into a dry sealed tube with a magnetic rotor, and nitrogen was purged for three times, and toluene (30 mL) and methyl iodide (384 mg, 2.71 mmol, 1.2 equiv.) were added under nitrogen protection. The sealed tube was stirred in an oil bath at 100°C for reaction for 2 days, cooled to the room temperature, and filtered after adding water. The solid was transferred to the sealed tube and methanol (30 mL) was added. After dissolution, a solution of ammonium hexafluorophosphate (550 mg, 3.38 mmol, 1.5 equivalents) in water (10 mL) was added, which was reacted at 50°C for 5 days and cooled to the room temperature, and the solvent was distilled off at a reduced pressure. The obtained crude was separated and purified by a silica gel chromatography column with a eluent of methanol/dichloromethane of 100: 1 to 5: 1 so as to obtain ligand M-B8-Me, 460 mg as white solid, with a yield of 34%. ¹H NMR (400 MHz, DMSO-*d*₆) δ 3.22 (d, *J* = 18.0 Hz, 1H), 3.49 (dd, *J* = 18.0, 6.8 Hz, 1H), 4.13 (s, 3H), 5.53 (ddd, *J* = 8.0, 6.8, 1.6 Hz, 1H), 5.71 (d, *J* = 7.6 Hz, 1H), 7.21-7.31 (m, 3H), 7.35-7.39 (m, 2H), 7.40-7.43 (m, 1H), 7.47 (t, *J* = 2.4 Hz, 1H), 7.52-7.57 (m, 2H), 7.60 (ddd, *J* = 8.0, 2.0, 0.8 Hz, 1H), 7.66 (ddd, *J* = 8.4, 7.2, 1.2 Hz, 1H), 7.70 (dt, *J* = 7.6, 1.2 Hz, 1H), 7.74-7.79 (m, 2H), 7.84 (dt, *J* = 8.4, 0.8 Hz, 1H), 8.12 (dt, *J* = 8.4, 0.8 Hz, 1H), 10.08 (s, 1H).
(3) Synthesis of *M-PtB8:* **M-B8-Me** (237 mg, 0.39 mmol, 1.0 equiv.), (1,5- cyclooctadiene) platinum dichloride (154 mg, 0.41 mmol, 1.05 equiv.) and sodium acetate (160 mg, 1.18 mmol, 3.0 equiv.) were sequentially added into a 100 mL dry three-necked flask with a magnetic rotor and a condenser tube. The nitrogen was purged for three times, and ethylene glycol dimethyl ether (25 mL) was added. After the reaction solution was bubbled with nitrogen for 30 minutes, this mixture was reacted at 120 C for 72 hours, cooled to the room temperature, quenched with water, and extracted with DCM, and then the solvent was distilled off at a reduced pressure. The crude was separated and purified by a silica gel chromatography column with an eluent of petroleum ether/dichloromethane of 1:1 so as to obtain a product *M*-PtB8, 23 mg as yellow solid, with a yield of 9%. ¹H NMR (400 MHz, DMSO-*d*₆) δ 3.39 (d, *J* = 14 Hz, 1H), 3.46-3.52 (m, 1H), 4.36 (s, 3H), 5.92-6.00 (m, 1H), 6.17 (d, *J* = 6.8 Hz, 1H), 6.93 (d, *J* = 8.0 Hz, 1H), 7.13-7.31 (m, 6H), 7.39 (d, *J* = 7.6 Hz, 1H), 7.48-7.54 (m, 2H), 7.57 (d, *J* = 7.6 Hz, 1H), 7.73 (d, *J* = 7.6 Hz, 1H), 7.81-7.86 (m, 1H), 8.36-8.38 (m, 1H).

**Example 30, not in accordance with the present invention and provided for illustrative purposes only:** synthesis of tetradentate cyclometalated platinum (II) complex ***P*-PtB9** as follow. 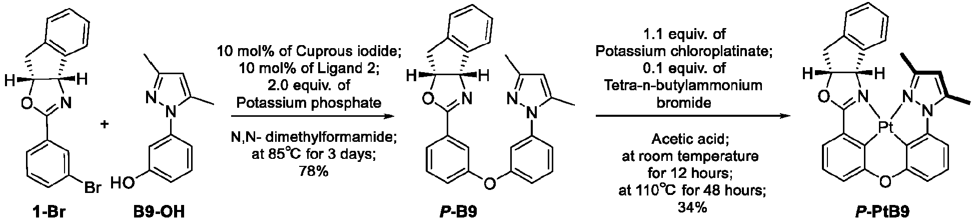
(1) Synthesis of ligand *P*-B9: **1-Br** (500 mg, 1.60 mmol, 1.0 equivalent), **B9-OH** (301 mg, 1.60 mmol, 1.0 equivalent) and cuprous iodide (31 mg, 0.16 mmol, 10 mol%), ligand 2 (55 mg, 0.16 mmol, 10 mol%) and potassium phosphate (680 mg, 3.20 mmol, 2.0 equiv.) were sequentially added into a dry sealed tube with a magnetic rotor. Nitrogen was purged for three times, and N, N-dimethylformamide (10 mL) was added under nitrogen protection. The sealed tube was placed in an oil bath at 85°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***P*-B9,** 526mg as a white solid, with a yield of 78%. ¹H NMR (400 MHz, DMSO-*d*₆) δ 2.14 (s, 3H), 2.27 (s, 3H), 3.21 (d, *J* = 18.0 Hz, 1H), 3.42-3.47 (m, 1H), 5.51 (t, *J* = 7.2 Hz, 1H), 5.68 (d, *J* = 7.6 Hz, 1H), 6.05 (s, 1H), 7.02 (d, *J* = 7.6 Hz, 1H), 7.12-7.14 (m, 1H), 7.21-7.32 (m, 5H), 7.40-7.43 (m, 2H), 7.50 (t, *J* = 8.0 Hz, 2H), 7.64 (d, *J* = 7.6 Hz, 1H). ¹³C NMR (125 MHz, CDCl₃) δ 12.42, 13.43, 39.70, 76.94, 83.26, 107.19, 114.91, 117.16, 119.05, 119.43, 122.19, 123.74, 125.24, 125.56, 127.42, 128.45, 129.72, 129.74, 130.08, 139.35, 139.64, 141.19, 141.78, 149.08, 156.49, 157.51, 163.25.
(2) Synthesis of ***P*-PtB9:** P-B9 (200 mg, 0.48 mmol, 1.0 equiv.), potassium chloroplatinate (208 mg, 0.50 mmol, 1.05 equiv.), tetra-n-butylammonium bromide (15 mg, 0.048 mmol, 0.1 equiv.) were sequentially added into a 100 mL dry three-necked flask with a magnetic rotor and a condenser tube, and nitrogen was purged for three times, acetic acid (30 mL) was added, the reaction solution was then bubbled with nitrogen for 30 minutes, stirred at the room temperature for 12 hours, reacted at 110 °C for 48 hours and cooled to the room temperature, and then the solvent was distilled off at a reduced pressure. The crude was separated and purified by a silica gel chromatography column with an eluent of petroleum ether/dichloromethane of 1:1 so as to obtain a product *P*-PtB9, 100 mg as yellow solid, with a yield of 34%. ¹H NMR (400 MHz, DMSO-*d*₆) δ 2.74 (s, 3H), 2.78 (s, 3H), 3.49 (d, *J* = 14 Hz, 1H), 3.56-3.62 (m, 1H), 5.89-5.94 (m, 1H), 5.98 (d, *J =* 6.8 Hz, 1H), 6.51 (s, 1H), 6.89 (dd, *J* = 8.0, 0.8 Hz, 1H), 7.08-7.15 (m, 3H), 7.17-7.23 (m, 2H), 7.27-7.31 (m, 2H), 7.39 (d, *J* = 7.6 Hz, 1H), 7.44 (d, *J* = 7.6 Hz, 1H). ¹³C NMR (125 MHz, CDCl₃) δ 14.49, 15.06, 38.25, 73.92, 88.08, 106.69, 109.71, 111.11, 113.94, 120.48, 121.45, 123.38, 123.94, 125.18, 125.34, 125.51, 128.07, 129.00, 133.24, 139.27, 140.27, 141.04, 147.62, 149.57, 150.57, 152.44, 180.59.

**Example 31, not in accordance with the present invention and provided for illustrative purposes only:** synthesis of tetradentate cyclometalated platinum (II) complex **M-PtB9** as follow. 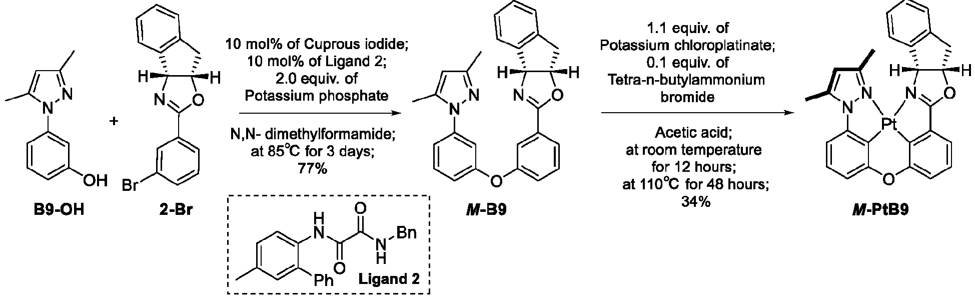
(1) Synthesis of ligand ***M*-B9: 2-Br** (500 mg, 1.60 mmol, 1.0 equivalent), **B9-OH** (301 mg, 1.60 mmol, 1.0 equivalent) and cuprous iodide (31 mg, 0.16 mmol, 10 mol%), ligand 2 (55 mg, 0.16 mmol, 10 mol%) and potassium phosphate (680 mg, 3.20 mmol, 2.0 equiv.) were sequentially added into a dry sealed tube with a magnetic rotor. Nitrogen was purged for three times, and N, N-dimethylformamide (10 mL) was added under nitrogen protection. The sealed tube was placed in an oil bath at 85°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain ligand ***M*-B9,** 514 mg as a white solid, with a yield of 77%. ¹H NMR (500 MHz, CDCl₃) δ 2.26 (s, 3H), 2.27 (s, 3H), 3.33-3.37 (m, 1H), 3.49 (dd, *J* = 18.0, 7.0 Hz, 1H), 5.47 (ddd, *J* = 8.0, 7.0, 1.5 Hz, 1H), 5.72 (d, *J* = 8.0 Hz, 1H), 5.96 (s, 1H), 6.94 (ddd, *J* = 8.0, 2.5, 1.0 Hz, 1H), 7.04 (t, *J* = 2.0 Hz, 1H), 7.13 (ddd, *J* = 8.0, 2.5, 1.0 Hz, 1H), 7.19 (ddd, *J* = 8.0, 2.0, 1.0 Hz, 1H), 7.26-7.29 (m, 3H), 7.33-7.36 (m, 1H), 7.38 (t, *J* = 7.0 Hz, 1H), 7.53-7.56 (m, 1H), 7.60-7.61 (m, 1H), 7.71 (dt, *J* = 7.5, 1.0 Hz, 1H). ¹³C NMR (125 MHz, CDCl₃) δ 12.34, 13.36, 39.62, 76.87, 83.19, 107.14, 114.83, 117.08, 118.96, 119.34, 122.10, 123.66, 125.16, 125.48, 127.33, 128.37, 129.67, 130.01, 139.26, 139.55, 141.14, 141.71, 148.99, 156.43, 157.43, 163.14.
(2) Synthesis of ***M*-PtB9: *M*-B9** (200 mg, 0.48 mmol, 1.0 equiv.), potassium chloroplatinate (208 mg, 0.50 mmol, 1.05 equiv.), tetra-n-butylammonium bromide (15 mg, 0.048 mmol, 0.1 equiv.) were sequentially added into a 100 mL dry three-necked flask with a magnetic rotor and a condenser tube, and nitrogen was purged for three times, acetic acid (30 mL) was added, the reaction solution was then bubbled with nitrogen for 30 minutes, stirred at the room temperature for 12 hours, reacted at 110 °C for 48 hours and cooled to the room temperature, and then the solvent was distilled off at a reduced pressure. The crude was separated and purified by a silica gel chromatography column with an eluent of petroleum ether/dichloromethane of 1:1 so as to obtain a product ***M*-PtB9,** 101 mg as yellow solid, with a yield of 34%. ¹H NMR (400 MHz, DMSO-*d*₆) δ 2.74 (s, 3H), 2.78 (s, 3H), 3.49 (d, *J* = 14 Hz, 1H), 3.56-3.62 (m, 1H), 5.89-5.94 (m, 1H), 5.98 (d, *J* = 6.8 Hz, 1H), 6.51 (s, 1H), 6.89 (d, *J* = 8.0 Hz, 1H), 7.08-7.15 (m, 3H), 7.17-7.23 (m, 2H), 7.27-7.31 (m, 2H), 7.39 (d, *J* = 7.6 Hz, 1H), 7.44 (d, *J* = 7.6 Hz, 1H). ¹³C NMR (125 MHz, CDCl₃) δ 14.53, 15.08, 38.28, 73.95, 88.10, 106.71, 109.74, 111.06, 113.99, 120.55, 121.47, 123.43, 123.98, 125.19, 125.36, 125.43, 128.11, 129.04, 133.20, 139.27, 140.26, 141.10, 147.63, 149.55, 150.59, 152.46, 180.67.

**Example 32, not in accordance with the present invention and provided for illustrative purposes only:** synthesis of tetradentate cyclometalated platinum (II) complex **(R, S)-*M*-PtLI1** as follow. 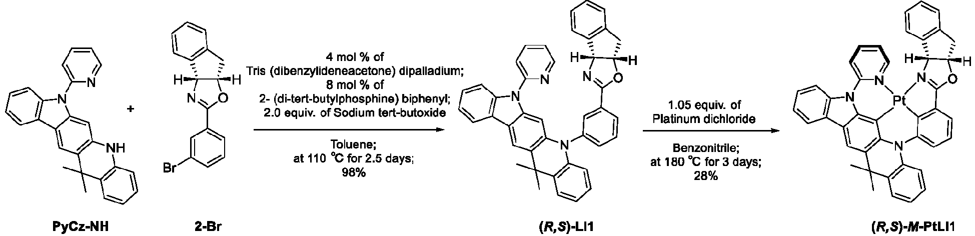
(1) Synthesis of ligand **(*R, S*)-LI1: PyCz-NH** (409 mg, 1.09 mmol, 1.0 equiv.), **2-Br** (515 mg, 1.64 mmol, 1.5 equiv.), tris (dibenzylideneacetone) dipalladium (40 mg, 0.044 mmol, 4 mol%), 2-(di-tert-butylphosphine) biphenyl (26 mg, 0.088 mmol, 8 mol%) and sodium tert-butoxide (209 mg, 2.18 mmol, 2.0 equivalents) were sequentially added into a dry sealed tube with a magnetic rotor, and then nitrogen was purged for three times and toluene (15 mL) was added under nitrogen protection. This mixture was reacted with stirring in an oil bath at 110 °C for 2.5 days, cooled to the room temperature, and the solvent was distilled off at a reduced pressure. The crude was separated and purified by a silica gel chromatography column with an eluent of petroleum ether/ethyl acetate of 10: 1 to 5: 1 so as to obtain a product **(*R, S*)-LI1,** 651mg as a white solid, with a yield of 98%. ¹H NMR (400 MHz, CDCl₃): δ 1.80 (s, 6H), 3.34-3.38 (m, 1H), 3.47-3.52 (m, 1H), 5.48-5.52 (m,1H), 5.77 (d, *J =* 8.0 Hz, 1H), 6.24-6.29 (m, 1H), 6.72 (s, 1H), 6.95-7.00 (m, 3H), 7.28-7.33 (m, 6H), 7.44 (td, *J* = 7.6, 2.0 Hz, 1H), 7.51-7.54 (m, 2H ), 7.58-7.59 (m, 1H), 7.64 (t, *J* = 8.0 Hz, 1H), 7.78 (d, *J* = 8.0 Hz, 1H),7.98 (t, *J* = 1.6 Hz, 1H), 8.02-8.04 (m, 1H), 8.09-8.13 (m, 2H), 8.35 (dd, *J* = 4.8, 1.2 Hz, 1H).
(2) Synthesis of **(*R, S*)-*M*-PtLI1:** ligand **(*R, S*)-LI1** (304 mg, 0.50 mmol, 1.0 equiv.) and platinum dichloride (141 mg, 0.53 mmol, 1.05 equiv) were sequentially added into a dry three-necked flask with a magnetic rotor. Nitrogen was purged for three times, benzonitrile (15 mL) was added under nitrogen protection. This mixture was reacted with stirring in an electric heating jacket at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product **(*R***, ***S*)-*M*-PtLI1,** 112 mg as yellow solid, with a yield of 28%. ¹H NMR (500 MHz, DMSO-*d*₆): δ 1.76 (s, 6H), 3.25-3.29 (m, 1H), 3.47-3.52 (m, 1H), 5.53-5.57 (m,1H), 5.74 (d, *J* = 8.0 Hz, 1H), 6.15 (dd, *J* = 7.5, 2.0 Hz, 1H), 6.68 (s, 1H), 6.93-7.00 (m, 2H), 7.13-7.16 (m, 1H), 7.22-7.33 (m, 5H), 7.43-7.46 (m, 2H), 7.57 (dd, *J* = 7.5, 2.0 Hz, 1H), 7.61-7.70 (m, 3H), 7.77 (t, *J* = 7.5 Hz, 1H), 7.81 (t, *J* = 1.5 Hz, 1H), 8.03 (dt, *J* = 8.0, 1.5 Hz, 1H), 8.19-8.22 (m, 2H), 8.35 (s, 1H).

**Example 33, not in accordance with the present invention and provided for illustrative purposes only:** synthesis of tetradentate cyclometalated platinum (II) complex **(R, S)-*P*-PtLI1** as follow. 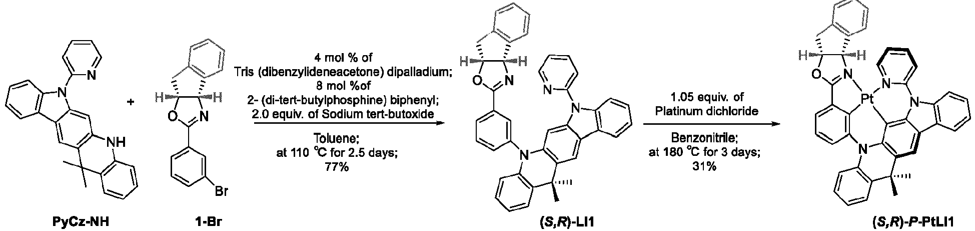
(1) Synthesis of ligand **(*S*, *R*)-LI1: PyCz-NH** (409 mg, 1.09 mmol, 1.0 equiv.), **2-Br** (515 mg, 1.64 mmol, 1.5 equiv.), tris (dibenzylideneacetone) dipalladium (40 mg, 0.044 mmol, 4 mol%), 2-(di-tert-butylphosphine) biphenyl (26 mg, 0.088 mmol, 8 mol%) and sodium tert-butoxide (209 mg, 2.18 mmol, 2.0 equivalents) were sequentially added into a dry sealed tube with a magnetic rotor, and then nitrogen was purged for three times and toluene (15 mL) was added under nitrogen protection. This mixture was reacted with stirring in an oil bath at 110 °C for 2.5 days, cooled to the room temperature, and the solvent was distilled off at a reduced pressure. The crude was separated and purified by a silica gel chromatography column with an eluent of petroleum ether/ethyl acetate of 10: 1 to 5: 1 so as to obtain a product **(*S*, *R*)-LI1,** 511mg as white solid, with a yield of 77%. ¹H NMR (400 MHz, CDCl₃): δ 1.81 (s, 6H), 3.34-3.38 (m, 1H), 3.47-3.53 (m, 1H), 5.48-5.52 (m,1H), 5.77 (d, *J* = 8.0 Hz, 1H), 6.24-6.28 (m, 1H), 6.72 (s, 1H), 6.94-7.0 (m, 3H), 7.27-7.33 (m, 6H), 7.44 (td, *J* = 7.6, 2.0 Hz, 1H), 7.51-7.56 (m, 2H), 7.57-7.59 (m, 1H), 7.64 (t, *J* = 8.0 Hz, 1H), 7.78 (d, *J* = 7.6 Hz, 1H),7.99 (t, *J* = 1.6 Hz, 1H), 8.02-8.04 (m, 1H), 8.09-8.13 (m, 2H), 8.35 (dd, *J* = 4.8, 1.2 Hz, 1H).
(2) Synthesis of **(*S, R*)-*P*-PtLI1:** ligand **(*S, R*)-LI1** (300 mg, 0.49 mmol, 1.0 equiv.) and platinum dichloride (136 mg, 0.51 mmol, 1.05 equiv) were sequentially added into a dry three-necked flask with a magnetic rotor. Nitrogen was purged for three times, benzonitrile (15 mL) was added under nitrogen protection. This mixture was reacted with stirring in an electric heating jacket at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product **(*S*, *R*)-*P*-PtLI1,** 122mg as yellow solid, with a yield of 31%. ¹H NMR (500 MHz, DMSO-*d*₆): δ 1.76 (s, 6H), 3.25-3.29 (m, 1H), 3.47-3.52 (m, 1H), 5.54-5.57 (m,1H), 5.74 (d, *J* = 7.5 Hz, 1H), 6.15 (dd, *J* = 8.0, 1.5 Hz, 1H), 6.68 (s, 1H), 6.93-7.00 (m, 2H), 7.13-7.16 (m, 1H), 7.23-7.34 (m, 5H), 7.44-7.46 (m, 2H), 7.57 (dd, *J* = 7.5, 2.0 Hz, 1H), 7.61-7.70 (m, 3H), 7.77 (t, *J* = 8.0 Hz, 1H), 7.81 (t, *J* = 2.0 Hz, 1H), 8.03 (dt, *J =* 8.0, 1.5 Hz, 1H), 8.19-8.22 (m, 2H), 8.35 (s, 1H).

**Example 34, not in accordance with the present invention and provided for illustrative purposes only:** synthesis of tetradentate cyclometalated platinum (II) complex **(R, S)-*M*-PtLK1** as follow. 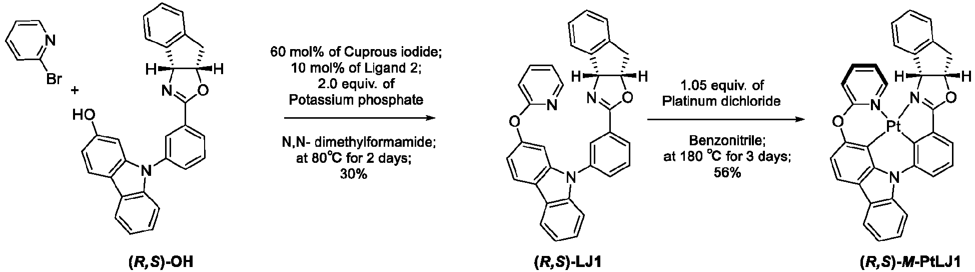
(1) Synthesis of **(*R, S*)-LJ1:** ligand **(*R, S*)-OH** (512 mg, 1.23 mmol, 1.0 equiv.), 2-bromopyridine (389 mg, 2.46 mmol, 2.0 equiv.), cuprous iodide (141 mg, 0.74 mmol, 60 mol%), ligand 2 (41 mg, 0.12 mmol, 10 mol%) and potassium phosphate (522 mg, 2.46 mmol, 2.0 equiv) were sequentially added into a dry three-necked flask with a magnetic rotor. Nitrogen was purged for three times and N, N- dimethylformamide (15 mL) was added under nitrogen protection. This mixture was placed in an oil bath at 80°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*R, S*)-LJ1,** 182 mg as a white solid, with a yield of 30%. ¹H NMR (500 MHz, DMSO-*d₆*): δ 3.22-3.26 (m, 1H), 3.47-3.52 (m, 1H), 5.56 (t, *J =* 8.0 Hz, 1H), 5.73 (d, *J =* 8.0 Hz, 1H), 7.01-7.12 (m, 4H), 7.21-7.34 (m, 5H), 7.42 (t, *J =* 8.0 Hz, 2H), 7.74 (t, *J = 7.5* Hz, 1H), 7.80-7.84 (m, 2H ), 7.95-7.98 (m, 2H ), 8.10(d, *J* = 4.0 Hz, 1H), 8.23-8.28 (m, 2H ).
(2) Synthesis of **(*R,* S)-M-PtLJ1:** ligand **(*R,* S)-LJ1** (160 mg, 0.32 mmol, 1.0 equiv.) and platinum dichloride (90 mg, 0.34 mmol, 1.05 equiv) were sequentially added into a dry three-necked flask with a magnetic rotor. Nitrogen was purged for three times, benzonitrile (10 mL) was added under nitrogen protection. This mixture was reacted with stirring in an oil bath at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product **(*R*, S)-*M*-PtLJ1,** 123mg as yellow solid, with a yield of 56%. ¹H NMR (400 MHz, DMSO-*d₆*): 3.50-3.56 (m, 1H), 3.60-3.66 (m, 1H), 6.00 (t, *J* = 8.0 Hz, 1H), 6.21 (d, *J* = 6.8 Hz, 1H), 6.45 (d, *J* = 8.0 Hz, 1H), 7.05 (t, *J* = 7.6 Hz, 1H), 7.20-7.25 (m, 2H), 7.28-7.48 (m, 6H), 7.69 (d, *J =* 8.0 Hz, 1H), 7.94 (d, *J* = 8.4 Hz, 1H), 8.20-8.31 (m, 4H), 9.25 (dd, *J* = 4.4, 1.6Hz, 1H).

**Example 35, not in accordance with the present invention and provided for illustrative purposes only:** synthesis of tetradentate cyclometalated platinum (II) complex **(R, S)-*P*-PtLK1** as follow. 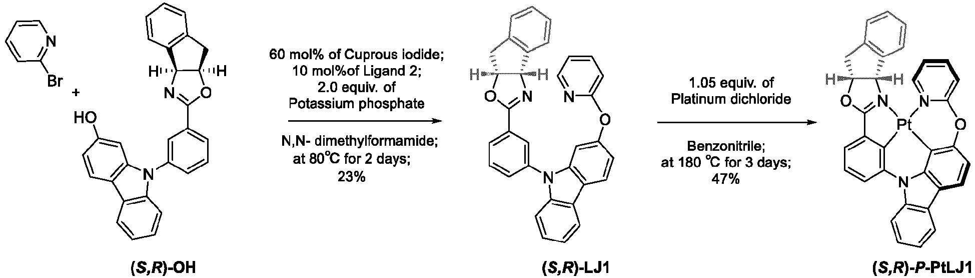
(1) Synthesis of **(*S, R*)-LJ1:** ligand **(*S*, *R*)-OH** (550 mg, 1.32 mmol, 1.0 equiv.), 2-bromopyridine (259 mg, 2.64 mmol, 2.0 equiv.), cuprous iodide (150 mg, 0.79 mmol, 60 mol%), ligand 2 (45 mg, 0.13 mmol, 10 mol%) and potassium phosphate (560 mg, 2.64 mmol, 2.0 equiv) were sequentially added into a dry three-necked flask with a magnetic rotor. Nitrogen was purged for three times and N, N- dimethylformamide (15 mL) was added under nitrogen protection. This mixture was placed in an oil bath at 80°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*S*, *R*)-LJ1,** 150 mg as white solid, with a yield of 23%. ¹H NMR (500 MHz, DMSO-*d₆*): δ 3.22-3.26 (m, 1H), 3.47-3.52 (m, 1H), 5.56 (t, *J =* 8.0 Hz, 1H), 5.73 (d, *J =* 8.0 Hz, 1H), 7.01-7.12 (m, 4H), 7.21-7.34 (m, 5H), 7.42 (t, *J* = 8.0 Hz, 2H), 7.74 (t, *J =* 7.5 Hz, 1H), 7.80-7.84 (m, 2H), 7.95-7.98 (m, 2H), 8.10(d, *J* = 4.0 Hz, 1H), 8.23-8.28 (m, 2H).
(2) Synthesis of ***(S, R*)-P-PtLJ1:** ligand **(*S*, *R*)-LJ1** (150 mg, 0.30 mmol, 1.0 equiv.) and platinum dichloride (85mg, 0.32 mmol, 1.05 equiv) were sequentially added into a dry three-necked flask with a magnetic rotor. Nitrogen was purged for three times, benzonitrile (10 mL) was added under nitrogen protection. This mixture was reacted with stirring in an oil bath at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product **(*S*, *R*)-*P*-PtLJ1,** 97mg as yellow solid, with a yield of 47%. ¹H NMR (500 MHz, DMSO-*d₆*): δ 3.51-3.54 (m, 1H), 3.60-3.65 (m, 1H), 6.00 (t, *J =* 6.5 Hz, 1H), 6.21 (d, *J =* 6.8 Hz, 1H), 6.46 (d, *J =* 8.0 Hz, 1H), 7.05 (t, *J =* 7.5 Hz, 1H), 7.20-7.25 (m, 2H), 7.29-7.48 (m, 6H), 7.68-7.70 (m, 1H), 7.93 (d, *J =* 8.0 Hz, 1H), 8.20-8.30 (m, 4H), 9.25 (dd, *J =* 6.0, 1.5Hz, 1H).

**Example 36, not in accordance with the present invention and provided for illustrative purposes only:** synthesis of tetradentate cyclometalated platinum (II) complex **(R, S)-*M*-PtLK1** as follow. 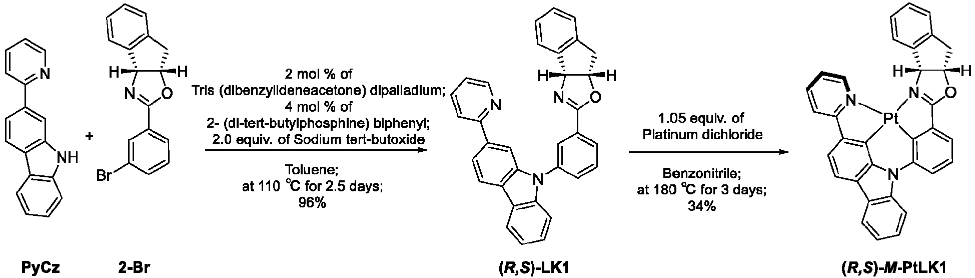
(1) Synthesis of ligand **(*R*, *S*)-LK1: PyCz** (300 mg, 1.23 mmol, 1.0 equiv.), **1-Br** (578 mg, 1.84 mmol, 1.5 equiv.), tris (dibenzylideneacetone) dipalladium (21 mg, 0.023 mmol, 2 mol%), 2-(di-tert-butylphosphine) biphenyl (14 mg, 0.046 mmol, 4 mol%) and sodium tert-butoxide (236 mg, 2.46 mmol, 2.0 equivalents) were sequentially added into a dry sealed tube with a magnetic rotor, and then nitrogen was purged for three times and toluene (15 mL) was added under nitrogen protection. This mixture was reacted with stirring in an oil bath at 110 °C for 3 days, cooled to the room temperature, and the solvent was distilled off at a reduced pressure. The crude was separated and purified by a silica gel chromatography column with an eluent of petroleum ether/ethyl acetate of 10: 1 to 5: 1 so as to obtain a product **(*R*, S)-LK1,** 564 mg as a white solid, with a yield of 96%. ¹H NMR (400 MHz, CDCl₃): δ 3.34-3.39 (m, 1H), 3.47-3.53 (m, 1H), 5.49-5.53 (m,1H), 5.78 (d, *J=* 7.6 Hz, 1H), 7.19-7.22 (m, 1H), 7.27-7.34 (m, 5H), 7.39-7.43 (m, 1H), 7.54-7.57 (m, 1H), 7.62-7.71 (m, 2H), 7.72-7.77 (m, 2H ), 7.91 (dd, *J =* 8.0, 1.6 Hz, 1H), 7.95 (d, 1H), 8.08 (dt, *J =* 7.6, 1.2 Hz, 1H), 8.15-8.17 (m, 2H), 8.21 (d, *J =* 8.4 Hz, 1H), 8.66 (d, *J =* 4.4 Hz, 1H).
(2) Synthesis of **(*R,* S)-M-PtLI1:** ligand **(*R, S*)-LI1** (253 mg, 0.53 mmol, 1.0 equiv.) and platinum dichloride (149 mg, 0.56 mmol, 1.05 equiv) were sequentially added into a dry three-necked flask with a magnetic rotor. Nitrogen was purged for three times, benzonitrile (15 mL) was added under nitrogen protection. This mixture was reacted with stirring in an electric heating jacket at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product (*R, S*)- *M -*PtLI1, 120 mg as yellow solid, with a yield of 34%. ¹H NMR (500 MHz, DMSO-*d₆*): δ 3.55-3.59 (m,1H), δ 3.63-3.68 (m, 1H), 6.05 (t, *J =* 6.5 Hz, 1H), 6.16 (d, *J =* 7.0 Hz, 1H), 7.21-7.34 (m,6H), 7.42-7.47 (m, 3H), 7.48-7.53 (m, 1H), 7.77 (d, *J =* 8.5 Hz, 1H), 7.90 (d, *J =* 8.0 Hz, 1H), 8.10 (td, *J =* 8.0, 1.0 Hz, 1H), 8.20-8.23 (m, 2H), 8.27-8.29 (m, 2H), 9.14 (d, *J =* 4.5 Hz, 1H).

**Example 37**, **not in accordance with the present invention and provided for illustrative purposes only:** synthesis of tetradentate cyclometalated platinum (II) complex **(R, S)-*P*-PtLK1** as follow. 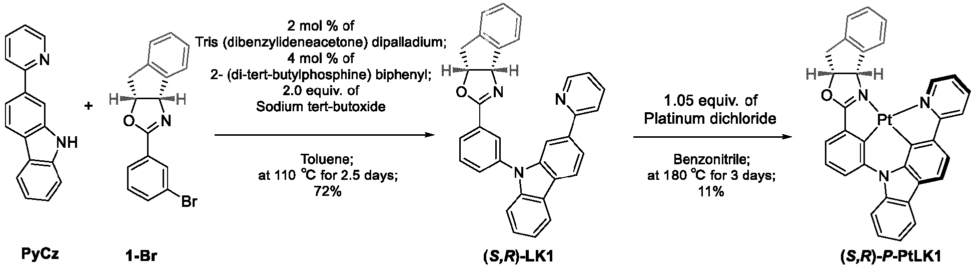
(1) Synthesis of ligand (***S*, *R*)-LK1: PyCz** (303 mg, 1.24 mmol, 1.0 equiv.), **1-Br** (584 mg, 1.86 mmol, 1.5 equiv.), tris (dibenzylideneacetone) dipalladium (23 mg, 0.025 mmol, 2 mol%), 2-(di-tert-butylphosphine) biphenyl (15 mg, 0.050 mmol, 4 mol%) and sodium tert-butoxide (238 mg, 2.48 mmol, 2.0 equivalents) were sequentially added into a dry sealed tube with a magnetic rotor, and then nitrogen was purged for three times and toluene (15 mL) was added under nitrogen protection. This mixture was reacted with stirring in an oil bath at 110 °C for 2.5 days, cooled to the room temperature, and the solvent was distilled off at a reduced pressure. The crude was separated and purified by a silica gel chromatography column with an eluent of petroleum ether/ethyl acetate of 10: 1 to 5: 1 so as to obtain a product **(*S*, *R*)**-LK1, 425 mg as a white solid, with a yield of 72%. ¹H NMR (400 MHz, CDCl₃): δ 3.34-3.39 (m, 1H), 3.47-3.53 (m, 1H), 5.49-5.53 (m,1H), 5.78 (d, *J =* 7.6 Hz, 1H), 7.19-7.22 (m, 1H), 7.27-7.34 (m, 5H), 7.39-7.43 (m, 1H), 7.54-7.57 (m, 1H), 7.62-7.71 (m, 2H), 7.72-7.77 (m, 2H ), 7.91 (dd, *J =* 8.0, 1.6 Hz, 1H), 7.95 (d, 1H), 8.08 (dt, *J =* 7.6, 1.2 Hz, 1H), 8.15-8.17 (m, 2H), 8.21 (d, *J =* 8.4 Hz, 1H), 8.66 (d, *J =* 4.4 Hz, 1H).
(2) Synthesis of **(*S, R*)-*P*-PtLK1:** ligand **(*S*, *R*)-LK1** (258 mg, 0.54 mmol, 1.0 equiv.) and platinum dichloride (152 mg, 0.57 mmol, 1.05 equiv) were sequentially added into a dry three-necked flask with a magnetic rotor. Nitrogen was purged for three times, benzonitrile (15 mL) was added under nitrogen protection. This mixture was reacted with stirring in an electric heating jacket at 180°C for 3 days, cooled to the room temperature, and the solvent was distilled off at a reduced pressure. The crude was separated and purified by a silica gel chromatography column with an eluent of petroleum ether/dichloromethane of 3:1 to 2:1 so as to obtain a product **(*S*, *R*)-*P*-PtLK1,** 40 mg as yellow solid, with a yield of 11%. ¹H NMR (500 MHz, DMSO-*d₆*): δ 3.56-3.60 (m,1H), δ 3.64-3.69 (m,1H), 6.06 (t, *J =* 6.5 Hz, 1H), 6.17 (d, *J =* 7.0 Hz, 1H), 7.22-7.35 (m,6H), 7.43-7.50 (m, 3H), 7.51-7.53 (m, 1H), 7.78 (d, *J =* 8.0 Hz, 1H), 7.91 (d, *J =* 8.0 Hz, 1H), 8.11 (td, *J =* 8.0, 1.5 Hz, 1H), 8.20-8.24 (m, 2H), 8.28-8.30 (m, 2H), 9.15 (d, *J =* 5.5 Hz, 1H).

**Example 38, not in accordance with the present invention and provided for illustrative purposes only:** synthesis of tetradentate cyclometalated palladium (II) complex ***P*-PdLA1** as follow.

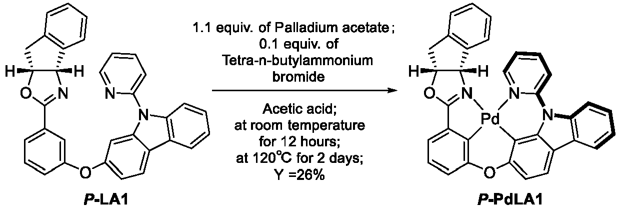

Synthesis of ***P*-PdLA1: *M*-LA1** (200 mg, 0.41 mmol, 1.0 equiv.), palladium acetate (101 mg, 0.45 mmol,1.1 equiv.) and tetra-n-butylammonium bromide (13 mg, 0.041 mmol, 10 mol%) were sequentially added in a 50 mL three-necked flask with a magnetic rotor, and nitrogen was purged for three times, acetic acid (25 mL) was added, which was then bubbled with nitrogen for 30 minutes, stirred at the room temperature for 12 hours, warmed again to 120 °C for reacting for 2 days. After being cooled to the room temperature, the solvent was removed by rotary evaporation at a reduced pressure, and stannous chloride (140 mg, 0.74 mmol, 2.0 equiv.) and dichloromethane (15 mL) were added, stirred for 1 day at the room temperature, washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 8: 1 to 1: 1, so as to obtain 63 mg pale primrose solid, with a yield of 26%. ¹H NMR (500 MHz, DMSO-*d*₆): δ (ppm) 3.46 (d, *J =* 18.5 Hz, 1H), 3.64 (dd, *J =* 18.5 Hz, 6.5 Hz, 1H), 5.97 (t, *J =* 6.5 Hz, 1H), 6.22 (d, *J =* 7.0 Hz, 1H), 7.00 (d, *J =* 7.5 Hz, 1H), 7.11 (t, *J* = 8.0 Hz, 1H), 7.17-7.24 (m, 4H), 7.28 (dd, *J =* 6.5, 2.0 Hz, 1H), 7.35 (d, *J =* 7.5 Hz, 1H), 7.39-7.42 (m, 1H), 7.47-7.51 (m, 2H), 7.92 (d, *J =* 8.5 Hz, 1H), 8.15-8.17 (m, 2H),8.20-8.23 (m, 1H), 8.28 (d, *J =* 8.5 Hz, 1H), 9.34 (dd, *J =* 6.0, 2.0 Hz, 1H).

**Example 39, not in accordance with the present invention and provided for illustrative purposes only:** synthesis of tetradentate cyclometalated palladium (II) complex ***M*-PdLA1** as follow.

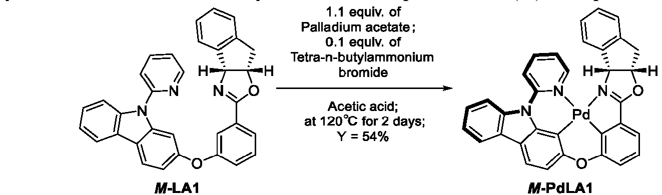

Synthesis of P-PdLA1: M-LA1 (200 mg, 0.41 mmol, 1.0 equiv.), palladium acetate (101 mg, 0.45 mmol,1.1 equiv.) and tetra-n-butylammonium bromide (13 mg, 0.041 mmol, 10 mol%) were sequentially added in a 50 mL three-necked flask with a magnetic rotor, and nitrogen was purged for three times, acetic acid (25 mL) was added, which was then bubbled with nitrogen for 30 minutes, warmed again to 120°C for reacting for 2 days. After being cooled to the room temperature, the solvent was removed by rotary evaporation at a reduced pressure, this mixture is washed with water and extracted three times. Then organic phases were combined, dried with anhydrous sodium sulfate and filtered, mixed with silica gel, loaded in a dry manner, and separated and purified by column chromatography, with an eluant of petroleum ether: dichloromethane of 2: 1 to 1: 1, so as to obtain 133 mg white solid, with a yield of 54%. ¹H NMR (500 MHz, CDCl₃): δ (ppm) 3.51-3.60 (m, 2H), 5.78-5.81 (m, 1H), 5.87 (d, *J =* 7.0 Hz, 1H), 7.04-7.08 (m, 2H), 7.12 (d, *J =* 7.5 Hz, 1H), 7.16-7.19 (m, 2H), 7.24 (d, *J* = 8.0 Hz, 1H), 7.26-7.27 (m, 1H), 7.29-7.31 (m, 2H), 7.34-7.42 (m, 2H), 7.82-7.86 (m, 2H), 7.99 (d, *J =* 8.0 Hz, 1H), 8.04 (dd, *J =* 7.5, 1.0 Hz, 1H), 8.17 (d, *J =* 8.5 Hz, 1H), 9.20 (dd, *J =* 6.0, 2.0 Hz, 1H).

**Example 40, not in accordance with the present invention and provided for illustrative purposes only:** synthesis of tetradentate cyclometalated platinum (II) complex ***P*-PtL1** as follow. 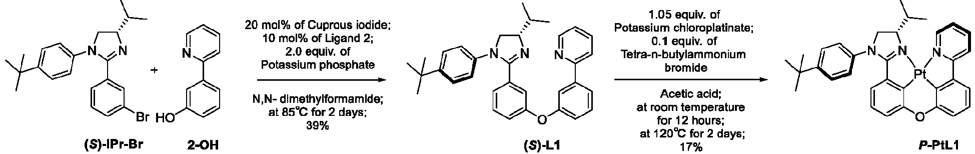
(1) Synthesis of ligand **(*S*)-L1: (*S*)-iPr-Br** (1.19 g, 2.99 mmol, 1.2 equivalent), 2-OH (426 g, 2.49 mmol, 1.0 equivalent) and cuprous iodide (95 mg, 0.50 mmol, 20 mol%), ligand 2 (86 mg, 0.25 mmol, 10 mol%) and potassium phosphate (1.06 g, 4.98 mmol, 2.0 equiv.) were sequentially added into a dry sealed tube with a magnetic rotor. Nitrogen was purged for three times, and N, N-dimethylformamide (25 mL) was added under nitrogen protection. The sealed tube was placed in an oil bath at 85°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*S*) L1**, 475 mg as a white solid, with a yield of 39%. ¹H NMR (400 MHz, DMSO-*d₆*): δ 0.86 (d, *J =* 6.4 Hz, 3H), 0.94 (d, *J* = 6.4 Hz, 3H), 1.17 (s, 9H), 1.70-1.78 (m, 1H), 3.54-3.59 (m, 1H), 3.89-3.95 (m, 1H), 3.97-4.03 (m, 1H), 6.70 (d, *J =* 8.8 Hz, 2H), 6.87-6.90 (m, 1H), 7.00 (t, *J =* 2.0 Hz, 1H), 7.11-7.14 (m, 1H), 7.17-7.21 (m, 2H), 7.27 (d, *J =* 7.6 Hz, 1H), 7.35-7.44 (m, 3H), 7.69 (t, *J =* 1.6 Hz, 1H), 7.83-7.90 (m, 2H), 7.95 (d, *J =* 8.0 Hz, 1H).
(2) Synthesis of ***P*-PtL1: (*S*)-L1** (431 mg, 0.88 mmol, 1.0 equiv.), potassium chloroplatinate (346 mg, 0.92 mmol, 1.05 equiv.), tetra-n-butylammonium bromide (28 mg, 0.088 mmol, 0.1 equiv.) were sequentially added into a 100 mL dry three-necked flask with a magnetic rotor and a condenser tube, followed by nitrogen purge for three times and addition of acetic acid (52 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure. The crude was separated and purified by a silica gel chromatography column with an eluent of petroleum ether/dichloromethane of 1:1 so as to obtain a product *P*-PtL1, 102 mg as red solid, with a yield of 17%. ¹H NMR (400 MHz, DMSO-*d₆*): δ 0.86 (d, *J =* 6.8 Hz, 3H), 1.03 (d, *J =* 6.8 Hz, 3H), 1.33 (s, 9H), 4.03-4.06 (m, 1H), 4.30-4.36 (m, 1H), 4.46-4.50 (m, 1H), 6.36-6.38 (m, 1H), 6.83 (t, *J =* 7.6 Hz, 1H), 6.99-7.03 (m, 2H), 7.17 (t, *J =* 7.6 Hz, 1H), 7.29-7.31 (m, 2H), 7.52-7.55(m, 3H), 7.62 (d, *J* = 7.2 Hz, 1H), 8.07-8.11(m, 1H), 8.20 (d, *J =* 7.6 Hz, 1H), 8.77 (d, *J =* 4.8 Hz, 1H).

**Example 41, not in accordance with the present invention and provided for illustrative purposes only:** synthesis of tetradentate cyclometalated platinum (II) complex **M-PtL1** as follow. 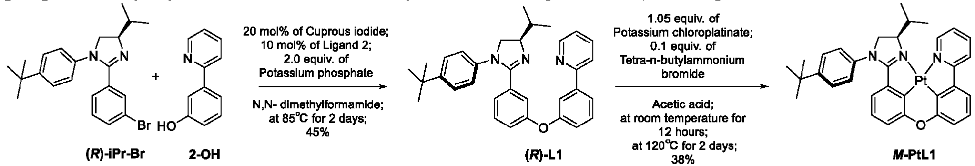
(1) Synthesis of ligand **(*R*)-L1: (*R*)-iPr-Br** (1.19 g, 2.99 mmol, 1.2 equivalent), 2-OH (426 g, 2.49 mmol, 1.0 equivalent) and cuprous iodide (95 mg, 0.50 mmol, 20 mol%), ligand 2 (86 mg, 0.25 mmol, 10 mol%) and potassium phosphate (1.06 g, 4.98 mmol, 2.0 equiv.) were sequentially added into a dry sealed tube with a magnetic rotor. Nitrogen was purged for three times, and N, N-dimethylformamide (25 mL) was added under nitrogen protection. The sealed tube was placed in an oil bath at 85 °C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*R*) L1**, 549 mg as a white solid, with a yield of 45%.
(2) Synthesis of *M*-PtL1: **L2** (150 mg, 0.31 mmol, 1.0 equiv.), potassium chloroplatinate (124 mg, 0.33 mmol, 1.05 equiv.), tetra-n-butylammonium bromide (10 mg, 0.031 mmol, 0.1 equiv.) were sequentially added into a 50 mL dry three-necked flask with a magnetic rotor and a condenser tube, followed by nitrogen purge for three times and addition of acetic acid (19 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure. The crude was separated and purified by a silica gel chromatography column with an eluent of petroleum ether/dichloromethane of 1:1 so as to obtain a product *M*-PtL1, 81 mg as red solid, with a yield of 38%. ¹H NMR (400 MHz, DMSO-*d₆*): δ 0.85 (d, *J* = 6.8 Hz, 3H), 1.03 (d, *J* = 6.8 Hz, 3H), 1.34 (s, 9H), 4.03-4.08 (m, 1H), 4.30-4.36 (m, 1H), 4.46-4.50 (m, 1H), 6.36-6.38 (m, 1H), 6.83 (t, *J =* 7.6 Hz, 1H), 6.99-7.03 (m, 2H), 7.16 (t, *J* = 7.6 Hz, 1H), 7.29-7.31 (m, 2H), 7.52-7.55(m, 3H), 7.62 (d, *J* = 7.2 Hz, 1H), 8.07-8.11(m, 1H), 8.20 (d, *J =* 7.6 Hz, 1H), 8.78 (d, *J* = 4.8 Hz, 1H).

**Example 42, not in accordance with the present invention and provided for illustrative purposes only:** synthesis of tetradentate cyclometalated platinum (II) complex ***P*-PtL2** as follow. 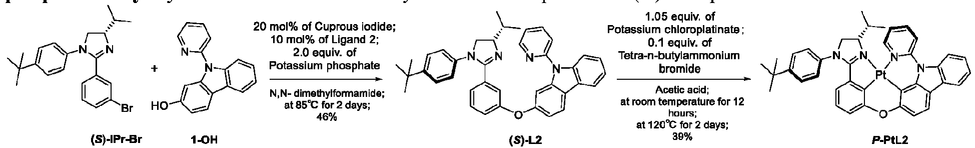
(1) Synthesis of ligand **(*S*)-L2: (*S*)-iPr-Br** (891 mg, 2.03 mmol, 1.2 equivalent), 1-OH (440 g, 1.69 mmol, 1.0 equivalent) and cuprous iodide (65 mg, 0.34 mmol, 20 mol%), ligand 2 (59 mg, 0.17 mmol, 10 mol%) and potassium phosphate (717 mg, 3.38 mmol, 2.0 equiv.) were sequentially added into a dry sealed tube with a magnetic rotor. Nitrogen was purged for three times, and N, N-dimethylformamide (20 mL) was added under nitrogen protection. The sealed tube was placed in an oil bath at 85°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*S*) L2**, 450mg as a white solid, with a yield of 46%. ¹H NMR (500 MHz, DMSO-*d₆*): δ 0.89-0.92 (m, 6H), 1.18 (s, 9H), 1.90-1.97 (m, 1H), 3.09-3.14 (m, 1H), 3.16-3.21 (m, 1H), 3.97-4.03 (m, 1H), 5.24 (t, *J =* 6.0 Hz, 1H), 6.48-6.51 (m, 2H), 7.03-7.06 (m, 3H), 7.17-7.19 (m, 1H), 7.33-7.36 (m, 1H), 7.43-7.47 (m, 3H), 7.49 (d, *J =* 2.0 Hz, 1H), 7.52-7.53 (m, 1H), 7.61 (dt, *J =* 8.0, 1.0 Hz, 1H), 7.77 (d, *J =* 8.0 Hz, 2H), 8.06-8.09 (m, 1H), 8.13 (d, *J =* 9.0 Hz, 1H), 8.23 (d, *J =* 7.5 Hz, 1H), 8.27 (d, *J =* 8.5Hz, 1H), 8.67-8.68 (m, 1H).
(2) Synthesis of ***P*-PtL3:** (*S*)-L2 (249 mg, 0.43 mmol, 1.0 equiv.), potassium chloroplatinate (169 mg, 0.45 mmol, 1.05 equiv.), tetra-n-butylammonium bromide (14 mg, 0.043 mmol, 0.1 equiv.) were sequentially added into a 50 mL dry three-necked flask with a magnetic rotor and a condenser tube, followed by nitrogen purge for three times and addition of acetic acid (26 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure. The crude was separated and purified by a silica gel chromatography column with an eluent of petroleum ether/dichloromethane of 1:1 so as to obtain a product *P*-PtL3, 129 mg as red solid, with a yield of 39%. ¹H NMR (600 MHz, DMSO-*d₆*): δ 0.76 (d, *J =* 7.2 Hz, 3H), 0.82 (d, *J =* 7.2 Hz, 3H), 1.30 (s, 9H), 1.90-1.95 (m, 1H), 3.83 (dd, *J =* 9.6, 3.6 Hz, 1H), 4.49-4.52 (m, 1H), 4.60-4.63 (m, 1H), 6.43 (dd, *J* = 7.8*,* 1.2 Hz, 1H), 6.85 (t, *J =* 7.8 Hz, 1H), 6.99 (dd, *J =* 7.8, 0.6 Hz, 1H), 7.12-7.25 (m, 3H), 7.33-7.36 (m, 1H), 7.38-7.40 (m, 1H), 7.44-7.49 (m, 3H), 7.82 (d, *J =* 8.4 Hz, 1H), 8.11-8.14 (m, 2H), 8.17-8.20 (m, 1H), 8.23 (d, *J =* 7.8 Hz, 1H), 9.26-9.27 (m, 1H).

**Example 43, not in accordance with the present invention and provided for illustrative purposes only:** synthesis of tetradentate cyclometalated platinum (II) complex **M-PtL2** as follow. 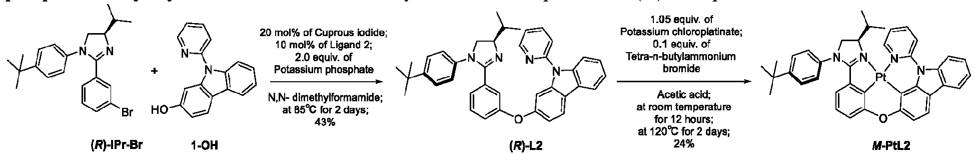
(1) Synthesis of ligand **(*R*)**-L2: **(*R*)**-iPr-**Br** (891 mg, 2.03 mmol, 1.2 equivalent), 1-OH (440 g, 1.69 mmol, 1.0 equivalent) and cuprous iodide (65 mg, 0.34 mmol, 20 mol%), ligand 2 (59 mg, 0.17 mmol, 10 mol%) and potassium phosphate (717 mg, 3.38 mmol, 2.0 equiv.) were sequentially added into a dry sealed tube with a magnetic rotor. Nitrogen was purged for three times, and N, N-dimethylformamide (20 mL) was added under nitrogen protection. The sealed tube was placed in an oil bath at 85°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*R*) L**2, 421mg as a white solid, with a yield of 43%. ¹H NMR (500 MHz, DMSO-*d₆*): δ 0.89-0.92 (m, 6H), 1.18 (s, 9H), 1.90-1.97 (m, 1H), 3.09-3.14 (m, 1H), 3.16-3.21 (m, 1H), 3.97-4.03 (m, 1H), 5.24 (t, *J =* 6.0 Hz, 1H), 6.48-6.51 (m, 2H), 7.03-7.06 (m, 3H), 7.17-7.19 (m, 1H), 7.33-7.36 (m, 1H), 7.43-7.47 (m, 3H), 7.49 (d, *J =* 2.0 Hz, 1H), 7.52-7.53 (m, 1H), 7.61 (dt, *J =* 8.0, 1.0 Hz, 1H), 7.77 (d, *J =* 8.0 Hz, 2H), 8.06-8.09 (m, 1H), 8.13 (d, *J =* 9.0 Hz, 1H), 8.23 (d, *J =* 7.5 Hz, 1H), 8.27 (d, *J =* 8.5Hz, 1H), 8.67-8.68 (m, 1H).
(2) Synthesis of ***M*-PtL2: (*R*)-L2** (150 mg, 0.26 mmol, 1.0 equiv.), potassium chloroplatinate (102 mg, 0.27 mmol, 1.05 equiv.), tetra-n-butylammonium bromide (8 mg, 0.026 mmol, 0.1 equiv.) were sequentially added into a 50 mL dry three-necked flask with a magnetic rotor and a condenser tube, followed by nitrogen purge for three times and addition of acetic acid (16 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure. The crude was separated and purified by a silica gel chromatography column with an eluent of petroleum ether/dichloromethane of 1:1 so as to obtain a product M-PtL2, 48 mg as red solid, with a yield of 24%. ¹H NMR (600 MHz, DMSO-*d₆*): δ 0.78 (d, *J =* 6.6 Hz, 3H), 0.83 (d, *J =* 6.6 Hz, 3H), 1.32 (s, 9H), 1.92-1.95 (m, 1H), 3.87 (dd, *J* =10.2, 4.2 Hz, 1H), 4.50-4.53 (m, 1H), 4.62-4.65 (m, 1H), 6.43 (dd, *J* = 7.8*,* 1.2 Hz, 1H), 6.85 (t, *J* = 7.8 Hz, 1H), 6.99 (dd, *J* = 7.8, 0.6 Hz, 1H), 7.12-7.26 (m, 3H), 7.34-7.37 (m, 1H), 7.38-7.41 (m, 1H), 7.44-7.51 (m, 3H), 7.82 (d, *J =* 7.8 Hz, 1H), 8.11-8.14 (m, 2H), 8.17-8.20 (m, 1H), 8.24 (d, *J =* 8.4 Hz, 1H), 9.27-9.28 (m, 1H).

**Example 44, not in accordance with the present invention and provided for illustrative purposes only:** synthesis of tetradentate cyclometalated platinum (II) complex ***P*-PtL3** as follow. 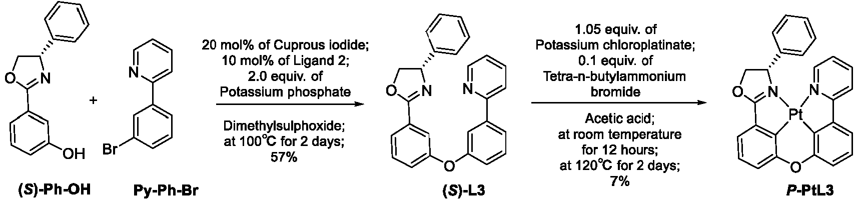
(1) Synthesis of ligand(*S*)-L3: **Py-Ph-Br** (440 mg, 1.88 mmol, 1.5 equivalent), **(*S*)-Ph-OH** (300 g, 1.25 mmol, 1.0 equivalent) and cuprous iodide (48 mg, 0.25 mmol, 20 mol%), ligand 2 (45 mg, 0.13 mmol, 10 mol%) and potassium phosphate (531 mg, 2.50 mmol, 2.0 equiv.) were sequentially added into a dry sealed tube with a magnetic rotor. Nitrogen was purged for three times, and dimethyl sulfoxide (20 mL) was added under nitrogen protection. The sealed tube was placed in an oil bath at 100°C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*S*)-L3,** 280mg as a white solid, with a yield of 57%. ¹H NMR (400 MHz, DMSO-*d₆*): δ 4.18 (t, *J =* 8.4 Hz, 1H), 4.82 (t, *J =* 9.6 Hz, 1H), 5.37 (t, *J =* 8.8 Hz, 1H), 7.17 (d, *J =* 8.0 Hz, 1H), 7.26-7.38 (m, 6H), 7.49-7.58 (m, 3H), 7.73 (d, *J =* 7.6 Hz, 1H), 7.80 (s, 1H), 7.86-7.93 (m, 2H), 8.00 (d, *J =* 8.0 Hz, 1H), 8.64 (d, *J =* 4.8 Hz, 1H).
(2) Synthesis of ***P*-PtL3:** (*S*)-**L3** (173 mg, 0.44 mmol, 1.0 equiv.), potassium chloroplatinate (173 mg, 0.46 mmol, 1.05 equiv.), tetra-n-butylammonium bromide (14 mg, 0.043 mmol, 0.1 equiv.) were sequentially added into a 50 mL dry three-necked flask with a magnetic rotor and a condenser tube, followed by nitrogen purge for three times and addition of acetic acid (26 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure. The crude was separated and purified by a silica gel chromatography column with an eluent of petroleum ether/dichloromethane of 1:1 so as to obtain a product *P*-PtL3, 18 mg as red solid, with a yield of 7%. ¹H NMR (400 MHz, DMSO-*d₆*): δ 4.65 (t, *J =* 7.2 Hz, 1H), 5.35 (t, *J =* 9.6 Hz, 1H), 5.81 (t, *J =* 7.2 Hz, 1H), 7.06-7.10 (m, 2H), 7.16-7.20 (m, 1H), 7.25 (d, *J* = 2.4 Hz, 2H), 7.31-7.42 (m, 2H), 7.40 (t, *J* = 7.6 Hz, 2H), 7.53-7.59 (m, 3H), 7.92 (t, *J =* 8.0 Hz, 1H), 8.05-8.09 (m, 2H).

**Example 45, not in accordance with the present invention and provided for illustrative purposes only:** synthesis of tetradentate cyclometalated platinum (II) complex **M-PtL3** as follow. 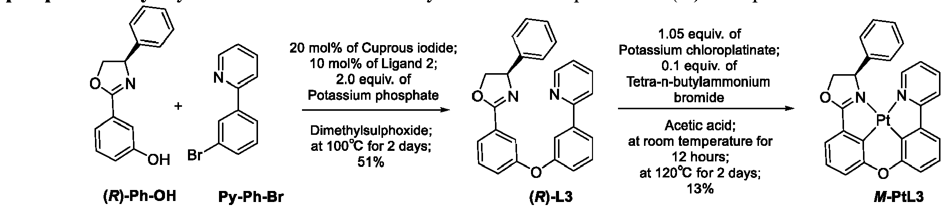
(1) Synthesis of ligand **(*R*)-L3: Py-Ph-Br** (735 mg, 3.14 mmol, 1.5 equivalent), **(*R*)-Ph-OH** (500 g, 2.09 mmol, 1.0 equivalent) and cuprous iodide (80 mg, 0.42 mmol, 20 mol%), ligand 2 (72 mg, 0.21 mmol, 10 mol%) and potassium phosphate (887 mg, 4.18 mmol, 2.0 equiv.) were sequentially added into a dry sealed tube with a magnetic rotor. Nitrogen was purged for three times, and dimethyl sulfoxide (20 mL) was added under nitrogen protection. The sealed tube was placed in an oil bath at 100 °C, stirred for reacting for 2 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 10: 1 to 5: 1 so as to obtain a product **(*R*) L3,** 420mg as a white solid, with a yield of 51%. ¹H NMR (400 MHz, CDCl₃): δ 4.26 (t, *J =* 8.4 Hz, 1H), 4.78 (dd, *J* = 10*,* 8.4 Hz, 1H), 5.37 (dd, *J =* 10, 8.4 Hz, 1H), 7.07-7.09 (m, 1H), 7.20-7.25 (m, 2H), 7.27-7.30 (m, 3H), 7.33-7.37 (m, 2H), 7.39-7.47 (m, 2H), 7.68-7.77 (m, 5H), 7.81 (dt, *J =* 8.0, 1.2 Hz, 1H), 8.67-7.69 (m, 1H).
(2) Synthesis of *M*-PtL3: (*R*)-L3 (200 mg, 0.51 mmol, 1.0 equiv.), potassium chloroplatinate (224 mg, 0.54 mmol, 1.05 equiv.), tetra-n-butylammonium bromide (16 mg, 0.051 mmol, 0.1 equiv.) were sequentially added into a 50 mL dry three-necked flask with a magnetic rotor and a condenser tube, followed by nitrogen purge for three times and addition of acetic acid (31 mL) pre-purged with nitrogen. After a reaction solution was bubbled with nitrogen for 30 minutes, it was stirred at the room temperature for 12 hours, and then was reacted with stirring at 120°C for 2 days, cooled to the room temperature, and solvent was distilled off at a reduced pressure. The crude was separated and purified by a silica gel chromatography column with an eluent of petroleum ether/dichloromethane of 1:1 so as to obtain a product M-PtL3, 40mg as red solid, with a yield of 13%. ¹H NMR (500 MHz, DMSO-*d₆*): δ 4.66 (dd, *J =* 8.5, 6.5 Hz, 1H), 5.35 (dd, *J =* 10, 8.5 Hz, 1H), 5.82 (dd, *J =* 10, 6.0 Hz, 1H), 7.06-7.10 (m, 2H), 7.23-7.27 (m, 2H), 7.30-7.33 (m, 2H), 7.40 (t, *J =* 8.0 Hz, 2H), 7.54-7.60 (m, 3H), 7.93 (td, *J =* 8.0, 2.0 Hz, 1H), 8.05-8.10 (m, 2H).

**Example 46, not in accordance with the present invention and provided for illustrative purposes only:** synthesis of tetradentate cyclometalated platinum (II) complex ***P*-PtOO** as follow. 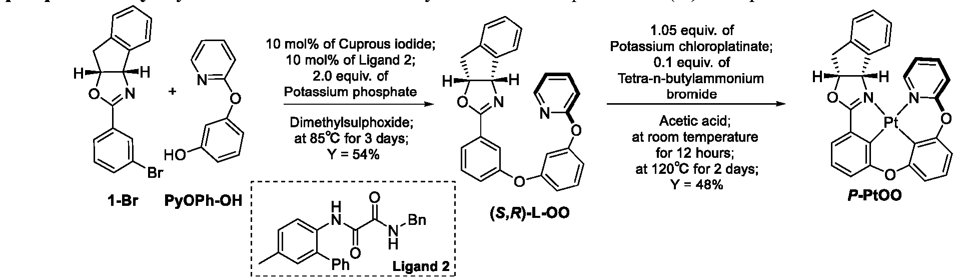
(1) Synthesis of ligand **(*S, R*)-L-OO:** 1-Br (800 mg, 2.55 mmol, 1.2 equivalent), **PyOPh-OH** (512 mg, 2.12 mmol, 1.0 equivalent) and cuprous iodide (40 mg, 0.21 mmol, 10 mol%), ligand 2 (69 mg, 0.21 mmol, 10 mol%) and potassium phosphate (900 mg, 4.24 mmol, 2.0 equiv.) were sequentially added into a dry sealed tube with a magnetic rotor. Nitrogen was purged for three time, and dimethyl sulfoxide (20 mL) was added under nitrogen protection. The sealed tube was placed in an oil bath at 85 °C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 15: 1 to 5: 1 so as to obtain the ligand **(*S, R*)-L-OO,** 480 mg as black solid, with a yield of 54%. ¹H NMR (500 MHz, DMSO-*d₆*): *δ* (ppm) 3.22 (d, *J =* 18.0 Hz, 1H), 3.60 (dd, *J =* 18.0, 7.0 Hz, 1H), 5.51 (ddd, *J =* 8.0, 7.0, 1.5 Hz, 1H), 5.69 (d, *J =* 8.0 Hz, 1H), 6.80 (t, *J =* 2.5 Hz, 1H), 6.85 (ddd, *J =* 8.5, 2.5, 0.5 Hz, 1H), 6.93 (ddd, *J =* 8.5, 2.0, 0.5 Hz, 1H), 7.04 (d, *J =* 8.0 Hz, 1H), 7.12 (ddd, *J =* 7.0, 5.0, 1.0 Hz, 1H), 7.22-7.30 (m, 4H), 7.40-7.44 (m, 3H),7.48 (t, *J =* 8.0 Hz, 1H), 7.62 (dt, *J =* 7.5, 1.0 Hz, 1H), 7.84 (ddd, *J =* 8.0, 7.0, 2.0 Hz, 1H), 8.14 (ddd, *J =* 5.0, 2.0, 0.5 Hz, 1H). ¹³C NMR (125 MHz, CDCl₃): *δ* (ppm) 39.73, 83.27, 111.61, 111.72, 114.53, 115.77, 118.72, 119.16, 122.26, 123.66, 125.25, 125.60, 127.44, 128.47, 129.66, 130.33, 139.45, 139.67, 141.80, 147.73, 155.39, 156.53, 158.20, 163.24, 163.36. HR (ESI): calcd for C₂₇H₂₁N₂O₅ [M+H]⁺ 421.15, found 421.15.
(2) Synthesis of ***P*-PtOO:** (***S*, *R*)-L-OO** (200 mg, 0.48 mmol, 1.0 equiv.), potassium chloroplatinate (208 mg, 0.50 mmol, 1.05 equiv.), tetra-n-butylammonium bromide (16 mg, 0.048 mmol, 0.1 equiv.) were sequentially added into a 50 mL dry three-necked flask with a magnetic rotor and a condenser tube, followed by nitrogen purge for three times and addition of acetic acid (15 mL) pre-purged with nitrogen. After a reaction solution was bubbled with nitrogen for 30 minutes, it was stirred at the room temperature for 12 hours, and then was reacted with stirring at 120 °C for 2 days, cooled to the room temperature, and solvent was distilled off at a reduced pressure, dichloromethane was dissolved, water was added for washing, an aqueous layer was extracted three times with dichloromethane, organic phases were combined, and solvent was distilled off at a reduced pressure. The crude was separated and purified by a silica gel chromatography column with an eluent of petroleum ether/dichloromethane of 3: 1 to 1: 1 so as to obtain a product *P*-PtOO, 140 mg as pale primrose solid, with a yield of 48%. ¹H NMR (500 MHz, DMSO-*d*₆): *δ* (ppm) 3.48 (d, *J =* 18.5 Hz, 1H), 3.61 (dd, *J =* 18.5, 6.5 Hz, 1H), 5.98 (t, *J* = 7.0 Hz, 1H), 6.10 (d, *J =* 7.0 Hz, 1H), 6.44 (d, *J =* 7.5 Hz, 1H), 6.89 (dq, *J* = 7.5, 1.0 Hz, 2H), 7.03-7.10 (m, 3H), 7.12 (t, *J =* 7.5 Hz, 1H), 7.18 (dd, *J =* 7.0, 1.0 Hz, 1H), 7.23 (t, *J =* 7.5 Hz, 1H), 7.35 (d, *J =* 8.0 Hz, 1H), 7.40 (ddd, *J =* 7.0, 5.5, 1.0 Hz, 1H), 7.22-7.30 (m, 4H), 7.61 (dd, *J =* 8.5, 0.5 Hz, 1H), 8.25 (ddd, *J* = 8.0*,* 7.0, 2.0 Hz, 1H), 9.12 (dd, *J =* 5.5, 1.5 Hz, 1H). HR (ESI): calcd for C₂₇H₁₉N₂O₃Pt [M+H]⁺ 614.10, found 614.10.

**Example 47, not in accordance with the present invention and provided for illustrative purposes only:** synthesis of tetradentate cyclometalated platinum (II) complex ***M*-PtOO** as follow. 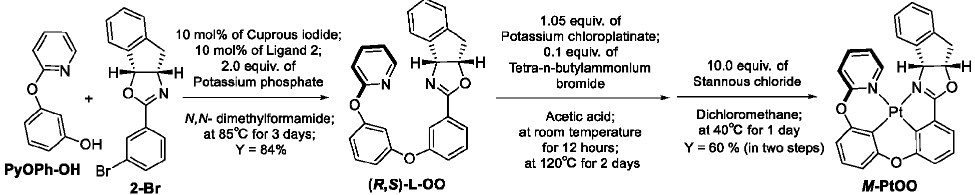
(1) Synthesis of ligand ***(R, S)-L-OO:* 2-Br** (800 mg, 2.55 mmol, 1.2 equivalent), **PyOPh-OH** (512 mg, 2.12 mmol, 1.0 equivalent) and cuprous iodide (40 mg, 0.21 mmol, 10 mol%), ligand 2 (69 mg, 0.21 mmol, 10 mol%) and potassium phosphate (900 mg, 4.24 mmol, 2.0 equiv.) were sequentially added into a dry sealed tube with a magnetic rotor. Nitrogen was purged for three times, and N, N- dimethylformamide (15 mL) was added under nitrogen protection. The sealed tube was placed in an oil bath at 85°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/ethyl acetate of 15: 1 to 5: 1 so as to obtain the ligand **(*R,* S)-L-OO,** 750 mg as yellow brown solid, with a yield of 84%. ¹³C NMR (125 MHz, CDCl₃): *δ* (ppm) 39.74, 83.28, 111.61, 111.73, 114.53, 115.77, 118.73, 119.19, 122.28, 123.68, 125.26, 125.62, 127.46, 128.48, 129.67, 130.33, 139.46, 139.68, 141.82, 147.75, 155.40, 156.53, 158.23, 163.26, 163.37. HR (ESI): calcd for C₂₇H₂₁N₂O₃ [M+H]⁺ 421.15, found 421.15.
(2) Synthesis of ***M*-PtOO: (*R, S*)-L-OO** (210 mg, 0.50 mmol, 1.0 equiv.), potassium chloroplatinate (220 mg, 0.53 mmol, 1.05 equiv.), tetra-n-butylammonium bromide (16 mg, 0.050 mmol, 0.1 equiv.) were sequentially added into a 50 mL dry three-necked flask with a magnetic rotor and a condenser tube, followed by nitrogen purge for three times and addition of acetic acid (15 mL) pre-purged with nitrogen. After a reaction solution was bubbled with nitrogen for 30 minutes, it was stirred at the room temperature for 12 hours, and then was reacted with stirring at 120°C for 2 days, cooled to the room temperature, and solvent was distilled off at a reduced pressure, and stannous chloride dihydrate (1.10 g, 5.00 mmol, 10.0 equiv.) and dichloromethane (15 mL) were added and stirred at 40°C for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 3: 1 to 1: 1 so as to obtain a product ***M* -PtOO,** 185 mg as pale primrose solid, with a yield of 60%. ¹H NMR (500 MHz, DMSO-*d*₆): *δ* (ppm) 3.48 (d, *J =* 18.5 Hz, 1H), 3.61 (dd, *J =* 18.5, 6.5 Hz, 1H), 5.98 (t, *J =* 7.0 Hz, 1H), 6.11 (d, *J =* 7.0 Hz, 1H), 6.44 (d, *J =* 7.5 Hz, 1H), 6.89 (dq, *J =* 7.5, 1.5 Hz, 2H), 7.03-7.14 (m, 4H), 7.18 (dd, *J =* 7.5, 1.5 Hz, 1H), 7.22-7.25 (m, 1H), 7.35 (d, *J =* 7.5 Hz, 1H), 7.40 (ddd, *J =* 7.0, 6.0, 1.5 Hz, 1H), 7.61 (dd, *J* = 8.0, 0.5 Hz, 1H), 8.26 (ddd, *J =* 8.5, 7.0, 1.5 Hz, 1H), 9.13 (dd, *J* = 5.5, 1.5 Hz, 1H). HR (ESI): calcd for C₂₇H₁₉N₂O₃Pt [M+H]⁺ 614.10, found 614.10.

**Example 48, not in accordance with the present invention and provided for illustrative purposes only:** synthesis of tetradentate cyclometalated platinum (II) complex ***P*-PtOC** as follow. 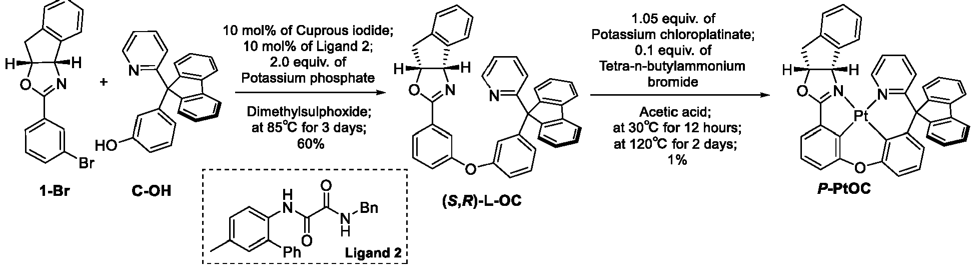
(1) Synthesis of ligand **(*S, R*)-L-OC:** 1-Br (337 mg, 1.07 mmol, 1.2 equivalent), **C-OH** (300 mg, 0.89 mmol, 1.0 equivalent) and cuprous iodide (17 mg, 0.09 mmol, 10 mol%), ligand 2 (29 mg, 0.09 mmol, 10 mol%) and potassium phosphate (378 mg, 1.78 mmol, 2.0 equiv.) were sequentially added into a dry sealed tube with a magnetic rotor. Nitrogen was purged for three time, and dimethyl sulfoxide (15 mL) was added under nitrogen protection. The sealed tube was placed in an oil bath at 85 °C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/dichloromethane of 3: 1 to 1: 1 so as to obtain the ligand **(*S*, *R*)-L-OC,** 305 mg as light brown solid, with a yield of 60%. ¹H NMR (500 MHz, DMSO-*d*₆): *δ* (ppm) 3.23 (d, *J =* 17.5 Hz, 1H), 3.50 (dd, *J =* 18.0, 6.5 Hz, 1H), 5.52 (ddd, *J =* 8.0, 7.0, 1.5 Hz, 1H), 5.71 (d, *J =* 8.0 Hz, 1H), 6.57 (t, *J =* 2.0 Hz, 1H), 6.74 (dq, *J =* 8.0, 1.0 Hz, 1H), 6.84 (ddd, *J =* 8.0, 2.0, 0.5 Hz, 1H), 7.02 (d, *J =* 8.0 Hz, 1H), 7.13 (ddd, *J =* 8.0, 2.5, 1.0 Hz, 1H), 7.18 (ddd, *J =* 7.5, 5.0, 1.0 Hz, 1H), 7.22-7.31 (m, 5H), 7.30-7.31 (m, 2H), 7.37-7.46 (m, 4H), 7.51-7.60 (m, 4H), 7.90 (d, *J =* 8.0 Hz, 2H), 8.53 (ddd, *J =* 4.5, 2.0, 1.0 Hz, 1H). ¹³C NMR (125 MHz, CDCl₃): *δ* (ppm) 39.74, 53.38, 66.98, 83.24, 116.52, 118.59, 119.00, 120.12, 121.30, 121.71, 121.74, 122.80, 123.12, 125.25, 125.65, 126.67, 127.46, 127.73, 127.80, 128.47, 129.42, 129.46, 129.47, 136.23, 139.68, 140.41, 141.88, 148.08, 149.43, 149.68, 156.66, 156.90, 163.35, 163.47. HR (ESI): calcd for C₄₀H₂₉N₂O₂ [M+H]⁺ 569.22, found 569.22.
(2) Synthesis of ***M-*PtOC: (*S, R*)-L-OC** (200 mg, 0.35 mmol, 1.0 equiv.), potassium chloroplatinate (154 mg, 0.37 mmol, 1.05 equiv.), tetra-n-butylammonium bromide (11 mg, 0.035 mmol, 0.1 equiv.) were sequentially added into a 50 mL dry three-necked flask with a magnetic rotor and a condenser tube, followed by nitrogen purge for three times and addition of acetic acid (15 mL) pre-purged with nitrogen. After a reaction solution was bubbled with nitrogen for 30 minutes, it was stirred at 30°C for 12 hours, and then was reacted with stirring at 120°C for 2 days, cooled to the room temperature, and solvent was distilled off at a reduced pressure, dichloromethane was dissolved, water was added for washing, an aqueous layer was extracted three times with dichloromethane, organic phases were combined, and solvent was distilled off at a reduced pressure. The crude was separated and purified by a silica gel chromatography column with an eluent of petroleum ether/dichloromethane of 3: 1 to 1: 1 so as to obtain a product ***M*-PtOC,** 57 mg as pale primrose solid, with a yield of 21%. ¹H NMR (500 MHz, DMSO-*d*₆): *δ* (ppm) 3.51 (d, *J =* 18.5 Hz, 1H), 3.60 (dd, *J* = 18.5, 5.5 Hz, 1H), 5.73-5.75 (m, 1H), 5.98-6.02 (m, 2H), 6.26 (dd, *J* = 8.0, 1.0 Hz, 1H), 6.38 (t, *J* = 7.0 Hz, 1H), 6.63-6.67 (m, 2H), 6.88-6.90 (m, 2H), 7.05 (dd, *J =* 7.5, 1.0 Hz, 1H), 7.13-7.19 (m, 2H), 7.23 (dd, *J =* 7.0, 1.0 Hz, 1H), 7.27 (td, *J =* 7.5, 1.0 Hz, 1H), 7.34 (d, *J =* 7.5 Hz, 1H), 7.39 (d, *J* = 7.5 Hz, 1H), 7.52-7.58 (m, 2H), 7.66 (td, *J =* 7.5, 1.0 Hz, 1H), 7.86-7.90 (m, 2H), 8.12 (d, *J =* 7.5 Hz, 1H), 9.23 (d, *J =* 8.0 Hz, 1H), 9.52 (dd, *J =* 5.5, 1.5 Hz, 1H). HR (ESI): calcd for C₄₀H₂₇N₂O₂Pt [M+H]⁺ 762.17, found 762.17.

**Example 49, not in accordance with the present invention and provided for illustrative purposes only:** synthesis of tetradentate cyclometalated platinum (II) complex ***M*-PtOC** as follow. 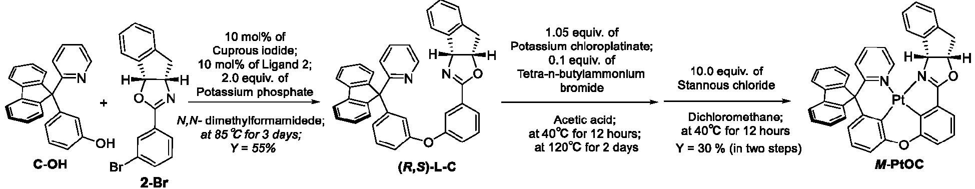
(1) Synthesis of ligand ***(R, S)-L-OC:* 2-Br** (337 mg, 1.07 mmol, 1.2 equivalent), **C-OH** (300 mg, 0.89 mmol, 1.0 equivalent) and cuprous iodide (17 mg, 0.09 mmol, 10 mol%), ligand 2 (29 mg, 0.09 mmol, 10 mol%) and potassium phosphate (378 mg, 1.78 mmol, 2.0 equiv.) were sequentially added into a dry sealed tube with a magnetic rotor. Nitrogen was purged for three times, and N, N-dimethylformamide (10 mL) was added under nitrogen protection. The sealed tube was placed in an oil bath at 85°C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/dichloromethane of 3: 1 to 1: 1 so as to obtain the ligand **(*R, S*)-L-OC,** 278 mg as light yellow solid, with a yield of 55%. ¹³C NMR (125 MHz, CDCl₃): *δ* (ppm) 26.89, 39.72, 60.36, 66.98, 83.30, 116.60, 118.56, 119.02, 120.12, 121.34, 121.75, 122.86, 123.11, 125.23, 125.71, 126.67, 127.47, 127.74, 127.81, 128.17, 128.33, 128.51, 128.80, 129.10, 129.27, 129.44, 129.53, 136.27, 139.65, 140.40, 141.78, 149.41, 149.70, 156.61, 156.90, 163.33, 163.56. HR (ESI): calcd for C₄₀H₂₉N₂O₂ [M+H]⁺ 569.22, found 569.22.
(2) Synthesis of ***M-*PtOC*:* (*R, S*)-L-OC** (200 mg, 0.35 mmol, 1.0 equiv.), potassium chloroplatinate (154 mg, 0.37 mmol, 1.05 equiv.), tetra-n-butylammonium bromide (11 mg, 0.035 mmol, 0.1 equiv.) were sequentially added into a 50 mL dry three-necked flask with a magnetic rotor and a condenser tube, followed by nitrogen purge for three times and addition of acetic acid (15 mL) pre-purged with nitrogen. After a reaction solution was bubbled with nitrogen for 30 minutes, it was stirred at 40°C for 12 hours, and then was reacted with stirring at 120°C for 2 days, cooled to the room temperature, and solvent was distilled off at a reduced pressure, and stannous chloride dihydrate (790 mg, 3.50 mmol, 10.0 equiv.) and dichloromethane (15 mL) were added and stirred at 40°C for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 2: 1 to 1: 1 so as to obtain a product ***M*-PtOC,** 80 mg as pale primrose solid, with a yield of 30%. ¹H NMR (500 MHz, DMSO-*d*₆): *δ* (ppm) 3.51 (d, *J =* 18.0 Hz, 1H), 3.60 (dd, *J =* 18.5, 5.5 Hz, 1H), 5.74 (d, *J =* 8.0 Hz, 1H), 5.98-6.02 (m, 2H), 6.26 (dd, *J* = 8.0*,* 1.0 Hz, 1H), 6.38 (t, *J =* 7.5 Hz, 1H), 6.63-6.67 (m, 2H), 6.88-6.90 (m, 2H), 7.05 (dd, *J =* 8.0, 1.5 Hz, 1H), 7.13-7.19 (m, 2H), 7.23 (dd, *J =* 7.5, 1.0 Hz, 1H), 7.27 (td, *J =* 7.5, 1.0 Hz, 1H), 7.34 (d, *J =* 8.0 Hz, 1H), 7.40 (d, *J =* 8.0 Hz, 1H), 7.53-7.58 (m, 2H), 7.66 (td, *J =* 7.5, 1.0 Hz, 1H), 7.86-7.90 (m, 2H), 8.13 (d, *J =* 7.5 Hz, 1H), 9.23 (d, *J =* 7.5 Hz, 1H), 9.52 (dd, *J =* 6.0, 1.5 Hz, 1H). HR (ESI): calcd for C₄₀H₂₇N₂O₂Pt [M+H]⁺ 762.17, found 762.17.

**Example 50, not in accordance with the present invention and provided for illustrative purposes only:** synthesis of tetradentate cyclometalated platinum (II) complex ***P*-PtS** as follow. 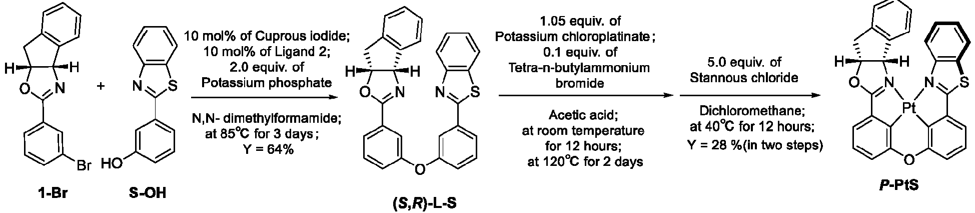
(1) Synthesis of ligand ***(S, R)-L-S:* 1-Br** (830 mg, 2.64 mmol, 1.2 equivalent), **S-OH** (500 mg, 2.20 mmol, 1.0 equivalent) and cuprous iodide (42 mg, 0.22 mmol, 10 mol%), ligand 2 (72 mg, 0.22 mmol, 10 mol%) and potassium phosphate (934 mg, 4.40 mmol, 2.0 equiv.) were sequentially added into a dry sealed tube with a magnetic rotor. Nitrogen was purged for three times, and N, N-dimethylformamide (20 mL) was added under nitrogen protection. The sealed tube was placed in an oil bath at 85 °C, stirred for reacting for 3 days, cooled to the room temperature, then sodium bicarbonate was added to adjust to weak base, ethyl acetate was added for extraction, with a water layer extracted with ethyl acetate for three times, then organic phases were combined, washed once with brine, dried with anhydrous sodium sulfate, and filtered, and solvent was distilled off at a reduced pressure. The crude was separated and purified by a silica gel chromatography column with an eluent of petroleum ether/dichloromethane of 3: 1 to 1: 1 so as to obtain the ligand **(*S, R*)-L-S,** 650 mg as light brown solid, with a yield of 64%. ¹H NMR (500 MHz, DMSO-*d*₆): *δ* (ppm) 3.22 (d, *J* = 18 Hz, 1H), 3.48 (dd, *J =* 18, 6.5 Hz, 1H), 5.52 (td, *J =* 8.0, 1.5 Hz, 1H), 5.69 (d, *J =* 8.0 Hz, 1H), 7.21-7.34 (m, 4H), 7.32 (ddd, *J* = 8.0, 2.5, 1.0 Hz, 1H), 7.41-7.43 (m, 1H), 7.50-7.49 (m, 2H), 7.52-7.56 (m, 2H), 7.61 (t, *J =* 8.0 Hz, 1H), 7.67-7.70 (m, 2H), 7.88 (ddd, *J =* 8.0, 1.5, 0.5 Hz, 1H), 8.04-8.05 (m, 1H), 8.14-8.16 (m, 1H). ¹³C NMR (125 MHz, CDCl₃): *δ* (ppm) 39.74, 83.31,
   117.73, 119.02, 121.12, 121.59, 122.10, 122.62, 123.35, 123.77, 125.26, 125.32, 125.61, 126.35, 127.46, 128.48, 129.8, 129.83, 130.43, 135.09, 135.43, 139.68, 141.80, 154.02, 156.69, 157.69, 163.35, 167.10.
(2) Synthesis of ***P*-PtS: (*S*, *R*)-L-S** (300 mg, 0.65 mmol, 1.0 equiv.), potassium chloroplatinate (282 mg, 0.68 mmol, 1.05 equiv.), tetra-n-butylammonium bromide (21 mg, 0.065 mmol, 0.1 equiv.) were sequentially added into a 50 mL dry three-necked flask with a magnetic rotor and a condenser tube, followed by nitrogen purge for three times and addition of acetic acid (20 mL) pre-purged with nitrogen. After a reaction solution was bubbled with nitrogen for 30 minutes, it was stirred at 30°C for 12 hours, and then was reacted with stirring at 120°C for 2 days, cooled to the room temperature, and solvent was distilled off at a reduced pressure, and stannous chloride dihydrate (734 mg, 3.25 mmol, 5.0 equiv.) and dichloromethane (15 mL) were added and stirred at 40°C for 1 day. The reaction solution was washed with water, the aqueous phase was extracted three times with dichloromethane, organic phases were combined, and solvent was distilled off at a reduced pressure. The crude was separated and purified by a silica gel chromatography column with an eluent of petroleum ether/dichloromethane of 4: 1 to 2: 1 so as to obtain a product P-PtS, 120 mg as pale primrose solid, with a yield of 28%. ¹H NMR (500 MHz, DMSO-*d*₆): *δ* (ppm) 3.54 (d, *J =* 18.5 Hz, 1H), 3.66 (dd, *J =* 18.5, 6.0 Hz, 1H), 6.05 (t, *J =* 6.0 Hz, 1H), 6.46 (d, *J =* 7.0 Hz, 1H), 7.01 (t, *J =* 7.5 Hz, 1H), 7.13 (dd, *J =* 8.0, 1.0 Hz, 1H), 7.15-7.18 (m, 1H), 7.19-7.25 (m, 4H), 7.30 (t, *J =* 7.5 Hz, 1H), 7.38 (d, *J =* 7.5 Hz, 1H), 7.57-7.60 (m, 1H), 7.65-7.70 (m, 2H), 8.33 (dd, *J =* 8.0, 1.0 Hz, 1H), 8.51 (d, *J =* 8.0 Hz, 1H).

### Electrochemical, photophysical testing and theoretical calculations

Absorption spectra were measured on an Agilent 8453 UV-Vis spectrometer, steady-state emission experiments and lifetime measurements on a Horiba Jobin Yvon FluoroLog-3 spectrometer, or steady-state emission spectroscopy on a SHIMADZU RF-6000 spectrometer. A low temperature (77 K) emission spectrum and lifetime were measured in a 2- methyltetrahydrofuran (2-MeTHF) solution cooled using liquid nitrogen. Theoretical calculation of Pt (II) complex was performed by using Gaussian 09 software package. A geometric structure of a molecular with a ground state (S₀) was optimized using density functional theory (DFT) and DFT calculation was performed by using B3LYP functional. 6-31G(d) baisc sets were used for C, H, O and N atoms, while LANL2DZ baisc sets were used for Pt atom. The enantiomeric purity (ee value) was determined on a chiral column EnantiopakR-C (with a specification of 4.6×250 mm, 5 um).

### Experimental data and analysis

FIG. 1 shows a design idea of an optically pure spiro chiral tetradentate cyclometalated platinum (II) and palladium (II) complex circularly polarized luminescence material with metal ion as a center: the optically pure raw materials are economical and easy to be obtained; spiro chirality can be generated by autonomous induction of central chirality; there's no need for chiral resolution for the circularly polarized luminescence material, which greatly saves preparation cost of the optical pure materials, and the circularly polarized luminescence materialcan be prepared in a large amount and is not limited by resolution of a chiral preparation column. From enantiomeric excess values (ee values) in Table 1, it can be seen that single class of chiral material molecules all with extremely high optical purity are obtained from material design ideas above, with ee values larger than 99%. Above experimental data also show that this material design idea is successful.

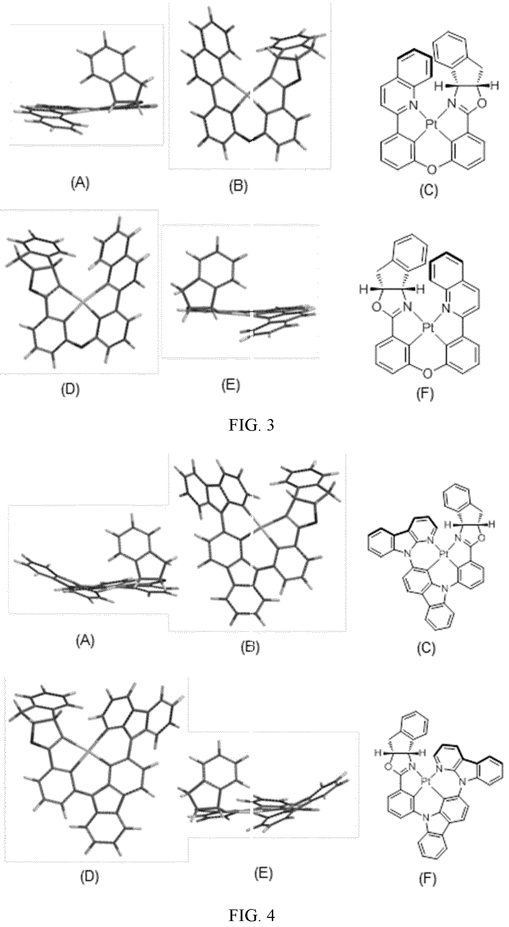

As can be seen from molecular structures calculated and optimized using density functional theory (DFT) of a series of tetradentate cyclometalated platinum (II) complexes with different ligand structures in FIGS. 2 to 5, ligands containing central chirality (R, S) can autonomously induce generation of M spiro chirality, ligands containing central chirality (S, R) can autonomously induce generation of P spiro chirality, and a M spiro chiral molecule and a P spiro chiral molecule are enantiomers to each other. An absolute configuration of the material molecules is supported by an X-ray diffraction single crystal structure of (*R*, *S*)-*M*-PtLA1 (left plot in FIG. 1). In addition, molecular structures of the tetradentate palladium (II) complexes calculated and optimized by DFT show similar results.

Structures of comparative tetradentate palladium (II) complexes in this disclosure are shown below. Since all of them are achiral molecules, both specific rotation ([a]²⁰_{D}) and asymmetry factor (*g*_{PL}) are zero.

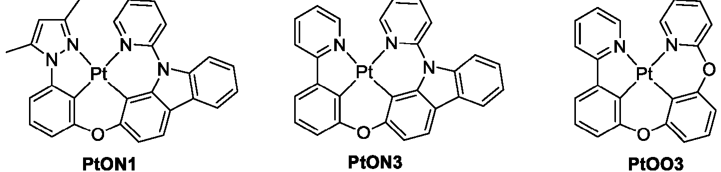

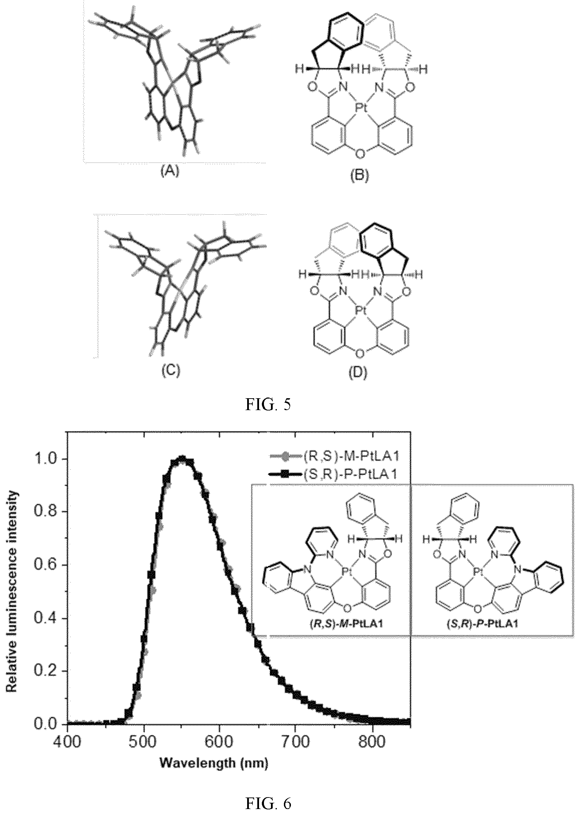

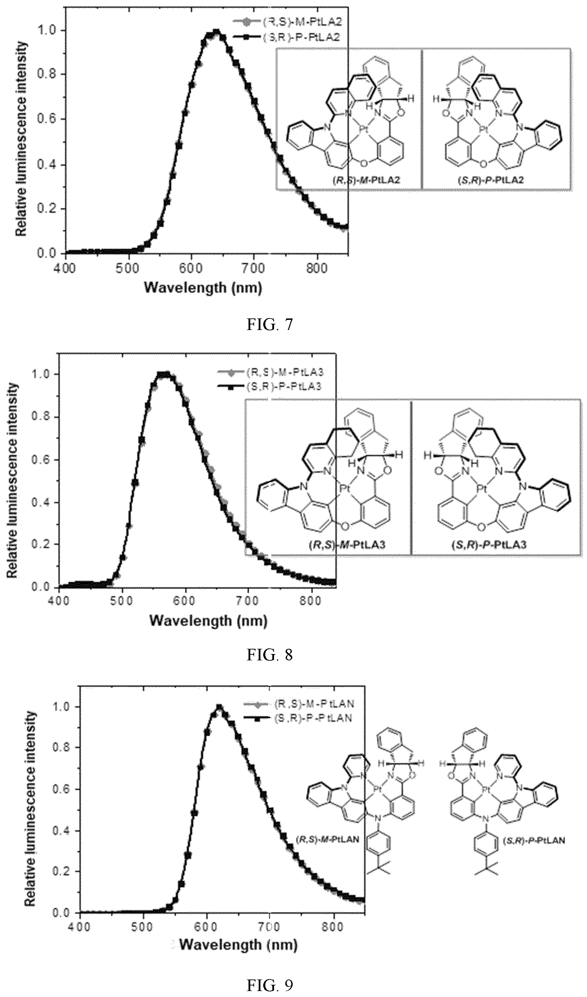

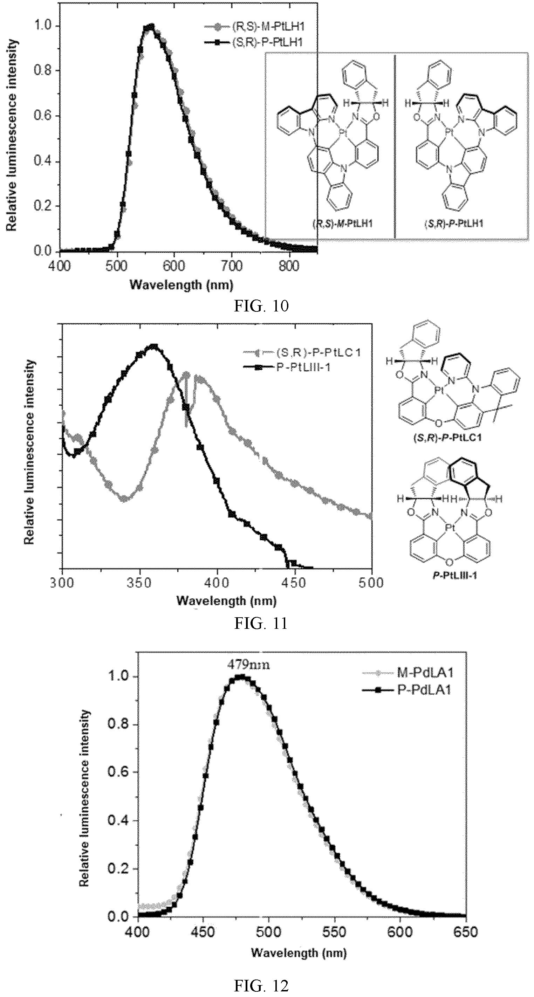

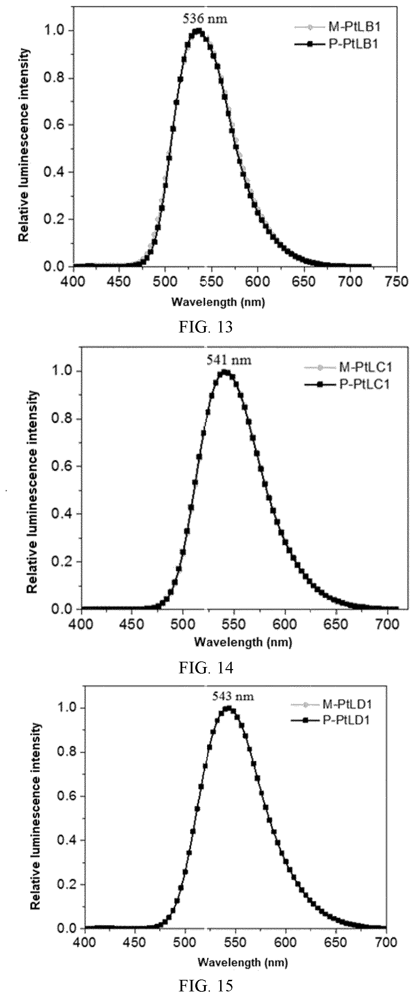

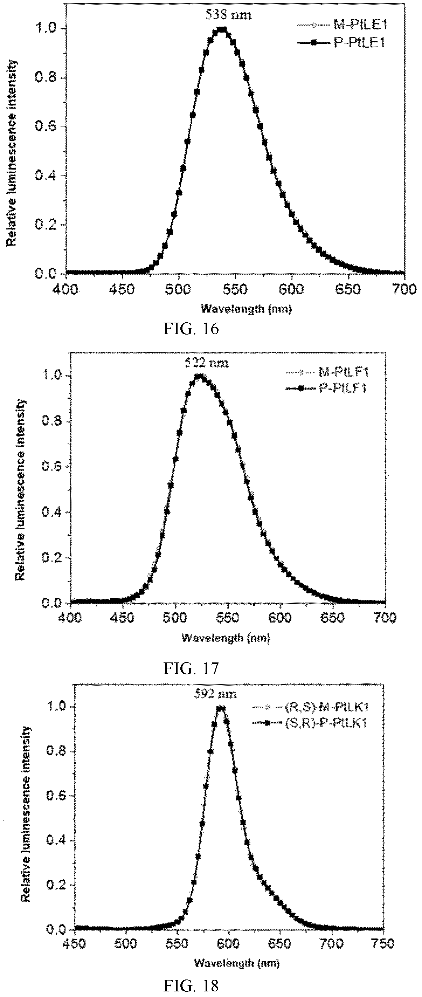

As can be seen from emission spectra of the optically pure spiro chiral material molecules synthesized in FIGS. 6 to 20 and table 2 in dichloromethane solution at the room temperature, the luminescent color of the tetradentate metal complex can be efficiently adjusted approximately by adjusting the structure of the tetradentate ligand, and adjustment from an ultraviolet region at about 360 nm to a red region at 650 nm can be achieved, and it is believed that infrared luminescence can also be achieved through further regulation of the tetradentate ligand structure. In addition, emission spectra from FIG. 6 to FIG. 20 are substantially completely coincident, which further proves that corresponding material molecules in the figures are enantiomers.

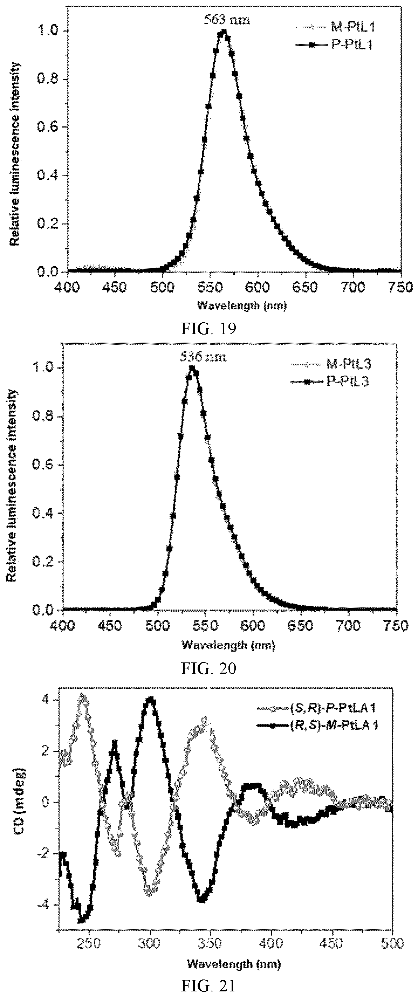

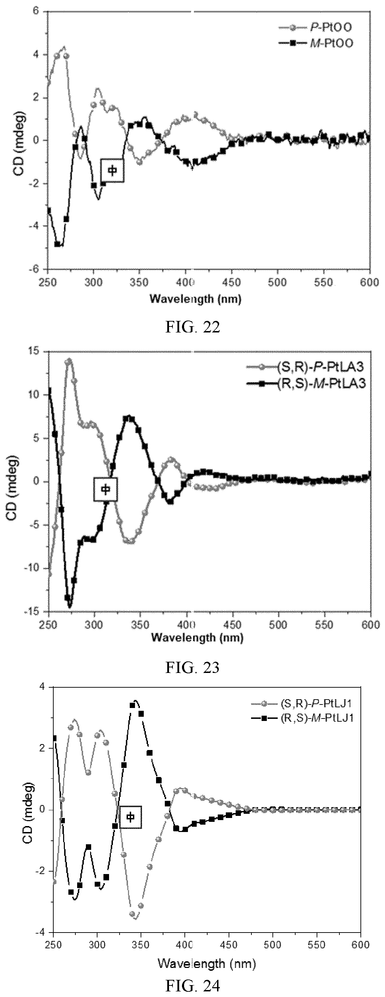

As can be seen from the circular dichroism of (*S, R*)-*P*-PtLA1 and (*R*, *S*)-*M*-PtLA1 in dichloromethane solution in FIG. 21, where CD means circular dichroism, with a sample concentration of 5×10⁻⁵ M, the spectra have high mirror symmetry and strong Cotton effect at about 248, 275, 300, and 248nm, indicating that enantiomers (*S, R*)-*P*-PtLA1 and (*R*, *S*)*-M-*PtLA1 have strong deflection ability for linearly polarized light. As can also be seen from the specific rotation ([a]²⁰_{D}) data in the following Table 1, the spiro chiral tetradentate metal material molecules in the present disclosure all show strong deflection ability for linearly polarized light, and circular dichroism spectra of other parts of the material molecules are shown in FIGS. 22 to 25. Specific rotation ([a]²⁰_{D}) of the comparative material molecules PtON1, PtON3 and PtOO3 are all zero.

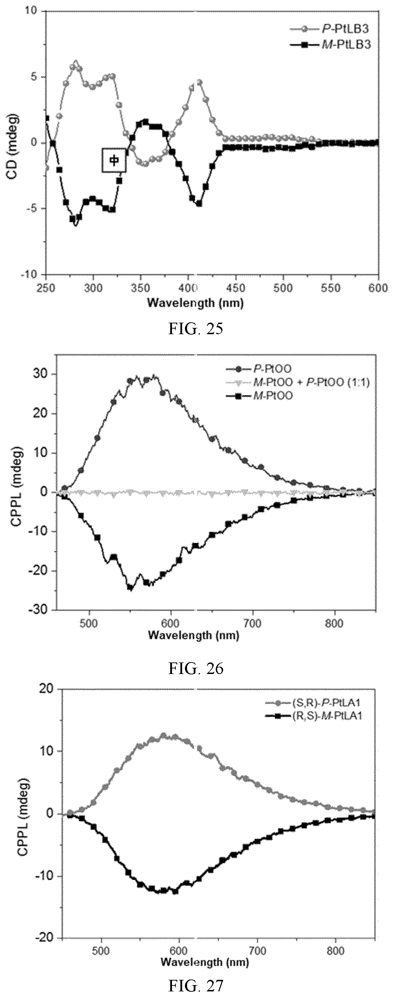

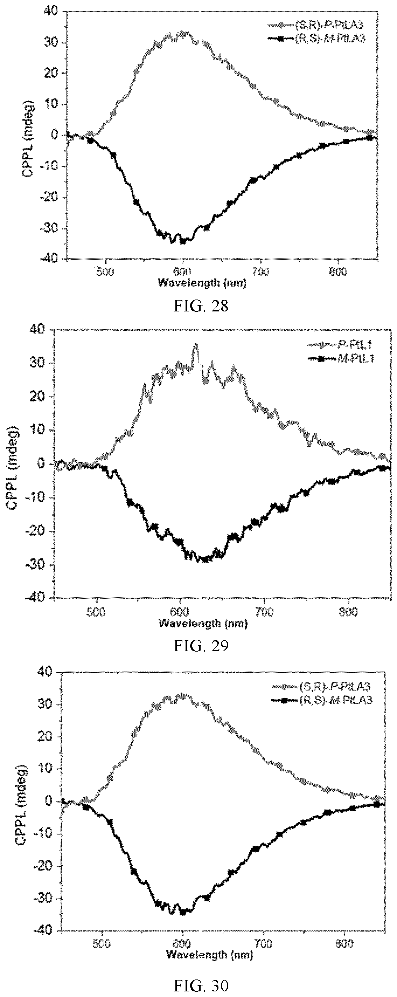

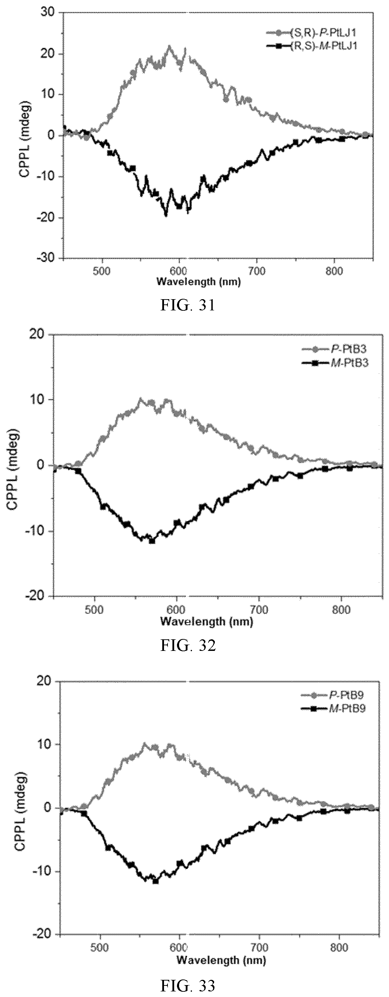

It can be seen from Table 2 that the spiro chiral material molecules in the application all show strong circularly polarized luminescence, with an absolute value of the asymmetry factor (*g*_{PL}) as high as 4.0×10⁻³. FIGS. 26 to 33 are circularly polarized luminescence spectra of part of material molecules. However, the comparative material molecules PtON1, PtON3 and PtOO3 have no circularly polarized luminescence.

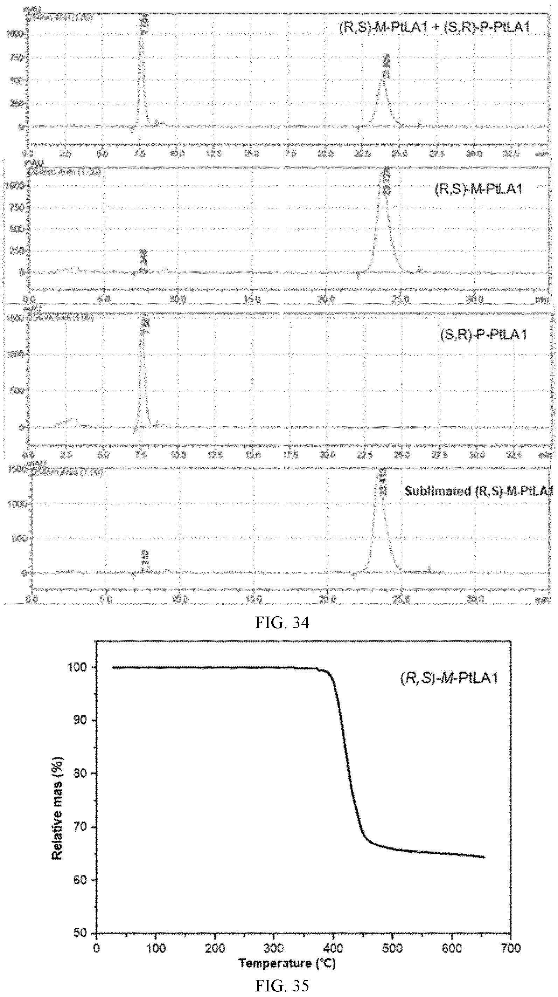

The material has high chemical stability and thermal stability. The designed and developed tetradentate ligand can well coordinate with dsp² hybridized platinum (II) and palladium (II) metal ions to form molecules with a stable and rigid quadrilateral configuration, with high chemical stability; meanwhile, since large steric hindrance effect exists between the designed central chiral ligand L^{a} and the ligand L¹ or L^{b} at another end, the metal complex molecule as a whole can form a stable spiro chiral tetradentate cyclometalated complex, so that the spiro chiral tetradentate cyclometalated complex can not be racemized and lose circularly polarized luminescence property in a solution or in a process of sublimation at a high temperature. As can be seen from a high performance liguid chromatography (HPLC) spectrum of a mixture of (R, S)-M-PtLA1 and (S, R)-P-PtLA1, a high performance liguid chromatography spectrum of optically pure (*R*, *S*)-*M*-PtLA1; a high performance liguid chromatography spectrum of optically pure (*S*, *R*)-*P*-PtLA1, and a high performance liguid chromatography spectrum of sublimated (*R*, *S*)-*M*-PtLA1 from top to bottom in FIG. 34, such material has high thermal stability, sublimation experiment shows that (*R*, *S*)-*M*-PtLA1 and (*S*, *R*)-*P*-PtLA1 materials sublimated at 320 °C respectively have no racemization. As can be seen from the thermogravimetric analysis curve of (*R, S*)-*M*-PtLA1 in FIG. 35, its decomposition temperature (a temperature with 5% of mass loss) is as high as 405°C.

As can be seen from specific rotation data of part of chiral raw intermediates and chiral metal complexes in the following Table 1, even if rotation directions of the raw intermediates and the chiral metal complexes containing same central chirality are completely different, their specific rotation values exhibit huge difference, which indicates that spiro chirality of the complexes with the metal ions as the center has decisive influence on rotation properties of the whole compound. In addition, for the complexes with same central chirality and spiro chirality, specific rotation may vary greatly with different ligand structures, for example, (*S, R*)-*P*-PtLA1 (+477.2) and (*S, R*)-*P*-PtLA1 (+784.6), indicating that the ligand structure had great effect on its rotation. Meanwhile, because stimulated luminescence of the metal complexes mainly involves metal-to-ligand charge transfer states (MLCT) and intra-ligand charge transfer (ILCT) states, spiro chirality and ligand structures of the metal complexes also have significant effect on circularly polarized light properties.

**Table 1 Specific rotation ([a]²⁰_{D}) of part of chiral raw intermediates and chiral metal complexes**

| Compound or metal complex | Enantiomeric purity (ee value) | Specific rotation (°) | Compound or metal complex | Enantiomeric purity (ee value) | Specific rotation (°) |
|---|---|---|---|---|---|
| **PtON1** | | 0 | **PtON3** | | 0 |
| | | | **PtOO3** | | 0 |
| **2-Br (1*R*,2*S*)** | | +157.2 | **1-Br (1*S,*2*R*)** | | -157.6 |
| **(*R, S*)*-M-*PtLA1** | >99% | -499.6 | **(*S, R*)*-P-*PtLA1** | >99% | +477.2 |
| **(*R, S*)*-M-*PtLA2** | >99% | -776.2 | **(*S, R*)*-P-*PtLA2** | >99% | +784.6 |
| **(*R, S*)*-M-*PtLA3** | >99% | -409.5 | **(*S*, *R*)-P-PtLA3** | >99% | +388.0 |
| **(*R, S*)*-M-*PtLAN** | >99% | -457.2 | **(*S*, *R*)-*P*-PtLAN** | >99% | +439.6 |
| **(*R, S*)*-M-*PtLH1** | >99% | -715.2 | **(*S, R)-P-*PtLH1** | >99% | +785.6 |
| **(*S, R*)*-M-*PtLI1** | >99% | -161.5 | **(*S, R*)-*P*-PtLI1** | >99% | +150.0 |
| **(*S,* R)-M-PtLJ1** | >99% | -361.2 | **(*S, R*)*-P-*PtLJ1** | >99% | +349.5 |
| **(*R, S*)-*M*-PtLK1** | >99% | -844.0 | **(*S*, *R*)-*P*-PtLK1** | >99% | +899.0 |
| ***M* -PtLIII-1** | >99% | -984.3 | **P-PtLIII-1** | >99% | +997.4 |
| ***M*-PtLB1** | >99% | -1026.6 | ***P*-PtLB1** | >99% | +1032.3 |
| ***M*-PtLC1** | >99% | -496.7 | ***P*-PtLC1** | >99% | +481.0 |
| ***M*-PtLD1** | >99% | -687.9 | ***P-*PtLD1** | >99% | +690.4 |
| ***M-*PtLE1** | >99% | -683.9 | ***P*-PtLE1** | >99% | +700.3 |
| ***M*-PtLF1** | >99% | -703.6 | ***P*-PtLF1** | >99% | +686.8 |
| ***M*-PtB1** | >99% | -672.0 | ***P*-PtB1** | >99% | +631.1 |
| ***M*-PtB2** | >99% | -923.7 | ***P*-PtB2** | >99% | +881.2 |
| ***M*-PtB3** | >99% | -1348.7 | ***P*-PtB3** | >99% | +1337.6 |
| ***M*-PtB5** | >99% | | ***P*-PtB5** | >99% | +715.0 |
| ***M*-PtB6** | >99% | | ***P*-PtB6** | >99% | +671.2 |
| ***M*-PtB8** | | -633.8 | | | |
| ***M*-PtB9** | >99% | -683.6 | ***P*-PtB9** | >99% | +656.7 |
| ***M*-PdLA1** | >99% | | ***P*-PdLA1** | >99% | |
| ***M*-PtL1** | >99% | -141.7 | ***P*-Pt L1** | >99% | +139.2 |
| ***M-*PtL2** | >99% | -125.8 | ***P*-Pt L2** | >99% | +137.0 |
| ***M*-PtL3** | >99% | -196.0 | ***P*-Pt L3** | >99% | +187.5 |
| ***M*-PtOO** | >99% | -585.7 | ***P*-PtOO** | >99% | +567.2 |
| ***M*-PtOC** | >99% | -29.2 | ***P*-PtOC** | >99% | +24.5 |
| | | | ***P*-PtS** | | +671.2 |

| | | | | | |
|---|---|---|---|---|---|
| Remarks: specific rotation values of all samples were determined in a dichloromethane solution. | | | | | |

**Table 2: maximum emission wavelength (λₘₐₓ) and asymmetry factor (g_{PL}) for chiral metal complexes**

| Metal complex | *λ*ₘₐₓ (nm) | *g*_{PL} (×10⁻³) | Metal complex | *λ*ₘₐₓ (nm) | *g*_{PL} (×10⁻³) |
|---|---|---|---|---|---|
| **PtON1** | | 0 | **PtON3** | | 0 |
| | | | **PtOO3** | | 0 |
| **(*R, S*)*-M-PtLA1*** | 535 | -2.8 | **(*S, R*)*-P-*PtLA1** | 535 | +2.7 |
| **(*R, S*)-*M-PtLA2*** | 610 | -1.8 | **(*S, R*)*-P-*PtLA2** | 610 | +1.8 |
| **(*R, S*)*-M-PtLA3*** | 546 | -4.1 | **(*S*, *R*)-*P*-PtLA3** | 546 | +3.7 |
| ***M*-PtLB1** | 536 | -2.8 | ***P*-PtLB1** | 536 | +2.9 |
| ***M*-PtLC1** | 541 | -3.1 | ***P*-PtLC1** | 541 | +3.3 |
| ***M*-PtLD1** | 543 | -3.8 | ***P*-PtLD1** | 543 | +3.6 |
| ***M*-PtLE1** | 538 | -3.2 | ***P*-PtLE1** | 538 | +3.1 |
| ***M*-PtLF1** | 522 | -2.4 | ***P*-PtLF1** | 522 | +2.4 |
| **(*R, S*)*-M-*PtLAN** | 620 | -1.1 | **(*S*, *R*)-*P*-PtLAN** | 620 | +1.2 |
| **(*S, R*)-*M*-PtLH1** | 556 | -1.8 | **(*S, R*)*-P-*PtLH1** | 556 | +1.6 |
| **(*S, R*)-*M*-PtLI1** | 574 | | **(*S, R*)-*P*-PtLI1** | 574 | |
| **(*S, R*)-*M*-PtLJ1** | 545 | -2.2 | **(*S, R*)*-P-*PtLJ1** | 545 | +2.30 |
| **(*R, S*)-*M*-PtLK1** | 592 | -0.38 | **(*S*, *R*)-*P*-PtLK1** | 592 | +0.40 |
| ***M*-PtLIII-1** | 358 | | ***P*-PtLIII-1** | 358 | |
| ***M*-PtB1** | 536 | -0.23 | ***P*-PtB1** | 536 | +0.26 |
| ***M*-PtB2** | 624 | -0.65 | ***P*-PtB2** | 624 | +0.59 |
| ***M*-PtB3** | 603 | -2.02 | ***P*-PtB3** | 603 | +1.95 |
| ***M*-PtB9** | 523 | -1.14 | ***P*-PtB9** | 523 | +1.03 |
| ***M*-PdLA1** | 479 | -1.80 | ***P*-PdLA1** | 479 | +1.10 |
| ***M*-PtL1** | 563 | -0.57 | ***P*-Pt L1** | 563 | +0.40 |
| ***M*-PtL2** | 562 | -2.94 | ***P*-Pt L2** | 562 | +3.50 |
| ***M*-PtL3** | 536 | -2.00 | ***P*-Pt L3** | 536 | +2.00 |
| ***M*-PtOO** | 542 | -4.00 | ***P*-PtOO** | 542 | +3.70 |
| ***M*-PtOC** | 531 | -2.50 | ***P*-PtOC** | 531 | +2.70 |
| | | | ***P*-PtS** | | |

| | | | | | |
|---|---|---|---|---|---|
| Remarks: the maximum emission wavelength (λₘₐₓ) and the asymmetry factor (*g*_{PL}) of all samples were determined in a dichloromethane solution. | | | | | |

As can be seen from experimental data for theoretical calculation of part of chiral metal complex material molecules in Table 3 below, such molecular parent nuclei containing chiral structural units are all in a distorted quadrilateral structure; and due to existence of ortho-chiral steric hindrance in a coordinated heterocyclic ring, two terminal heterocyclic rings coordinated with the central metal ions can be respectively positioned at two sides of a plane, so that spiro chiral molecules with the central metal as the center is formed, which all can be used as circularly polarized luminescence materials.

In addition, a large amount of experimental data for theoretical calculation also indicates importance of the steric hindrance of ortho groups in the chiral structural units coordinated with the central metal, which is the key for the central chiral induction of the chiral structural units of the ligand to generate the spiro chirality of the whole material molecules with the metal as the center. Meanwhile, a large number of synthetic experimental examples and characterization and testing of their photophysical properties also show that the design method of the circular polarized luminescent material molecules in the present application is completely successful.

**Table 3: Experimental data for theoretical calculation of part of chiral metal complex material molecules**

| Metal complex material molecules | Front view | Side view | LUMO energy level eV | HOMO energy level eV | Energy gap eV | Dihedral angle (°) |
|---|---|---|---|---|---|---|
| 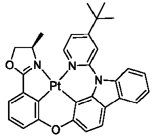 | 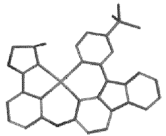 | 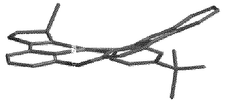 | -1.28 | -4.72 | 3.44 | 37.6 |
| 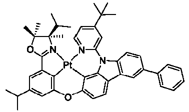 | 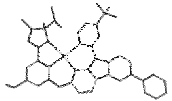 | 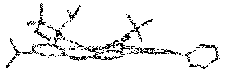 | -1.34 | -4.72 | 3.38 | 38.7 |
| 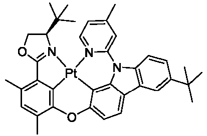 | 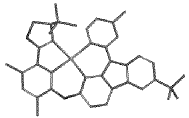 | 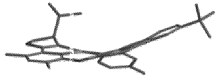 | -1.23 | -4.63 | 3.40 | 39.0 |
| 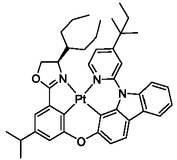 | 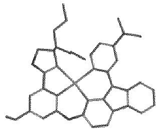 | 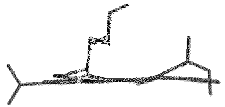 | -1.31 | -4.68 | 3.37 | 18.9 |
| 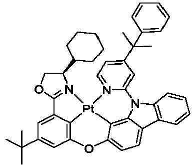 | 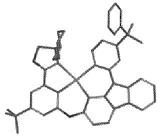 | 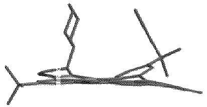 | -1.30 | -4.67 | 3.37 | 15.9 |
| 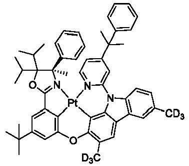 | 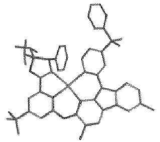 | 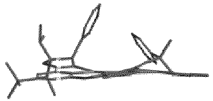 | -1.18 | -4.59 | 3.41 | 26.6 |
| 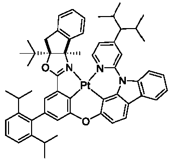 | 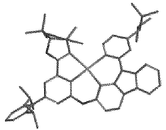 | 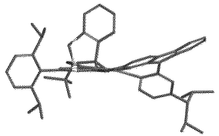 | -1.30 | -4.75 | 3.45 | 87.2 |
| 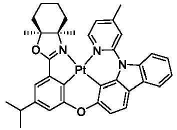 | 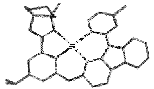 | 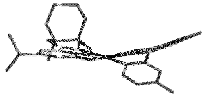 | -1.24 | -4.66 | 3.42 | 47.2 |
| 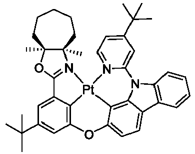 | 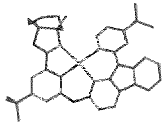 | 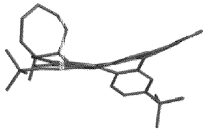 | -1.21 | -4.63 | 3.42 | 46.7 |
| 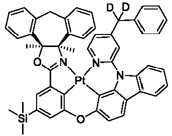 | 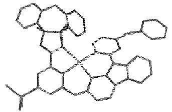 | 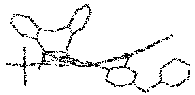 | -1.28 | -4.75 | 3.47 | 66.1 |
| 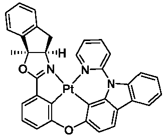 | 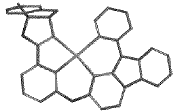 | 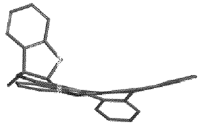 | -1.37 | -4.73 | 3.36 | 28.8 |
| 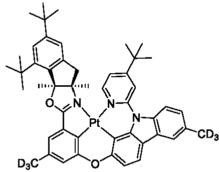 | 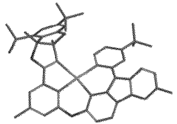 | 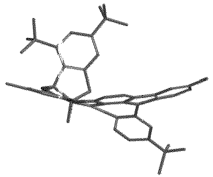 | -1.18 | -4.65 | 3.47 | 48.7 |
| 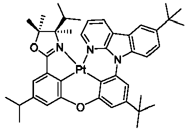 | 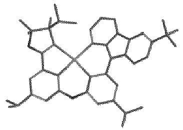 | 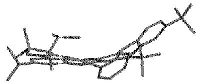 | -1.53 | -4.73 | 3.2 | 53.0 |
| 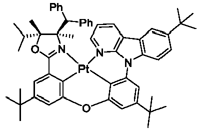 | 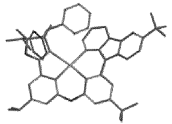 | 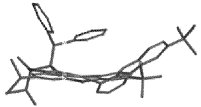 | -1.51 | -4.80 | 3.29 | 44.0 |
| 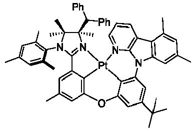 | 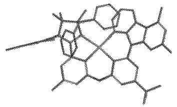 | 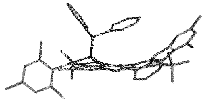 | -1.34 | -4.60 | 3.26 | 41.2 |
| 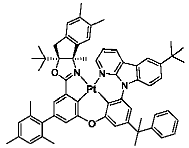 | 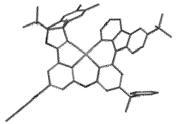 | 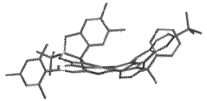 | -1.56 | -4.77 | 3.21 | 58.2 |
| 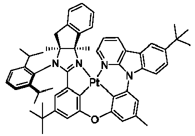 | 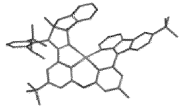 | 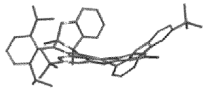 | -1.47 | -4.54 | 3.07 | 65.7 |
| 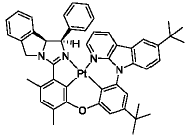 | 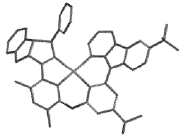 | 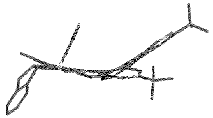 | -1.38 | -4.67 | 3.28 | 31.4 |
| 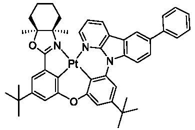 | 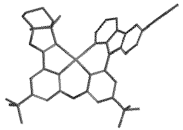 | 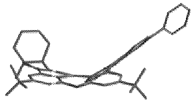 | -1.58 | -4.74 | 3.16 | 49.3 |
| 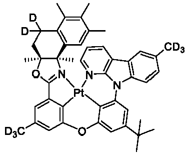 | 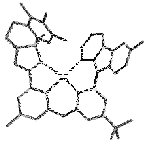 | 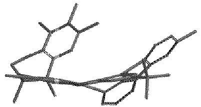 | -1.52 | -4.78 | 3.26 | 45.3 |
| 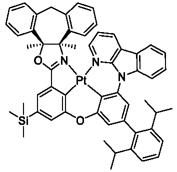 | 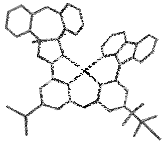 | 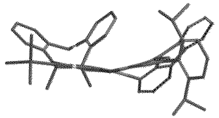 | -1.64 | -4.99 | 3.35 | 49.4 |
| 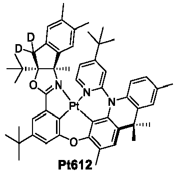 | 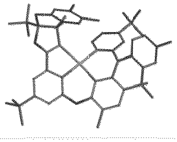 | 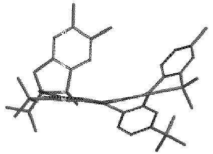 | -1.13 | -4.58 | 3.45 | 53.9 |
| 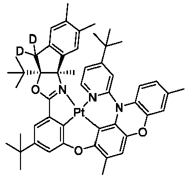 | 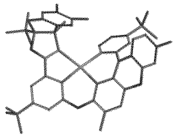 | 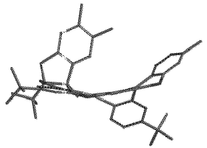 | -1.13 | -4.52 | | 55.3 |
| 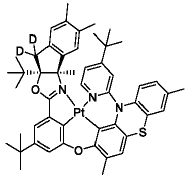 | 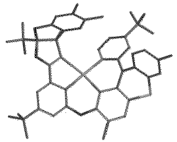 | 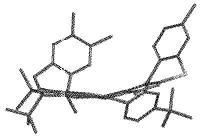 | -1.13 | -4.64 | 3.51 | 49.4 |
| 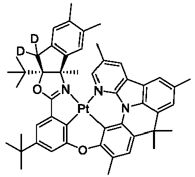 | 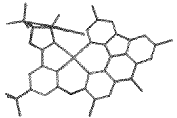 | 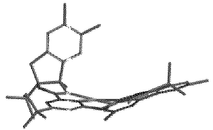 | -1.43 | -4.66 | 3.23 | 44.4 |
| 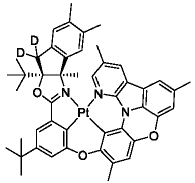 | 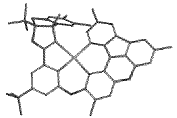 | 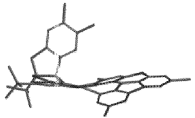 | -1.48 | -4.55 | 3.07 | 43.5 |
| 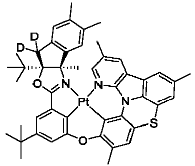 | 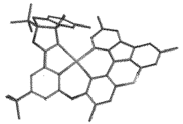 | 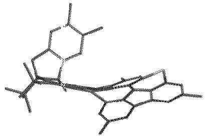 | -1.53 | -4.60 | | 42.0 |
| 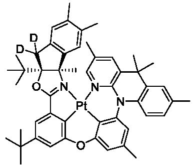 | 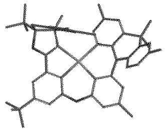 | 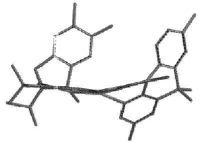 | -1.12 | -4.63 | 6.51 | 51.4 |
| 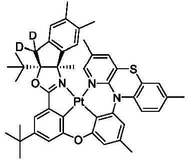 | 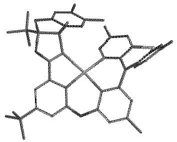 | 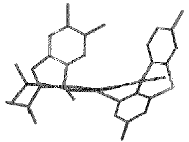 | -1.28 | -4.69 | 3.41 | 51.1 |
| 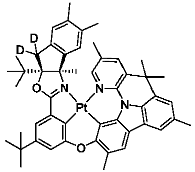 | 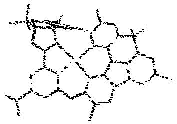 | 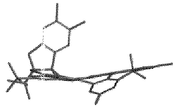 | -1.14 | -4.61 | 3.47 | 45.9 |
| 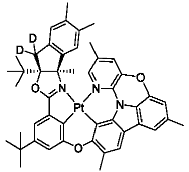 | 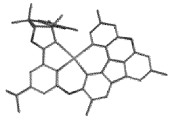 | 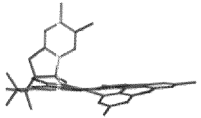 | -1.25 | -4.71 | 3.46 | 41.0 |
| 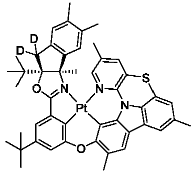 | 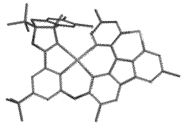 | 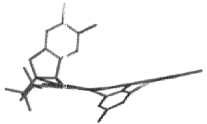 | -1.30 | -4.71 | 3.41 | 48.4 |
| 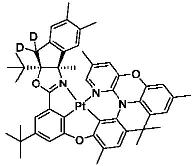 | 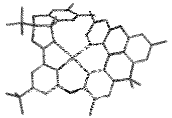 | 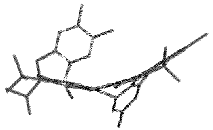 | -1.11 | -4.69 | 3.58 | 63.5 |
| 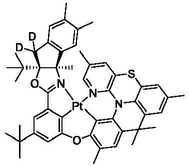 | 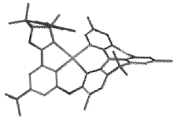 | 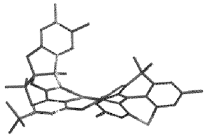 | -1.18 | -4.62 | 3.44 | 61.1 |
| 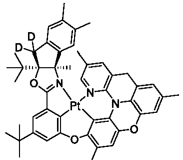 | 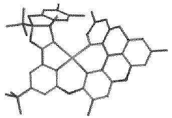 | 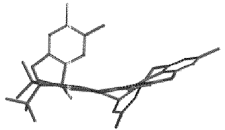 | -1.11 | -4.50 | 3.39 | 56.0 |
| 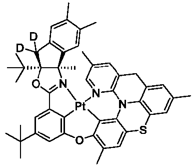 | 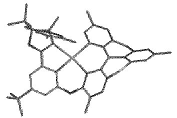 | 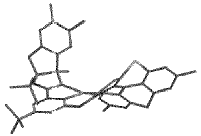 | -1.19 | -4.54 | 3.35 | 61.8 |
| 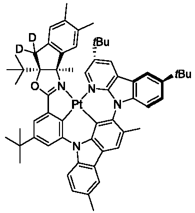 | 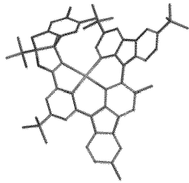 | 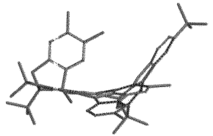 | -1.48 | -4.43 | 2.95 | 56.1 |
| 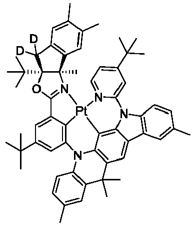 | 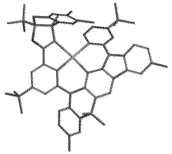 | 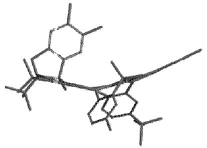 | -1.14 | -4.23 | 3.09 | 52.5 |
| 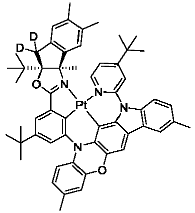 | 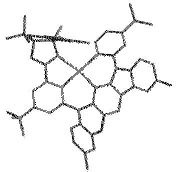 | 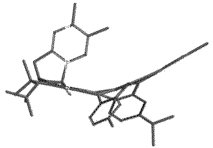 | -1.19 | -4.15 | 2.96 | 53.0 |
| 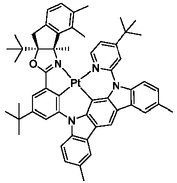 | 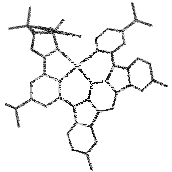 | 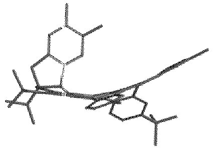 | -1.16 | -4.37 | 3.21 | 49.5 |
| 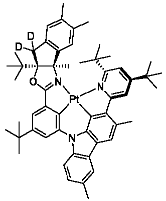 | 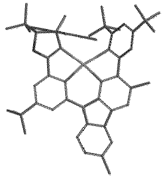 | 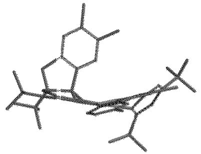 | -1.32 | -4.42 | 3.1 | 61.2 |

| | | | | | | |
|---|---|---|---|---|---|---|
| Remarks: the dihedral angle refers to an angle formed between the two terminal heterocyclic rings coordinated with the central metal ions. | | | | | | |

In an organic light emitting element, carriers are injected into a luminescent material from positive and negative electrodes to generate a luminescent material at an excited state and cause it to illuminate. The complex of the present disclosure represented by the general formula (1) can be applied as a phosphorescent luminescent material to an excellent organic light emitting element such as an organic photoluminescent element or an organic electroluminescent element. The organic photoluminescence element has a structure in which at least a light-emitting layer is formed on a substrate. In addition, the organic electroluminescent element has a structure in which at least an anode, a cathode, and an organic layer between the anode and the cathode are formed.
The organic layer includes at least a light-emitting layer and may be composed of only the light-emitting layer or may have more than one organic layer in addition to the light-emitting layer. Example of such other organic layers include a hole transport layer, a hole injection layer, an electron blocking layer, a hole blocking layer, an electron injection layer, an electron transport layer, an exciton blocking layer, and the like. The hole transport layer may be a hole injection and transport layer having a hole injection function, and the electron transport layer may be an electron injection and transport layer having an electron injection function. Specifically, a structure of the organic light emitting element is schematically shown in FIG. 35. In FIG. 35, there are seven layers in total from bottom to top, in which the substrate, the anode, the hole injection layer, the hole transport layer, the light-emitting layer, the electron transport layer, and the cathode are sequentially shown, where the light-emitting layer is a mixed layer in which a guest material is doped into a host material.

Compounds represented in examples 1 to 50 were applied to an OLED device as a phosphorescent luminescent material, with a structure being expressed as:
ITO /HATCN (10 nm)/TAPC (65 nm)/host material: luminescent material (10 wt.%, 20nm)/TmPyPB (55 nm)/LiF/Al.

ITO represents a transparent anode; HATCN represents the hole injection layer, TAPC represents the hole transport layer, and the host material is mCBP and 26mCPy, respectively. TmPyPB represents the electron transport layer, LiF represents the electron injection layer, and Al represents the cathode. Numbers in nanometer (nm) in parentheses are thicknesse of films.

A molecular formula of the material used in the device is as follows:

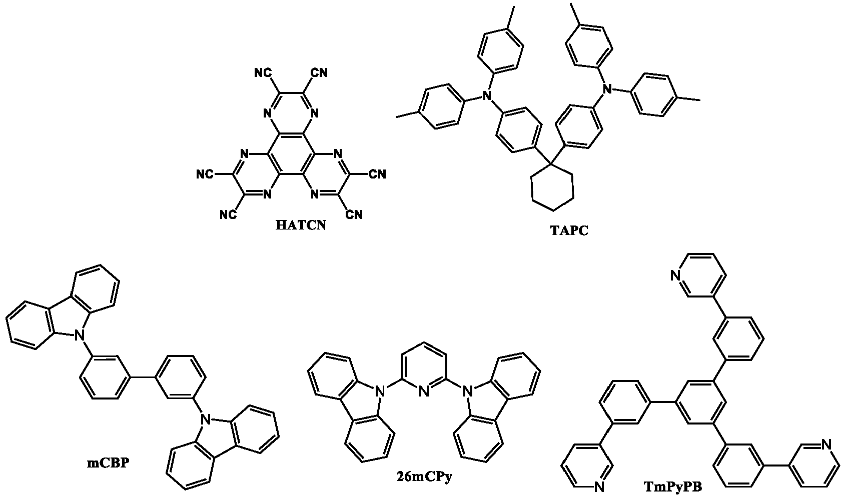

Further, in an organic light emitting device, carriers are injected into a luminescent material from positive and negative electrodes to generate a luminescent material at an excited state and cause it to illuminate. The complex of the present disclosure can be applied as a phosphorescent luminescent material to an excellent organic light emitting element such as an organic photoluminescent element or an organic electroluminescent element. The organic photoluminescence device has a structure in which at least a light-emitting layer is formed on a substrate. In addition, the organic electroluminescent element has a structure in which at least an anode, a cathode, and an organic layer between the anode and the cathode are formed. The organic layer includes at least a light-emitting layer and may be composed of only the light-emitting layer or may have more than one organic layer in addition to the light-emitting layer. Example of such other organic layers include a hole transport layer, a hole injection layer, an electron blocking layer, a hole blocking layer, an electron injection layer, an electron transport layer, an exciton blocking layer, and the like. The hole transport layer may be a hole injection and transport layer having a hole injection function, and the electron transport layer may be an electron injection and transport layer having an electron injection function. Specifically, a structure of the organic light emitting element is schematically shown in FIG. 6. In FIG. 6, there are seven layers in total from bottom to top, in which the substrate, the anode, the hole injection layer, the hole transport layer, the light-emitting layer, the electron transport layer, and the cathode are sequentially shown, where the light-emitting layer is a mixed layer in which a guest material is doped into a host material.

Respective layers of the organic light emitting device of the present disclosure may be formed by methods such as vacuum evaporation, sputtering, ion plating, or can be formed in a wet manner such as spin coating, printing, Screen Printing, and the like, and solvent used is not specifically limited.

In a preferred embodiment of the present disclosure, an OLED device of the present disclosure contains a hole transport layer, a hole transport material may preferably be selected from known or unknown materials, particularly preferably from following structures, which does not imply that the present disclosure is limited to the following structures:

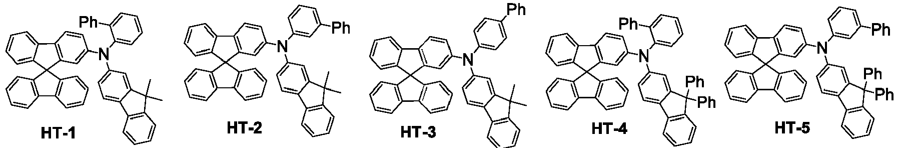

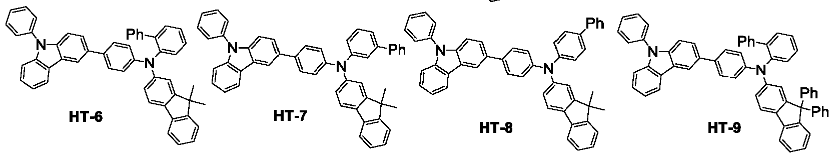

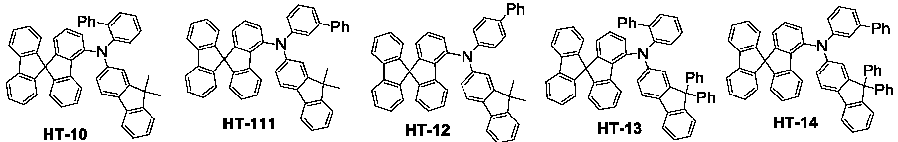

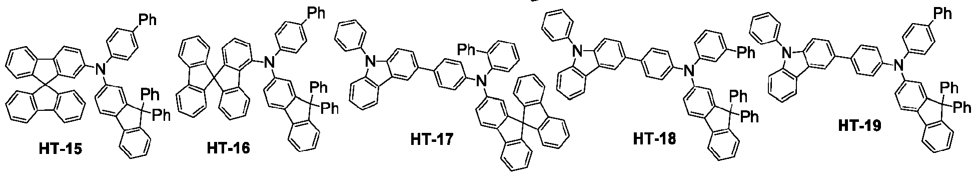

In a preferred embodiment of the present disclosure, the OLED device of the present disclosure contains a hole transport layer comprising one or more p-type dopants. A preferred p-type dopant of the present disclosure is of following structures, which does not imply that the present disclosure is limited to the following structures:

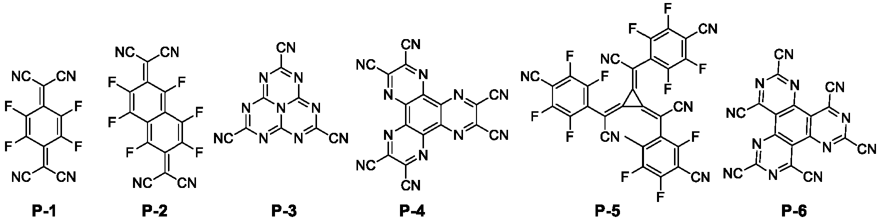

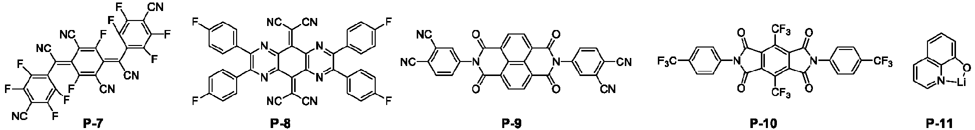

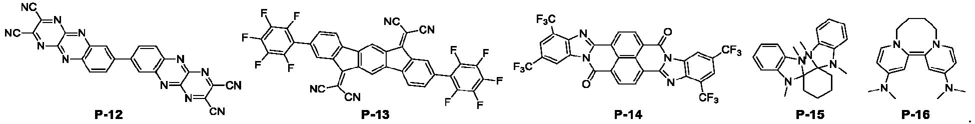

In a preferred embodiment of the present disclosure, the electron transport layer may be selected from at least one of compounds ET-1 to ET-13, which does not imply that the present disclosure is limited to following structures:

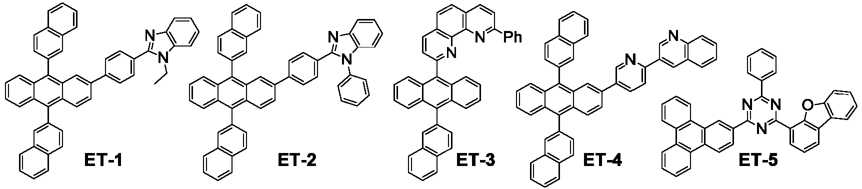

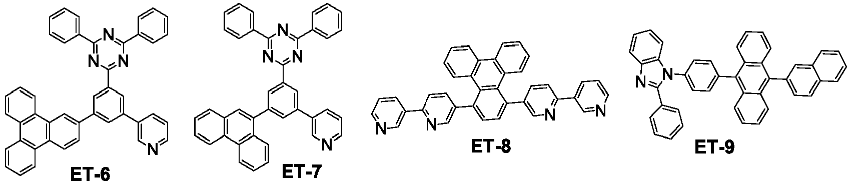

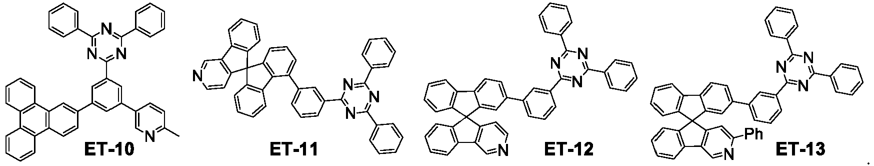

The electron transport layer may be formed of an organic material in combination with one or more n-type dopants such as LiQ.

The compound represented in example 1 was applied to an OLED device as a circularly polarized luminescence material, with a structure also being expressed as: on ITO-containing glass, a hole injection layer (HIL) of HT-1:P-3 (95: 5 v/v ), with a thickness of 10 nm; a hole transport layer (HTL) of HT-1, with a thickness of 90 nm; an electron blocking layer (EBL) of HT-10, with a thickness of 10 nm; a light-emitting layer (EML) of the host material (H-1 or H-2 or H-3 or H-4 or H-5 or H-6): the platinum metal complex of the present disclosure (95: 5 v/v), with a thickness of 35 nm; an electron transport layer (ETL) of ET-13: LiQ (50: 50 v/v ), with a thickness of 35 nm; and then a 70 nm vapor-deposited cathode Al.

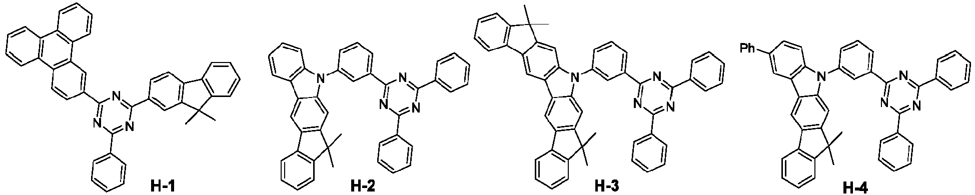

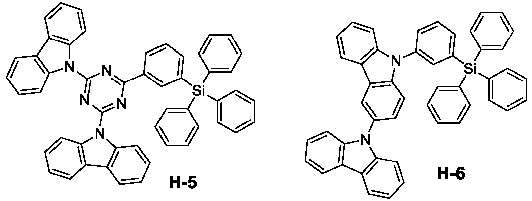

The fabricated organic light emitting device was tested at a current of 10 mA/cm² using standard methods well known in the art, in which a device with (*S*, *R*)-*P*-PtLA1 as the luminescent material had a significant circularly polarized electroluminescent signal, with an asymmetry factor (*g*_{EL}) of up to 1.4×10⁻³ and a maximum external quantum efficiency (EQE) of up to 18%.

It should be noted that the structure is an example of application of the circularly polarized luminescence material of the present disclosure and does not limit a specific structure of the OLED device of the circularly polarized luminescence material of the present disclosure, and the circularly polarized luminescence material is not limited to the compounds represented in the examples.

It should be noted that the structure is an example of application of the phosphorescent material of the present disclosure and does not limit a specific structure of the OLED device of the phosphorescent material of the present disclosure, and the phosphorescent material is not limited to the compounds represented in the examples.

## Claims

1. A central chirality induced spiro chiral tetradentate cyclometalated platinum (II) and palladium (II) complex-based circularly polarized luminescence material, wherein the circularly polarized luminescence material has a chemical formula as shown in general formulas (III) and (III'), where (III) and (III') are enantiomers of each other: 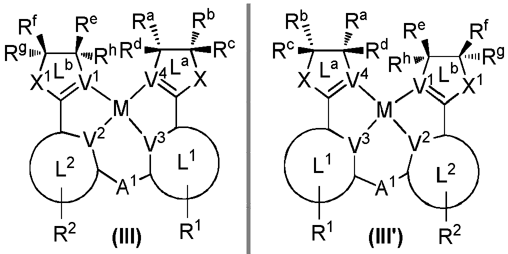
wherein M is Pt or Pd; and V¹, V², V³ and V⁴ are independently N or C;
L¹, L² and L³ are each independently a five or six membered carbocyclic, heterocyclic, aromatic or heteroaromatic ring; and L^{a} and L^{b} are each independently a five-membered central chiral carbocyclic or heterocyclic ring;
A¹, A², X and X¹ are each independently O, S, CR^{x}R^{y}, C=O, SiR^{x}R^{y}, GeR^{x}R^{y}, NR^{z}, PR^{z}, R^{z}P=O, AsR^{z}, R^{z}As=O, S=O, SO₂, Se, Se=O, SeO₂, BH, BR^{z}, R^{z}Bi=O or BiR^{z}; and
R¹ and R² each independently represent mono-,di-,tri-, tetra-substituted or unsubstituted, and meanwhile, R¹, R², R^{a}, R^{b}, R^{c}, R^{d}, R^{e}, R^{f}, R^{g}, R^{h}, R^{x}, R^{y} and R^{z} are each independently hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphonamido, imino, sulfo, carboxyl, hydrazino, substituted silyl, or combinations thereof; two adjacent R¹ and R² can be selectively connected to form a fused ring; any two of R^{a}, R^{b}, R^{c} and R^{d} are connected to form a cyclic system, and any two of R^{e}, R^{f}, R^{g} and R^{h} can be connected to form a cyclic system.

2. The central chirality induced spiro chiral tetradentate cyclometalated platinum (II) and palladium (II) complex-based circularly polarized luminescence material according to claim 1, wherein structures of L^{a} and L^{b} in the general formulas are preferably selected from: 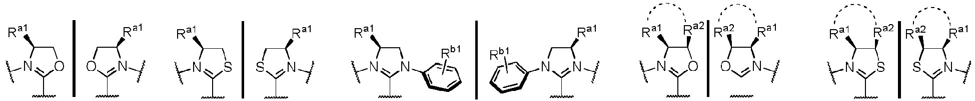 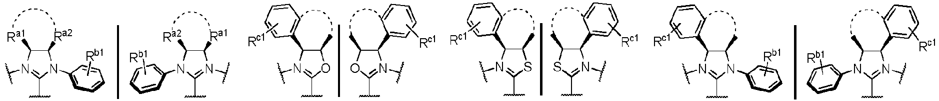 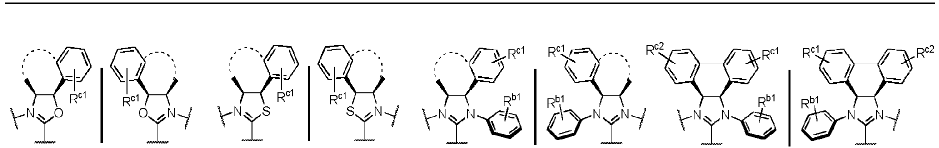 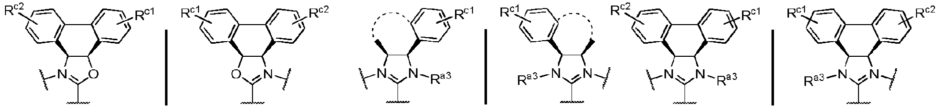
wherein R^{a1}, R^{a2} and R^{a3} each independently represent hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, substituted silyl, or combinations thereof; and
wherein R^{b1}, R^{c1} and R^{c2} each independently represent mono-,di-,tri-, tetra-substituted or unsubstituted, while R^{b1}, R^{c1} and R^{c2} are each independently hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, substituted silyl, or combinations thereof; and two or more adjacent R^{b1}, R^{c1}, and R^{c2} are selectively joined to form a fused ring.

3. The central chirality induced spiro chiral tetradentate cyclometalated platinum (II) and palladium (II) complex-based circularly polarized luminescence material according to claim 2, wherein the structures of L^{a} and L^{b} are selected from: 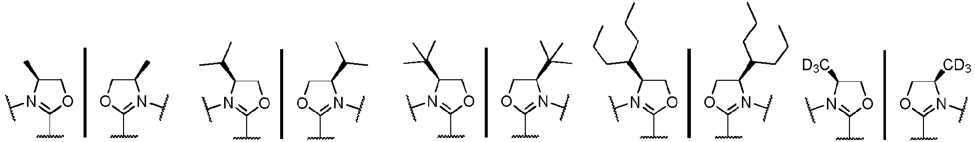 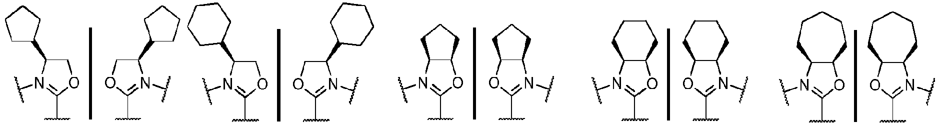 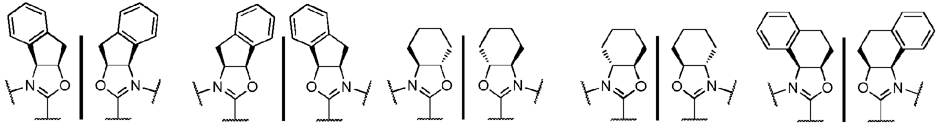 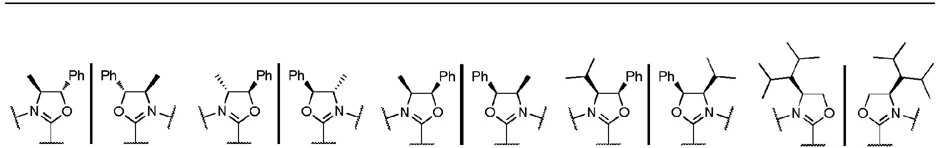 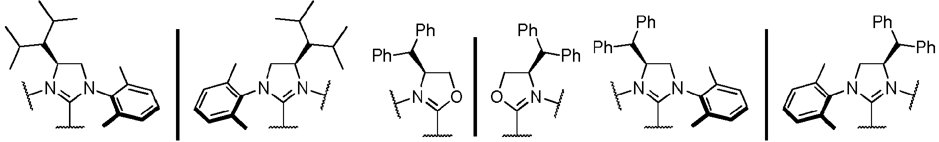 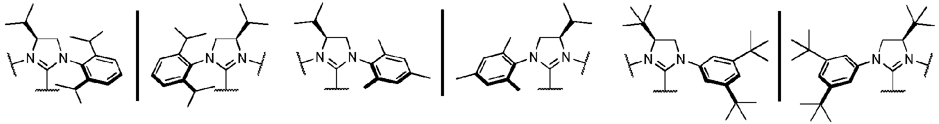 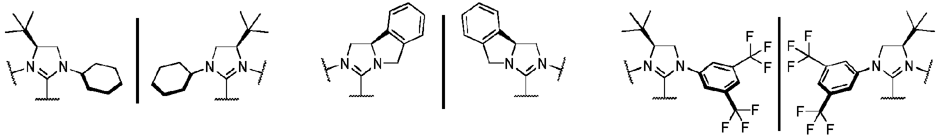 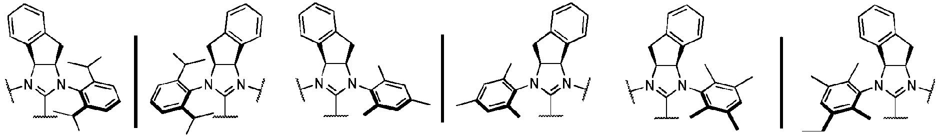 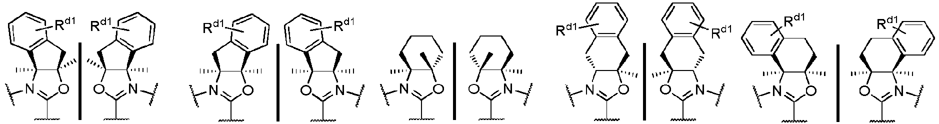 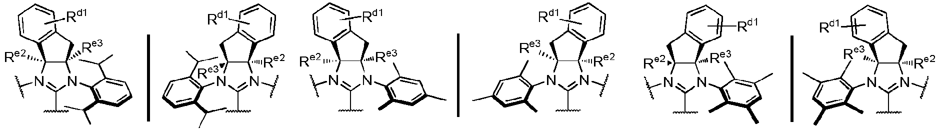 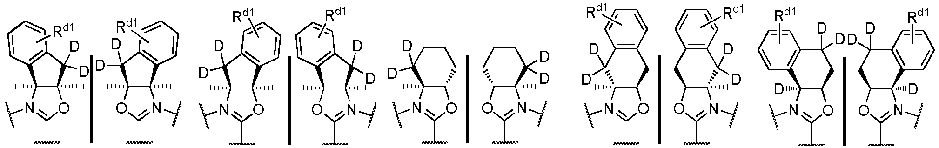 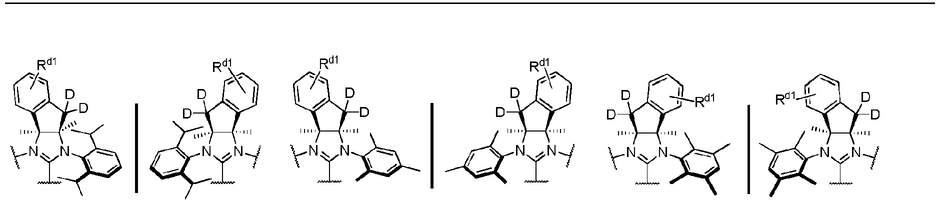 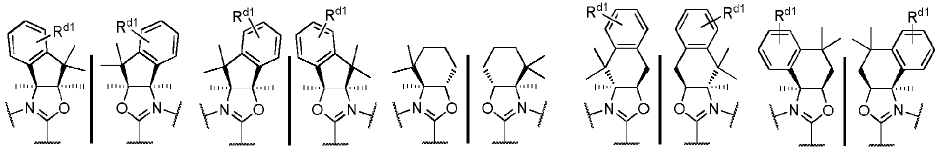 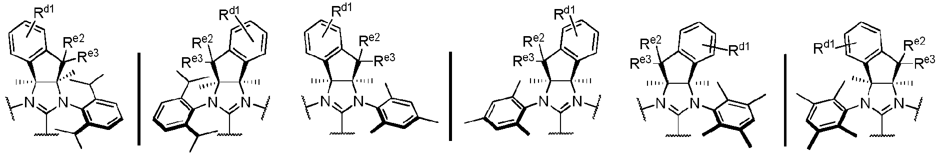 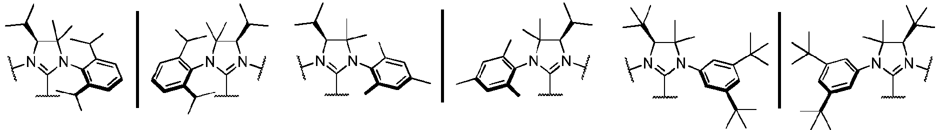 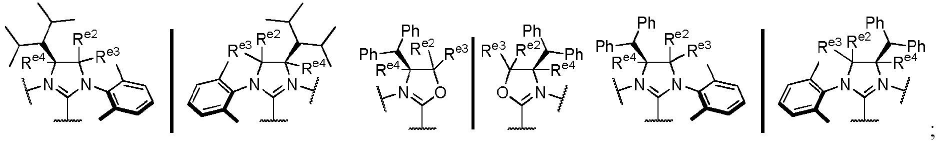 wherein R^{d1}, R^{e1}, R^{e2}, R^{e3} and R^{e4} each independently represent mono-,di-,tri-or tetra-substituted or unsubstituted, while R^{b1}, R^{c1} and R^{c2} are each independently hydrogen, deuterium, halogen, alkyl, cycloalkyl, aryl, heteroalkyl, heterocycloalkyl, heteroaryl, haloalkyl, haloaryl, haloheteroaryl, alkoxy, aryloxy, alkenyl, cycloalkenyl, alkynyl, hydroxyl, mercapto, nitro, cyano, amino, mono-or dialkylamino, mono-or diarylamino, ester, nitrile, isonitrile, heteroaryl, alkoxycarbonyl, amido, alkoxycarbonylamino, aryloxycarbonylamino, sulfonylamino, sulfamoyl, carbamoyl, alkylthio, sulfinyl, ureido, phosphoramido, imino, sulfo, carboxyl, hydrazino, substituted silyl, or combinations thereof; and two or more adjacent R^{b1}, R^{c1}, and R^{c2} are selectively joined to form a fused ring.

4. The central chirality induced spiro chiral tetradentate cyclometalated platinum (II) and palladium (II) complex-based circularly polarized luminescence material according to claim 1, wherein the circularly polarized luminescence material is preferably selected from:

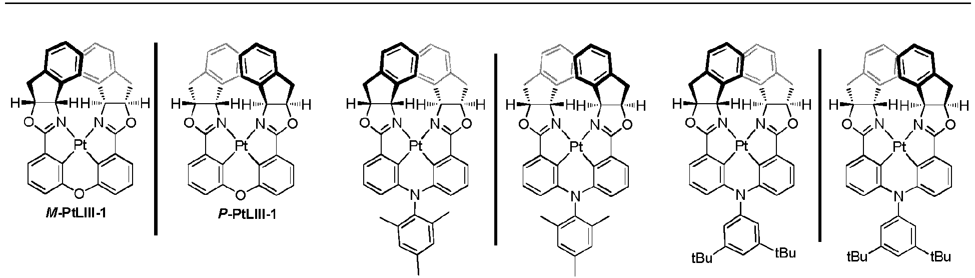

5. Application of the central chirality induced spiro chiral tetradentate cyclometalated platinum (II) and palladium (II) complex-based circularly polarized luminescence material according to claim 1 in an organic light emitting element, 3D display devices, three-dimensional imaging devices, optical information encryption devices, information storage devices and biological imaging devices.

6. The application according to claim 5, wherein the organic light emitting element is an organic light emitting diode, a light emitting diode or a light emitting electrochemical cell.

7. The application according to claim 6, wherein the organic light emitting element comprises a first electrode, a second electrode and at least one organic layer provided between the first electrode and the second electrode, the organic layer comprising the central chirality induced spiro chiral tetradentate cyclometalated platinum (II) and palladium (II) complex-based circularly polarized luminescence material.

8. A display apparatus, comprising an organic light emitting element, wherein the organic light emitting element comprises a first electrode, a second electrode and at least one organic layer provided between the first electrode and the second electrode, the organic layer comprising the central chirality induced spiro chiral tetradentate cyclometalated platinum (II) and palladium (II) complex-based circularly polarized luminescence material according to claim 1.

## Patentansprüche

1. Zirkular polarisiertes Lumineszenzmaterial auf der Basis eines durch zentrale Chiralität induzierten, spirochiralen vierzähnigen cyclometallierten Platin(II)-und-Palladium(II)-Komplexes, wobei das zirkular polarisierte Lumineszenzmaterial eine wie in den allgemeinen Formeln (III) und (III') gezeigte chemische Formel aufweist, wobei (III) und (III') Enantiomere voneinander sind: 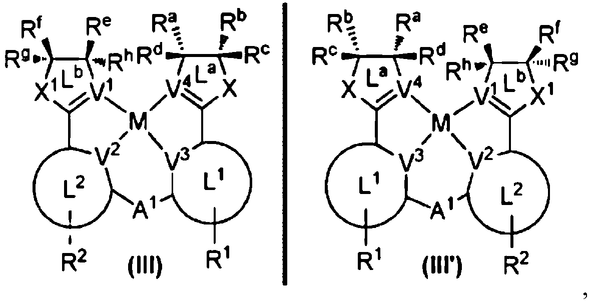
wobei M Pt oder Pd ist; und V¹, V², V³ und V⁴ unabhängig N oder C sind;
L¹, L² und L³ jeweils unabhängig ein fünf- oder sechsgliedriger carbocyclischer, heterocyclischer, aromatischer oder heteroaromatischer Ring sind; und L^{a} und L^{b} jeweils unabhängig ein fünfgliedriger zentral chiraler carbocyclischer oder heterocyclischer Ring sind;
A¹, A², X und X¹ jeweils unabhängig O, S, CR^{x}CR^{y}, C=O, SiR^{x}R^{y}, GeR^{x}CR^{y}, NR^{z}, PR^{z}, R^{z}P=O, AsR^{z}, R^{z}As=O, S=O, SO₂, Se, Se=O, SeO₂, BH, BR^{z}, R^{z}Bi=O oder BiR^{z} sind; und R¹ und R² jeweils unabhängig für mono-, di-, tri-, tetrasubstituiert oder unsubstituiert stehen und währenddessen R¹, R², R^{a}, R^{b}, R^{c}, R^{d}, R^{e}, R^{f}, R^{g}, R^{h}, R^{x}, R^{y} und R^{z} jeweils unabhängig Wasserstoff, Deuterium, Halogen, Alkyl, Cycloalkyl, Aryl, Heteroalkyl, Heterocycloalkyl, Heteroaryl, Haloalkyl, Haloaryl, Haloheteroaryl, Alkoxy, Aryloxy, Alkenyl, Cycloalkenyl, Alkinyl, Hydroxyl, Mercapto, Nitro, Cyano, Amino, Mono- oder Dialkylamino, Mono- oder Diarylamino, Ester, Nitril, Isonitril, Heteroaryl, Alkoxycarbonyl, Amido, Alkoxycarbonylamino, Aryloxycarbonylamino, Sulfonylamino, Sulfamoyl, Carbamoyl, Alkylthio, Sulfinyl, Ureido, Phosphoramido, Imino, Sulfo, Carboxyl, Hydrazino, substituiertem Silyl oder Kombinationen davon ausgewählt sind; wobei benachbarte R¹ und
R² selektiv verbunden sein können, um einen anellierten Ring zu bilden; R^{a}, R^{b}, R^{c} und R^{d} verbunden sind, um ein cyclisches System zu bilden, und beliebige zwei von R^{e}, R^{f}, R^{g} und R^{h} verbunden sein können, um ein cyclisches System zu bilden.

2. Zirkular polarisiertes Lumineszenzmaterial auf der Basis eines durch zentrale Chiralität induzierten, spirochiralen vierzähnigen cyclometallierten Platin(II)-und-Palladium(II)-Komplexes nach Anspruch 1, wobei Strukturen von L^{a} und L^{b} in den allgemeinen Formeln vorzugsweise ausgewählt sind aus: 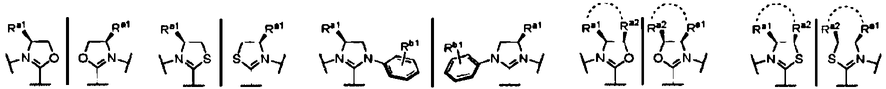 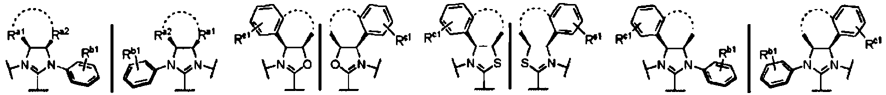 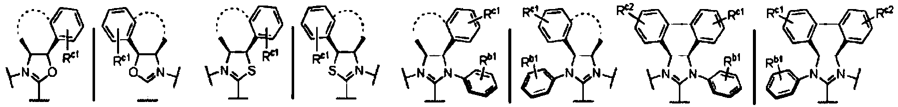 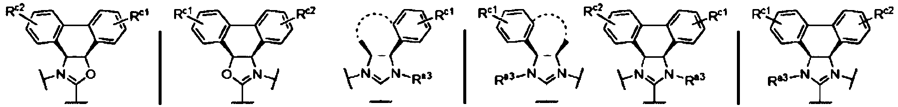
wobei R^{a1}, R^{a2} und R^{a3} jeweils unabhängig für Wasserstoff, Deuterium, Halogen, Alkyl, Cycloalkyl, Aryl, Heteroalkyl, Heterocycloalkyl, Heteroaryl, Haloalkyl, Haloaryl, Haloheteroaryl, Alkoxy, Aryloxy, Alkenyl, Cycloalkenyl, Alkinyl, Hydroxyl, Mercapto, Nitro, Cyano, Amino, Mono- oder Dialkylamino, Mono- oder Diarylamino, Ester, Nitril, Isonitril, Heteroaryl, Alkoxycarbonyl, Amido, Alkoxycarbonylamino, Aryloxycarbonylamino, Sulfonylamino, Sulfamoyl, Carbamoyl, Alkylthio, Sulfinyl, Ureido, Phosphoramido, Imino, Sulfo, Carboxyl, Hydrazino, substituiertes Silyl oder Kombinationen davon stehen; und
wobei R^{b1}, R^{c1} und R^{c2} jeweils unabhängig für mono-, di-, tri-, tetrasubstituiert oder unsubstituiert stehen, während R^{b1}, R^{c1} und R^{c2} jeweils unabhängig Wasserstoff, Deuterium, Halogen, Alkyl, Cycloalkyl, Aryl, Heteroalkyl, Heterocycloalkyl, Heteroaryl, Haloalkyl, Haloaryl, Haloheteroaryl, Alkoxy, Aryloxy, Alkenyl, Cycloalkenyl, Alkinyl, Hydroxyl, Mercapto, Nitro, Cyano, Amino, Mono- oder Dialkylamino, Mono- oder Diarylamino, Ester, Nitril, Isonitril, Heteroaryl, Alkoxycarbonyl, Amido, Alkoxycarbonylamino, Aryloxycarbonylamino, Sulfonylamino, Sulfamoyl, Carbamoyl, Alkylthio, Sulfinyl, Ureido, Phosphoramido, Imino, Sulfo, Carboxyl, Hydrazino, substituiertes Silyl oder Kombinationen sind; und zwei oder mehrere benachbarte R^{b1}, R^{c1} und R^{c2} selektiv verknüpft sind, um einen anellierten Ring zu bilden.

3. Zirkular polarisiertes Lumineszenzmaterial auf der Basis eines durch zentrale Chiralität induzierten, spirochiralen vierzähnigen cyclometallierten Platin(II)-und-Palladium(II)-Komplexes nach Anspruch 2, wobei die Strukturen von L^{a} und L^{b} ausgewählt sind aus: 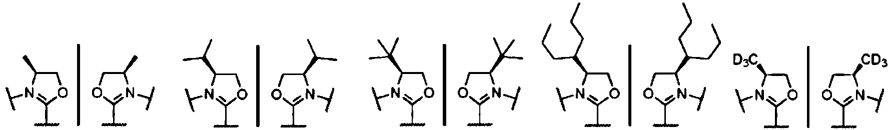 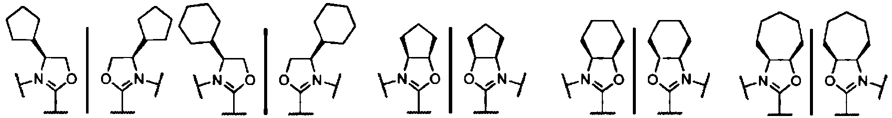 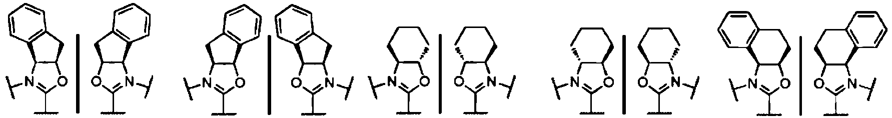 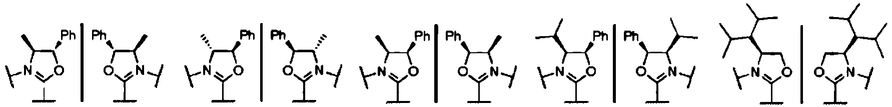 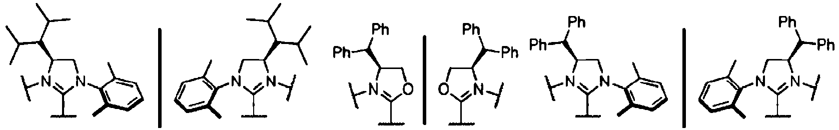 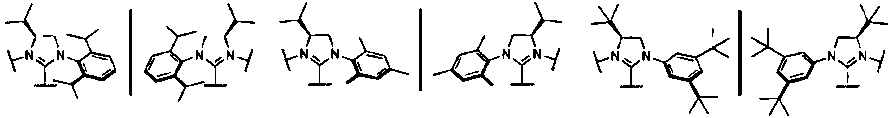 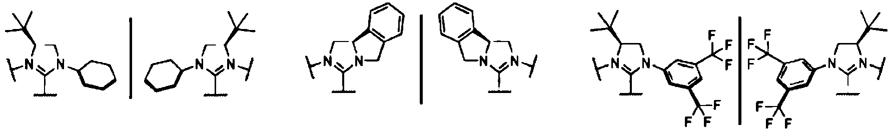 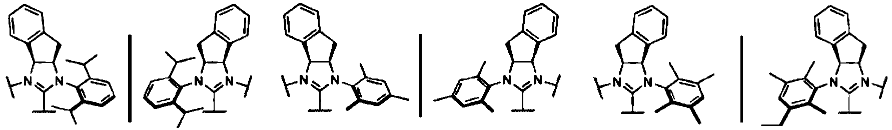 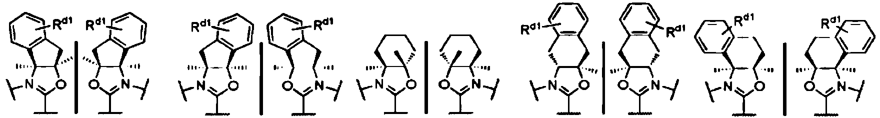 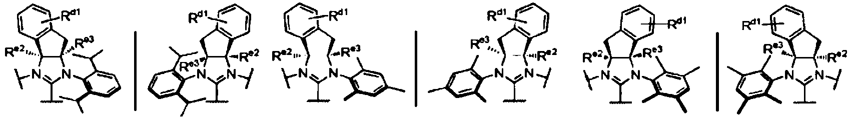 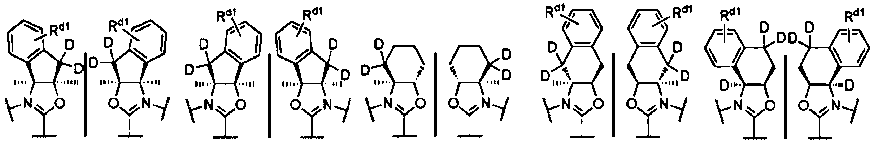 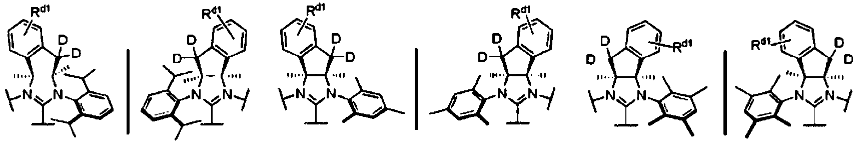 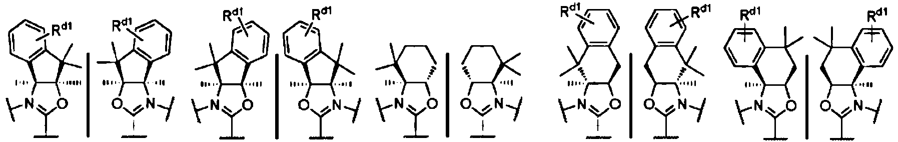 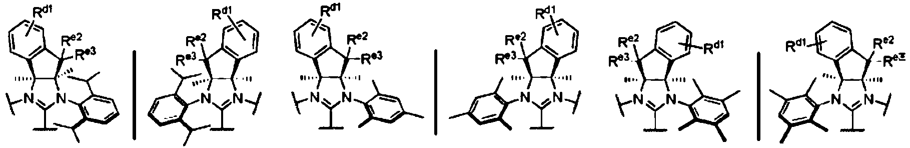 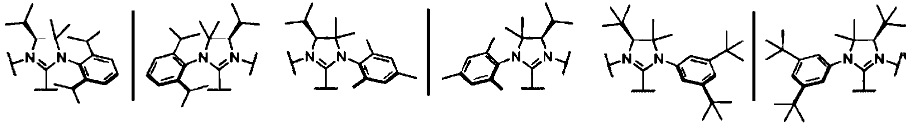 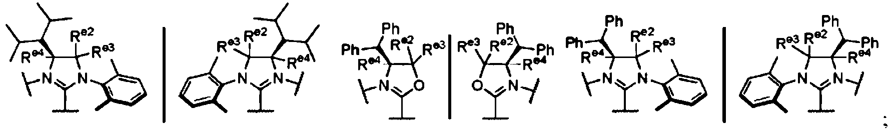 wobei R^{d1}, R^{e1}, R^{e2}, R^{e3} und R^{e4} jeweils unabhängig für mono-, di-, tri- oder tetrasubstituiert oder unsubstituiert stehen, während R^{b1}, R^{c1} und R^{c2} jeweils unabhängig Wasserstoff, Deuterium, Halogen, Alkyl, Cycloalkyl, Aryl, Heteroalkyl, Heterocycloalkyl, Heteroaryl, Haloalkyl, Haloaryl, Haloheteroaryl, Alkoxy, Aryloxy, Alkenyl, Cycloalkenyl, Alkinyl, Hydroxyl, Mercapto, Nitro, Cyano, Amino, Mono- oder Dialkylamino, Mono- oder Diarylamino, Ester, Nitril, Isonitril, Heteroaryl, Alkoxycarbonyl, Amido, Alkoxycarbonylamino, Aryloxycarbonylamino, Sulfonylamino, Sulfamoyl, Carbamoyl, Alkylthio, Sulfinyl, Ureido, Phosphoramido, Imino, Sulfo, Carboxyl, Hydrazino, substituiertes Silyl oder Kombinationen sind; und zwei oder mehrere benachbarte R^{b1}, R^{c1} und R^{c2} selektiv verknüpft sind, um einen anellierten Ring zu bilden.

4. Zirkular polarisiertes Lumineszenzmaterial auf der Basis eines durch zentrale Chiralität induzierten, spirochiralen vierzähnigen cyclometallierten Platin(II)-und-Palladium(II)-Komplexes nach Anspruch 1, wobei das zirkular polarisierte Lumineszenzmaterial vorzugsweise ausgewählt ist aus:

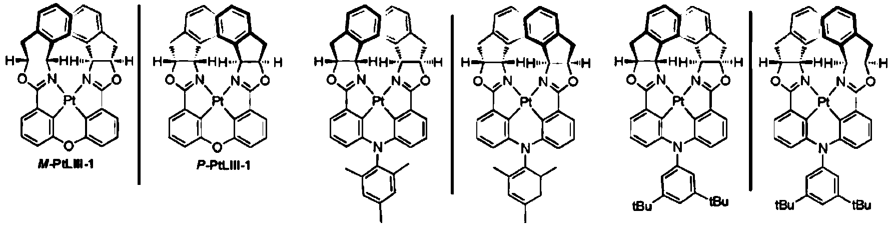

5. Anwendung des zirkular polarisierten Lumineszenzmaterials auf der Basis eines durch zentrale Chiralität induzierten, spirochiralen vierzähnigen cyclometallierten Platin(II)-und-Palladium(II)-Komplexes nach Anspruch 1 in einem organischen lichtemittierenden Element, 3D-Anzeigevorrichtungen, Vorrichtungen zur dreidimensionalen Bildgebung, Vorrichtungen zur Verschlüsselung von optischen Informationen, Informationsspeichervorrichtungen und Vorrichtungen zur biologischen Bildgebung.

6. Anwendung nach Anspruch 5, wobei das organische lichtemittierende Element eine organische Leuchtdiode, eine Leuchtdiode oder eine lichtemittierende elektrochemische Zelle ist.

7. Anwendung nach Anspruch 6, wobei das organische lichtemittierende Element eine erste Elektrode, eine zweite Elektrode und mindestens eine organische Schicht, die zwischen der ersten Elektrode und der zweiten Elektrode vorgesehen ist, umfasst, wobei die organische Schicht das zirkular polarisierte Lumineszenzmaterial auf der Basis eines durch zentrale Chiralität induzierten, spirochiralen vierzähnigen cyclometallierten Platin(II)-und-Palladium(II)-Komplexes umfasst.

8. Anzeigegerät, umfassend ein organisches lichtemittierendes Element, wobei das organische lichtemittierende Element eine erste Elektrode, eine zweite Elektrode und mindestens eine organische Schicht, die zwischen der ersten Elektrode und der zweiten Elektrode vorgesehen ist, umfasst, wobei die organische Schicht das zirkular polarisierte Lumineszenzmaterial auf der Basis eines durch zentrale Chiralität induzierten, spirochiralen vierzähnigen cyclometallierten Platin(II)-und-Palladium(II)-Komplexes nach Anspruch 1 umfasst.

## Revendications

1. Matériau de luminescence à polarisation circulaire à base de complexe de platine cyclométallé de tétradentate spiro chiral induit par chiralité centrale (II) et de palladium (II), dans lequel le matériau de luminescence à polarisation circulaire a une formule chimique telle que montrée dans les formules générales (III) et (III'), où (III) et (III') sont des énantiomères l'une de l'autre : 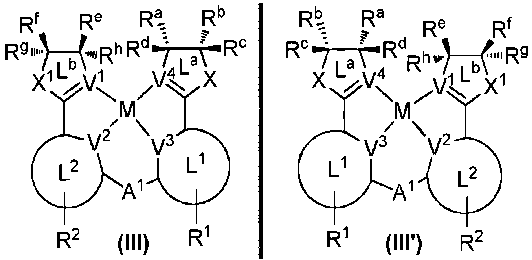
dans laquelle M est Pt ou Pd ; et V¹, V², V³ et V⁴ sont indépendamment N ou C ;
L¹, L² et L³ sont chacun indépendamment un cycle carbocyclique, hétérocyclique, aromatique ou hétéroaromatique à cinq ou six chaînons ; et L^{a} et L^{b} sont chacun indépendamment un cycle carbocyclique ou hétérocyclique chiral central à cinq chaînons ;
A¹, A², X et X¹ sont chacun indépendamment 0, S, CR^{x}R^{y}, C=O, SiR^{x}R^{y}, GeR^{x}R^{y}, NR^{z}, PR^{z}, R^{z}P=O, AsR^{z}, R^{z}As=O, S=0, SO₂, Se, Se=O, SeO₂, BH, BR^{z}, R^{z}Bi=O ou BiR^{z} ; et
R¹ et R² représentent chacun indépendamment mono-, di-, tri-, tétra-substitués ou non substitués, et dans le même temps R¹, R², R^{a}, R^{b}, R^{c}, R^{d}, R^{e}, R^{f}, R^{g}, R^{h}, R^{x}, R^{y} et R^{z} sont chacun indépendamment hydrogène, deutérium, halogène, alkyle, cycloalkyle, aryle, hétéroalkyle, hétérocycloalkyle, hétéroaryle, haloalkyle, haloaryle, halohétéroaryle, alcoxy, aryloxy, alcényle, cycloalcényle, alcynyle, hydroxyle, mercapto, nitro, cyano, amino, mono-ou dialkylamino, mono- ou diarylamino, ester, nitrile, isonitrile, hétéroaryle, alcoxycarbonyle, amido, alcoxycarbonylamino, aryloxycarbonylamino, sulfonylamino, sulfamoyle, carbamoyle, alkylthio, sulfinyle, uréido, phosphonamido, imino, sulfo, carboxyle, hydrazino, silyle substitué, ou des combinaisons de ceux-ci ; deux R¹ et R² adjacents peuvent être connectés sélectivement pour former un cycle fusionné ; deux quelconques de R^{a}, R^{b}, R^{c} et R^{d} sont connectés pour former un système cyclique, et deux quelconques de R^{e}, R^{f}, R^{g} et R^{h} peuvent être connectés pour former un système cyclique.

2. Matériau de luminescence à polarisation circulaire à base de complexe de platine cyclométallé de tétradentate spiro chiral induit par chiralité centrale (II) et de palladium (II) selon la revendication 1, dans lequel les structures de L^{a} et L^{b} dans les formules générales sont de préférence choisies parmi : 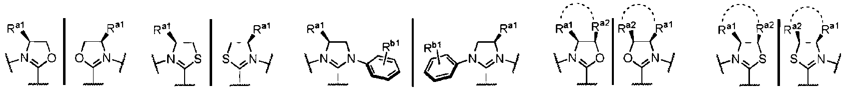 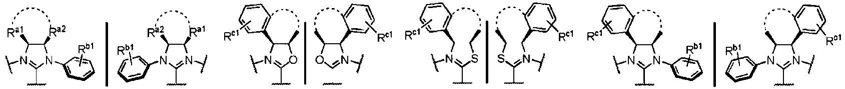 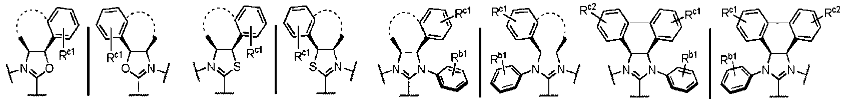 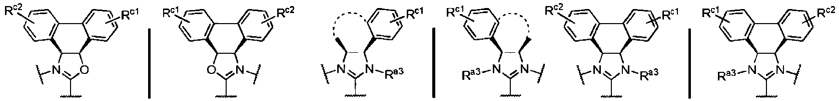 dans lesquelles R^{a1}, R^{a2} et R^{a3} représentent chacun indépendamment hydrogène, deutérium, halogène, alkyle, cycloalkyle, aryle, hétéroalkyle, hétérocycloalkyle, hétéroaryle, haloalkyle, haloaryle, halohétéroaryle, alcoxy, aryloxy, alcényle, cycloalcényle, alcynyle, hydroxyle, mercapto, nitro, cyano, amino, mono- ou dialkylamino, mono-ou diarylamino, ester, nitrile, isonitrile, hétéroaryle, alcoxycarbonyle, amido, alcoxycarbonylamino, aryloxycarbonylamino, sulfonylamino, sulfamoyle, carbamoyle, alkylthio, sulfinyle, uréido, phosphoramide, imino, sulfo, carboxyle, hydrazino, silyle substitué, ou des combinaisons de ceux-ci ; et
dans lesquelles R^{b1}, R^{c1} et R^{c2} représentent chacun indépendamment mono-, di-, tri-, tétra-substitué ou non substitué, tandis que R^{b1}, R^{c1} et R^{c2} sont chacun indépendamment hydrogène, deutérium, halogène, alkyle, cycloalkyle, aryle, hétéroalkyle, hétérocycloalkyle, hétéroaryle, haloalkyle, haloaryle, halohétéroaryle, alcoxy, aryloxy, alcényle, cycloalcényle, alcynyle, hydroxyle, mercapto, nitro, cyano, amino, mono-ou dialkylamino, mono-ou diarylamino, ester, nitrile, isonitrile, hétéroaryle, alcoxycarbonyle, amido, alcoxycarbonylamino, aryloxycarbonylamino, sulfonylamino, sulfamoyle, carbamoyle, alkylthio, sulfinyle, uréido, phosphoramido, imino, sulfo, carboxyle, hydrazino, silyle substitué, ou des combinaisons de ceux-ci ; et deux R^{b1}, R^{c1} et R^{c2} adjacents ou plus sont sélectivement reliés pour former un cycle fusionné.

3. Matériau de luminescence à polarisation circulaire à base de complexe de platine cyclométallé de tétradentate spiro chiral induit par chiralité centrale (II) et de palladium (II) selon la revendication 2, dans lequel les structures de L^{a} et L^{b} sont choisies parmi : 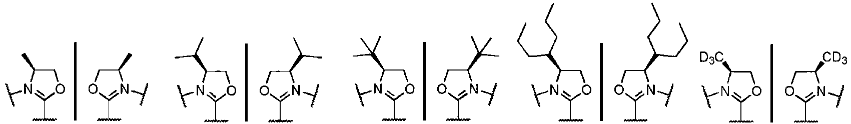 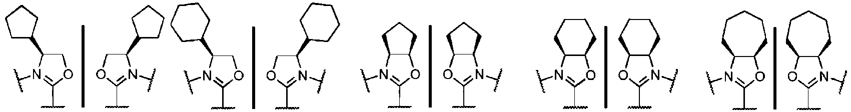 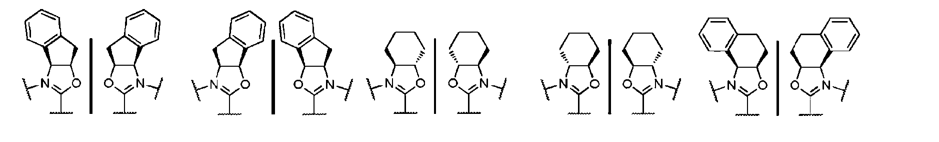 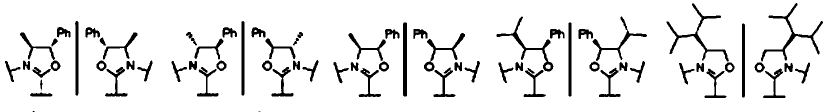 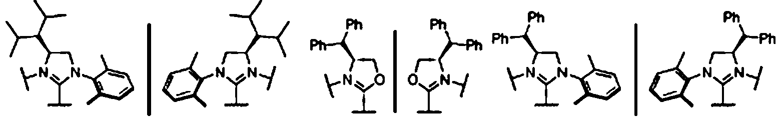 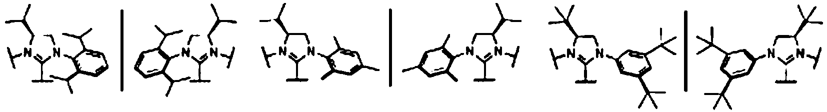 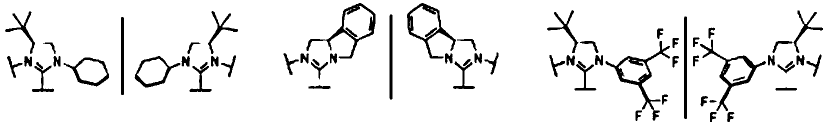 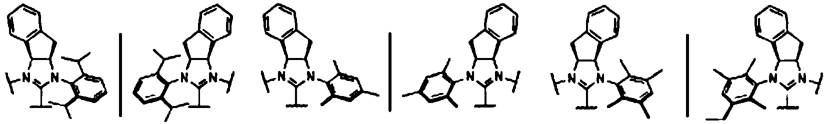 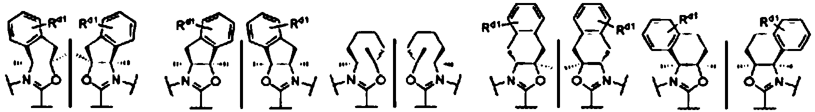 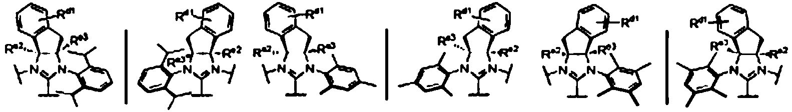 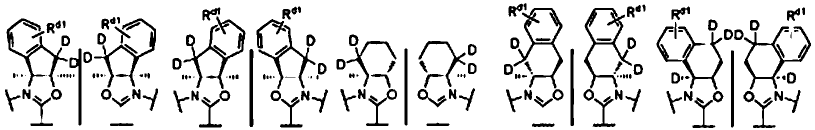 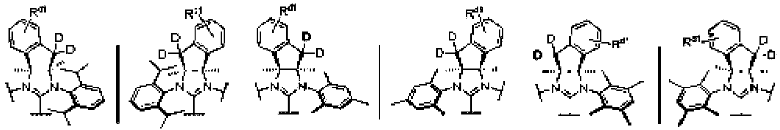 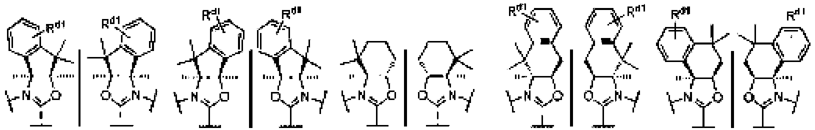 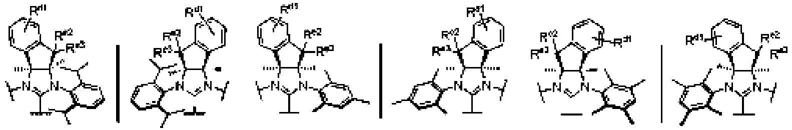 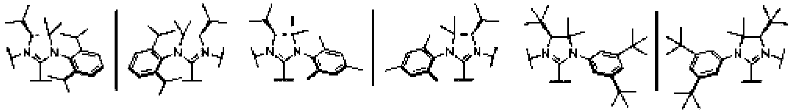 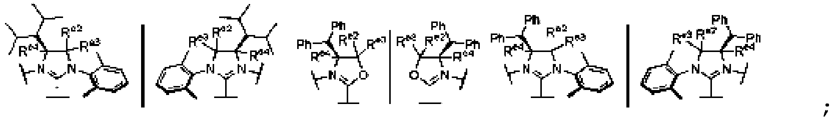 dans lesquelles R^{d1}, R^{e1}, R^{e2}, R^{e3} et R^{e4} représentent chacun indépendamment mono-, di-, tri- ou tétra-substitués ou non substitués, tandis que R^{b1}, R^{c1} et R^{c2} sont chacun indépendamment hydrogène, deutérium, halogène, alkyle, cycloalkyle, aryle, hétéroalkyle, hétérocycloalkyle, hétéroaryle, haloalkyle, haloaryle, halohétéroaryle, alcoxy, aryloxy, alcényle, cycloalcényle, alcynyle, hydroxyle, mercapto, nitro, cyano, amino, mono-ou dialkylamino, mono-ou diarylamino, ester, nitrile, isonitrile, hétéroaryle, alcoxycarbonyle, amido, alcoxycarbonylamino, aryloxycarbonylamino, sulfonylamino, sulfamoyle, carbamoyle, alkylthio, sulfinyle, uréido, phosphoramide, imino, sulfo, carboxyle, hydrazino, silyle substitué, ou des combinaisons de ceux-ci ; et deux R^{b1}, R^{c1}, et R^{c2} adjacents ou plus sont sélectivement reliés pour former un cycle fusionné.

4. Matériau de luminescence à polarisation circulaire à base de complexe de platine cyclométallé de tétradentate spiro chiral induit par chiralité centrale (II) et de palladium (II) selon la revendication 1, dans lequel le matériau de luminescence à polarisation circulaire est de préférence choisi parmi :

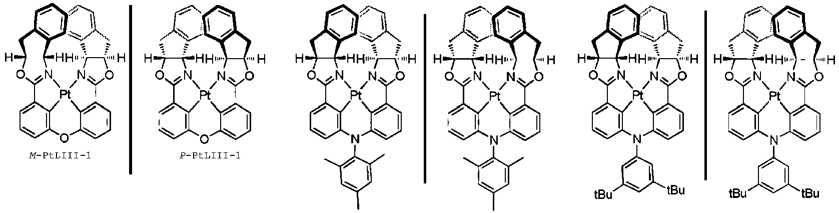

5. Application du matériau de luminescence à polarisation circulaire à base de complexe de platine cyclométallé de tétradentate spiro chiral induit par chiralité centrale (II) et de palladium (II) selon la revendication 1 dans un élément électroluminescent organique, des dispositifs d'affichage 3D, des dispositifs d'imagerie tridimensionnelle, des dispositifs de chiffrement d'informations optiques, des dispositifs de stockage d'informations et des dispositifs d'imagerie biologique.

6. Application selon la revendication 5, dans laquelle l'élément électroluminescent organique est une diode électroluminescente organique, une diode électroluminescente ou une cellule électrochimique électroluminescente.

7. Application selon la revendication 6, dans laquelle l'élément électroluminescent organique comprend une première électrode, une seconde électrode et au moins une couche organique prévue entre la première électrode et la seconde électrode, la couche organique comprenant le matériau de luminescence à polarisation circulaire à base de complexe de platine cyclométallé de tétradentate spiro chiral induit par chiralité centrale (II) et de palladium (II).

8. Appareil d'affichage, comprenant un élément électroluminescent organique, dans lequel l'élément électroluminescent organique comprend une première électrode, une seconde électrode et au moins une couche organique prévue entre la première électrode et la seconde électrode, la couche organique comprenant le matériau de luminescence à polarisation circulaire à base de complexe de platine cyclométallé de tétradentate spiro chiral induit par chiralité centrale (II) et de palladium (II) selon la revendication 1.
